(19) Europäisches Patentamt
European Patent Office
Office européen des brevets

(11) **EP 4 410 995 A1**

(12) **EUROPEAN PATENT APPLICATION**

(43) Date of publication:
**07.08.2024  Bulletin 2024/32**

(21) Application number: **23382089.3**

(22) Date of filing: **01.02.2023**

(51) International Patent Classification (IPC):
**C12Q 1/6855** (2018.01)

(52) Cooperative Patent Classification (CPC):
(C-Sets available)
**C12Q 1/6855; C12Q 1/68**           (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **4basebio, S.L.U.**
  **28049 Cantoblanco (Madrid) (ES)**
• **4basebio UK Ltd**
  **Cambridge CB24 5QE (GB)**

(72) Inventors:
• **LANCKRIET, Heikki**
  **Cambridge, CB24 5QE (GB)**

• **WALKER, Amy**
  **Cambridge, CB24 5QE (GB)**
• **PICHER, Ángel**
  **E-28049 Cantoblanco, Madrid (ES)**
• **DIXON, Daniel**
  **Cambridge, CB24 5QE (GB)**
• **PAVLICKOVA, Milena**
  **Cambridge, CB24 5QE (GB)**

(74) Representative: **Boult Wade Tennant LLP**
  **Salisbury Square House**
  **8 Salisbury Square**
  **London EC4Y 8AP (GB)**

Remarks:
The complete document including Reference Table(s) and the Sequence Listing(s) can be downloaded from the EPO website

(54) **LINEAR DNA WITH ENHANCED RESISTANCE AGAINST EXONUCLEASES AND METHODS FOR THE PRODUCTION THEREOF**

(57)    The present invention relates to methods for producing a linear deoxyribonucleic acid (DNA) product (e.g. a closed linear DNA product) with enhanced resistance to nuclease digestion. The present invention relates to methods comprising the steps: (a) contacting a double-stranded DNA molecule with an endonuclease, a ligase and first and second terminal adaptor molecules and intermediate adaptor molecules, to form a single contiguous aqueous volume; and (b) incubating the single contiguous aqueous volume to generate a linear DNA product (e.g. a closed linear DNA product). The present invention also relates to linear deoxyribonucleic acid (DNA) products (e.g. a closed linear DNA products) with enhanced resistance to nuclease digestion and uses thereof.

Fig. 1

**EP 4 410 995 A1**

(52) Cooperative Patent Classification (CPC): (Cont.)

C-Sets
**C12Q 1/6855, C12Q 2521/301, C12Q 2521/319,
C12Q 2521/507, C12Q 2525/125,
C12Q 2525/131, C12Q 2525/143,
C12Q 2525/149, C12Q 2525/191,
C12Q 2525/301, C12Q 2531/125,
C12Q 2537/101, C12Q 2537/162**

**Description**

**TECHNICAL FIELD**

The present invention relates to methods for producing a linear deoxyribonucleic acid (DNA) product (e.g. a closed linear DNA product) with enhanced resistance to nuclease digestion. The present invention relates to methods comprising the steps: (a) contacting a double-stranded DNA molecule with an endonuclease, a ligase and first and second terminal adaptor molecules and intermediate adaptor molecules, to form a single contiguous aqueous volume; and (b) incubating the single contiguous aqueous volume to generate a linear DNA product (e.g. a closed linear DNA product). The present invention also relates to linear deoxyribonucleic acid (DNA) products (e.g. a closed linear DNA products) with enhanced resistance to nuclease digestion and uses thereof.

**BACKGROUND**

DNA is susceptible to degradation by nucleases which are naturally occurring enzymes within organisms and which have a vital role in the regulation of many cellular processes, while also protecting against foreign DNA species. Enzymatic DNA degradation can render gene therapies ineffective and is a substantial consideration when developing gene therapies or DNA vaccines.

Considerable efforts have been made to extend the effective molecular lifetime of nucleic acids by increasing resistance of the nucleic acid molecules to both extracellular and intracellular nucleases.

For linear molecules, one of the proposed solutions includes the use of phosphorothioated nucleotides (i.e. 2'-deoxynucleotides-5'-($\alpha$-thio)-triphosphate).

Phosphorothioated nucleotides comprise a sulphur atom instead of a non-bridging oxygen atom. These modified nucleotides show comparable physical and chemical characteristics to corresponding unmodified nucleotides, but are resistant to exonuclease digestion. As such, the incorporation of the phosphorothioate functional group can prolong the half-life of the nucleic acid molecule.

Phosphorothioate modifications are used in nucleic acid drug development programmes. In therapeutic nucleic acids, the phosphorothioated nucleotides are incorporated into short, single-stranded polynucleotide chains. For example, an antisense oligonucleotide fomivirsen is a 21-mer phosphorothioate oligodeoxynucleotide used to treat cytomegalovirus retinitis (Stein and Castanotto, "FDA-approved oligonucleotide therapies in 2017." Molecular Therapy 25.5 (2017): 1069-1075). Similarly, pegaptanib (brand name Macugen) is a short (27-nucleotides) aptamer with a phosphorothioate 3'-3' deoxythymidine cap used for treating age-related macular degeneration of the retina.

Phosphorothioate modifications have also been used in the context of a linear double-stranded polynucleotide chain (e.g. double-stranded DNA) to cap the ends of the polynucleotide chain to increase resistance to exonuclease digestion (Putney et al. "A DNA fragment with an alpha-phosphorothioate nucleotide at one end is asymmetrically blocked from digestion by exonuclease III and can be replicated in vivo." Proceedings of the National Academy of Sciences 78.12 (1981): 7350-7354). To cap the ends of the polynucleotide chain, the ends are digested with a restriction enzyme and treated with a DNA polymerase and a mixture of deoxyribonucleotide triphosphates (dNTPs), at least one type of which is a phosphorothioated nucleotide complementary to a nucleotide in the overhanging strand. Since DNA polymerases add nucleotides in the 5' to 3' direction, the result of this treatment is a blunt-ended polynucleotide fragment with a phosphorothioated nucleotide located at the 3'-end of each strand (i.e. in "the cap").

Resistance to nuclease digestion can also be accomplished by using closed DNA molecules, such as plasmids or minicircles. However, plasmids and minicircles have limited utility *in vivo* due to their frequent contamination with toxic agents derived from cell components, fidelity issues that alter the sequence of interest, and presence of different species (supercoiled, linear and open circular).

Alternatively, resistance to nuclease digestion may be accomplished by producing closed linear DNA molecules. For example, WO2010/086626 A1 describes a method for producing a closed linear DNA by utilizing a protelomerase. However, this method is limited in that the action of protelomerase produces the same sequence at both ends of the closed linear DNA molecules, allowing for little flexibility.

WO2008/095927 describes a system known as "Golden Gate" assembly, whereby separately digested fragments of a desired construct are ligated into a vector. This method is inefficient due to the number of ligation events that must occur and having to use multiple different restriction sites. Furthermore, the resulting yield is low and the desired DNA product must be amplified using bacteria to increase the amount of DNA, requiring the presence of, e.g. antibiotic resistance genes, meaning commercial scalability of the "Golden Gate" process is not possible without the use of fermentation.

Thus, a need exists for a more flexible method for producing a linear DNA product with an enhanced resistance to nuclease (e.g. exonuclease) digestion, whilst allowing for additional features to be incorporated for different applications.

## DESCRIPTION

The invention provides a method for producing a linear deoxyribonucleic acid (DNA) product (e.g. a closed linear DNA product) with enhanced resistance to nuclease digestion. The invention is based on the addition of adaptor molecules to a double-stranded DNA molecule. The method of the invention relies on the addition of the adaptor molecules, an endonuclease and a ligase to the double-stranded DNA molecule in a single reaction volume (or single contiguous aqueous volume). Thus, the method for producing a linear DNA product comprises the steps: (a) contacting a double-stranded DNA molecule with an endonuclease, a ligase and first and second terminal adaptor molecules and n+m intermediate adaptor molecules and n+m intermediate adaptor molecules, to form a single contiguous aqueous volume, wherein n and m are each 0 or an integer of at least 1, and wherein n+m is at least 1, wherein n and m are each 0 or an integer of at least 1, and wherein n + m is at least 1; and (b) incubating the single contiguous aqueous volume to generate the linear DNA product. Preferably, the linear DNA product has enhanced resistance to exonuclease (e.g. exonuclease I, exonuclease III and/or exonuclease VIII) digestion. The linear DNA product may be a closed linear DNA product. The linear DNA product may comprise nuclease-resistant nucleotides (i.e. protected nucleotides), such as phosphorothioated nucleotides. The linear DNA product may be a partially closed linear DNA product. The partially closed linear DNA product may comprise nuclease-resistant nucleotides (i.e. protected nucleotides), such as phosphorothioated nucleotides. The linear DNA product may comprise a cassette. The cassette may comprise a coding sequence.

The invention provides a method for producing a linear DNA product, wherein the method comprises: (a) contacting a double-stranded DNA molecule with an endonuclease and first and second terminal adaptor molecules and n + m intermediate adaptor molecules, , to form a single contiguous aqueous volume, wherein n and m are each 0 or an integer of at least 1, and wherein n+m is at least 1; and (b) incubating the single contiguous aqueous volume to generate the linear DNA product, wherein the linear DNA product comprises a linear double-stranded region, wherein the linear double-stranded region comprises a portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The first and second terminal adaptor molecules may be identical molecules or they may be different molecules. For example, the first terminal adaptor molecule and/or the second terminal adaptor molecule may comprise a hairpin. The first terminal adaptor molecule and/or the second terminal adaptor molecule may double-stranded linear nucleic acid molecules comprising one or more nuclease-resistant nucleotides. The first terminal adaptor molecule may comprise a hairpin and the second terminal adaptor molecule may be a double-stranded linear nucleic acid molecule comprising one or more nuclease-resistant nucleotides. Thus, the linear DNA product produced by the methods described herein is resistant to nuclease (e.g. exonuclease) digestion.

The intermediate adaptor molecules may be the same or they may be different. One or more of the n+m intermediate adaptor molecules may comprise a spacer or a linker sequence. One or more of the n+m intermediate adaptor molecules may comprise a fluorophore, a barcode, a polyA signal, a biotinylated nucleotide, a protected nucleotide, a modified nucleotide, a spacer, polyA sequence, a promotor, an open reading frame, a UTR (untranslated region), a transcription factor binding site, or a terminator sequence. The n intermediate adaptor molecules sequentially appended to a first end of the double-stranded DNA molecule may be the same or may be different from the m intermediate adaptor molecules sequentially appended to the second end of the double-stranded DNA molecule.

A modified nucleotide may be 2-MethoxyEthoxy A, 2-MethoxyEthoxy MeC, 2-MethoxyEthoxy G, 2-MethoxyEthoxy T, 2'-O-Methyl RNA Bases, Fluoro Bases, 2-Aminopurine, 5-Bromo dU, deoxyUridine, 2,6-Diaminopurine (2-Amino-dA), Dideoxy-C, deoxyInosine, Hydroxymethyl dC, Iso-dG, Iso-dC, 5-Methyl dC or 5-Nitroindole.

The step of contacting the double-stranded DNA molecule with the endonuclease and first and second terminal adaptor molecules is preferably performed in the presence of a ligase. Thus, the invention provides a method for producing a linear deoxyribonucleic acid (DNA) product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region.

The appending (or linking or closing) of the first terminal adaptor molecule and/or the second terminal adaptor molecule and/or the intermediate adaptor molecules may be performed by hybridization or ligation of the adaptor molecules to the ends of the linear double-stranded region and/or to sequential adaptor molecules. Thus, the n intermediate adaptor molecules may be hybridized to the first end of the linear double-stranded region. The m intermediate adaptor molecules may be hybridized to the second end of the linear double-stranded region. The n intermediate adaptor molecules may be ligated to the first end of the linear double-stranded region. The m intermediate adaptor molecules may be ligated to the second end of the linear double-stranded region. The appending of the n+m intermediate adaptor molecules, first terminal adaptor molecule and the second terminal adaptor molecule may be performed by both hybridization and ligation of the adaptor molecules to the ends of the linear double-stranded region and/or to sequential adjacent adaptor molecules. Thus, n intermediate adaptor molecules may be hybridized and ligated to the first end of the linear double-stranded region. The m intermediate adaptor molecules may be hybridized and ligated to the second end of the linear double-stranded region. The appending may be performed via a linker or spacer molecule which facilitates joining of the adaptor molecule to the first and/or second end of the linear double-stranded region or to another adaptor molecule. The first terminal adaptor molecules may be hybridized, ligated or hybridized and ligated to the nth intermediate adaptor molecule or to the first end of the double-stranded DNA molecule. The second terminal adaptor molecule may hybridized, ligated or hybridized and ligated to the mth intermediate adaptor molecule or the second end of the double stranded DNA molecule. n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3or 4..

The method may further comprise, before step (a) (i.e. the step of contacting the double-stranded DNA molecule with the endonuclease, the ligase and the first and second terminal adaptor molecules and the n+m intermediate adaptor molecules), a step of amplification of a DNA template molecule to produce the double-stranded DNA molecule. Thus, the invention provides a method for producing a linear DNA product, the method comprises:

(a) amplification of a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the linear DNA product, wherein the linear DNA product comprises a linear double-stranded region, and wherein n intermediate adaptor molecules are sequentially ligated to a first end of the linear double stranded region and m intermediate adaptor molecules are sequentially ligated to a second end of the linear double-stranded region, and wherein the first terminal adaptor molecule is ligated to the nth intermediate adaptor molecule or, when n is 0 , to the first end of the linear double stranded region, and the second terminal adaptor molecule is ligated to the mth intermediate adaptor molecule, or when m is 0, to the second end of the linear double stranded region.

The amplification may be an in vitro or in vivo amplification. Preferably, the amplification is an in vitro amplification. For example, the amplification may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification. Thus, the invention provides a method for producing a linear deoxyribonucleic acid (DNA) product, the method comprises:

(a) rolling circle amplification of a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and n+m is at least 1; and
(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially ligated to a first end of the linear double stranded region and m intermediate adaptor molecules are sequentially ligated to a second end of the linear double-stranded region, and wherein the first terminal adaptor molecule is ligated to the nth intermediate adaptor molecule or, when n is 0 , to the first end of the linear double stranded region, and the second terminal adaptor molecule is ligated to the mth intermediate adaptor molecule, or when m is 0, to the second end of the linear double stranded region.

The method may further comprise (after the step of amplification and before the step of contacting the double-stranded DNA molecule with an endonuclease, a ligase and first and second terminal adaptor molecules) a step of heat-deactivation. Thus, the invention provides a method for producing a linear DNA product, the method comprises:

(a) amplification of a DNA template molecule comprising at least one cleavable (e.g. endonuclease) target sequence to generate a double-stranded DNA molecule;
(b) heat-deactivation of the reaction of step (a);
(c) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1 and n+m is at least 1; and
(d) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially ligated to a first end of the linear double stranded region and m intermediate adaptor molecules are sequentially ligated to a second end of the linear double-stranded region, and wherein the first terminal adaptor molecule is ligated to the nth intermediate adaptor molecule or, when n is 0 , to the first end of the linear double stranded region, and the second terminal adaptor molecule is ligated to the mth intermediate adaptor molecule, or when m is 0, to the second end of the linear double stranded region.

Preferably, amplification is rolling-circle amplification.

The step of heat-deactivation may be performed under conditions sufficient to inactive the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

The inventors of the present application have surprisingly discovered that large concatemeric products of the rolling circle amplification reaction can be used to produce the DNA products described herein. This is surprising as the product of the rolling circle amplification has high viscosity and typically has to undergo purification steps before it can be utilized for downstream applications.

In the method described herein, after the step of amplification, the step of contacting the double-stranded DNA molecule with an endonuclease, a ligase and first and second terminal adaptor molecules and n+m intermediate adaptor molecules, to form a single contiguous aqueous volume, may be performed without purifying the product of the amplification reaction. That is to say that the step of contacting the double-stranded DNA molecule with an endonuclease, a ligase and first and second terminal adaptor molecules and n+m intermediate adaptor molecules may be performed directly after the step of amplification. The step of contacting the double-stranded DNA molecule with an endonuclease, a ligase and first and second terminal adaptor molecules and n+m intermediate adaptor molecules may be performed directly after the step of heat-deactivation.

The inventors of the present application have discovered a method which requires a very few steps to produce the DNA product described herein. The methods described herein are very time efficient. This is, in part, due to the fact that a step of purification is not required after the amplification reaction. Optionally, the product of the amplification reaction may be heat-deactivated. Surprising, the method described herein where the step of contacting the double-stranded DNA molecule with an endonuclease, a ligase and first and second terminal adaptor molecules and intermediate adaptor molecules is performed directly after the step of amplification (i.e. without the step of purification) produces higher yields of the DNA product described herein when compared to a method in which the two steps are separated by purification of the amplification product.

The method may further comprise, after the step of incubating the single contiguous aqueous volume, a step of purification of the linear DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purification. The step of nuclease digestion may allow for removal of any double-stranded DNA molecules and/or adaptor molecules which have not been used to produce linear DNA products. Thus, the method for producing a linear DNA product may comprise the steps:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0 , to the first end of the linear double stranded region, and the second terminal adaptor molecule is appended to the mth intermediate adaptor molecule, or when m is 0, to the second end of the linear double stranded region; and
(c) incubating the single contiguous aqueous volume with a nuclease (e.g. an exonuclease).

n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The method for producing a linear DNA product may comprise the steps:

(a) amplification of a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially ligated to a first end of the linear double stranded region and m intermediate adaptor molecules are sequentially ligated to a second end of the linear double-stranded region, and wherein the first terminal adaptor molecule is ligated to the nth intermediate adaptor molecule or, when n is 0 , to the first end of the linear double stranded region, and the second terminal adaptor molecule is ligated to the mth intermediate adaptor molecule, or when m is 0, to the second end of the linear double stranded region; and
(d) incubating the single contiguous aqueous volume with a nuclease (e.g. an exonuclease).

The method for producing a linear DNA product may comprise the steps:

(a) amplification of a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0 , to the first end of the linear double stranded region, and the second terminal adaptor molecule is appended to the mth intermediate adaptor molecule, or when m is 0, to the second end of the linear double stranded region;
(d) purification of the linear DNA product; and
(e) incubating the product of step (d) with a nuclease (e.g. an exonuclease).

The method for producing a linear DNA product may comprise the steps:

(a) amplification of a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0 , to the first end of the linear double stranded region, and the second terminal adaptor molecule is appended to the mth intermediate adaptor molecule, or when m is 0, to the second end of the linear double stranded region;

(d) incubating the single contiguous aqueous volume with a nuclease (e.g. an exonuclease); and

(e) purification of the linear DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a BbsI, BsaI, BsmBI, BspQI, BtgZI, Esp3I,SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BsIFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, Btsl-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI restriction enzyme.

Type IIS restriction endonucleases cleave the double-stranded DNA molecule outside of the recognition sequence (i.e. an endonuclease target sequence), which means that the recognition sequence (i.e. endonuclease target sequence) is not included in the linear DNA product.

The present inventors have surprisingly discovered methods for production of a linear DNA product of enhanced resistance to nuclease digestion. Specifically, the linear DNA product produced by the methods described herein has enhanced resistance to exonuclease digestion (e.g. exonuclease III digestion). The enhanced resistance to exonuclease digestion extends the life of the linear DNA product in a cell (i.e. the linear DNA product has enhanced resistance to intracellular exonucleases) and in a cell-free system (i.e. the linear DNA product has enhanced resistance to extracellular exonucleases). The present inventors have developed a method which relies on the addition of adaptor molecules (i.e. a first terminal adaptor molecule and a second terminal adaptor molecule and intermediate adaptor molecules) to one or both ends of a double-stranded DNA molecule. The terminal adaptor molecules may comprise a hairpin or a loop to form a closed linear DNA product with enhanced resistance to nuclease (e.g. exonuclease) digestion. In addition, the present inventors have discovered a method for efficient introduction of protected nucleotides in the form of a first terminal adaptor molecule and a second terminal adaptor molecule at both ends of a linear double-stranded region of the linear DNA product. The methods described herein may use a hairpin or a loop adaptor on one end of the DNA product and a linear adaptor comprising protected nucleotides on the other end of the DNA product. That is to say that any type of adaptors described herein may be used as long as the final DNA product is protected from nuclease (e.g. exonuclease) digestion. The methods of the invention provide protection from digestion by exonucleases that cleave the 3'-end nucleotides (e.g. exonuclease III) and exonucleases that cleave the 5'-end nucleotides (e.g. exonuclease VIII). Thus, the linear DNA product produced by the methods of the invention has prolonged in vivo expression when compared to a linear DNA product that does not comprise an adaptor molecule described herein.

The use of the intermediate adaptor molecules, that are appended sequentially, that is to say each intermediate adaptor molecule is appended to an adjacent intermediate adaptor molecule or to the first or second terminal adaptor molecule, gives greater flexibility to the structure, function and/or length of the linear DNA product. For example, the intermediate adaptor molecules may comprise a label, a targeting sequence, a coding sequence, or may be used to provide greater length to the DNA product.

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to nuclease digestion (e.g. exonuclease III digestion). For example, the adaptor molecules may comprise nuclease resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

As used herein, "terminal adaptor molecule" refers to an adaptor molecule that is appended to one end to the linear DNA region, directly or indirectly via one or more intermediate adaptor molecules, to form the end of the linear DNA product. A terminal adaptor molecule is nuclease-resistant once it is appended to the linear double-stranded region, directly or indirectly. A terminal adaptor molecule confers nuclease-resistance to the linear DNA product.

As used herein an "intermediate adaptor molecule" refers to an adaptor molecule that is appended to an end of another intermediate adaptor molecule or to an end of the linear double-stranded region by its first end, and has another intermediate adaptor molecule or a terminal adaptor molecule appended to its second end; that is, an intermediate adaptor molecule is hybridized or ligated to another molecule at both ends.

As used herein "sequentially", means one after the other. That is to say, the n intermediate adaptor molecules are appended end-to-end from the first end of the double-stranded DNA molecule such that the n-(n-1)th adaptor molecule is appended to the first end of the double-stranded DNA molecule, the nth adaptor is appended to the terminal adaptor molecule; and the m intermediate adaptor molecules are appended end-to-end from the second end of the double-stranded DNA molecule such that the m-(m-1)th adaptor molecule is appended to the first end of the double-stranded DNA molecule, the mth adaptor is appended to the terminal adaptor molecule. For example, in the case where n is two and m is one, the linear DNA molecule comprises, from one end to the other end, a first terminal adaptor molecule--n2 intermediate adaptor molecule--n1 intermediate adaptor molecule--double-stranded linear region--m1 intermediate adaptor molecule--second terminal adaptor molecule.

Each adaptor molecule may comprise a sequence at its first end that is compatible with a sequence of a second end of the next adaptor molecule to be sequentially appended or ligated. For example, when n=2, the first n intermediate adaptor will have a sequence at its second end that is complementary to a sequence at the first end of the linear double-stranded region. The second (or nth) intermediate adaptor molecule may comprise a sequence at its second end that is complementary to the sequence at the first end of the first n intermediate adaptor molecule and a sequence at its first end that is complementary to a sequence at an end of the first terminal adaptor molecule. When each adaptor molecule comprises a different complementary sequence, the adaptor molecules will be appended, or ligated, in sequence. The different complementary sequences may be generated by using TypeIIS restriction endonuclease that cuts at a target sequence at a separate location from the overhang that results from the cleaving by the endonuclease, e.g., BsaI. Up to 256 different 4 nucleotide overhangs can be created using typeIIS endonucleases such that the intermediate and terminal adaptor molecules sequentially and directionally append.

As used herein, "adjacent intermediate adaptor molecule" or "adjacent adaptor molecule" refers to an adaptor molecule that is appended or ligated directly to an end of another adaptor molecule.

The closed DNA product comprises n intermediate adaptor molecules between the first terminal adaptor molecule and the first end of the linear double stranded region, and comprises m intermediate adaptor molecules between the second terminal adaptor molecule and a second end of the linear double-stranded region, where n and m are each 0 or an integer of at least 1, and n+m is at least 1.

The first end of the linear double stranded region and the first terminal adaptor molecule may be at the 3' or 5' end of the closed linear DNA product. The first end of the linear double-stranded DNA product and the first terminal adaptor molecule may be upstream or downstream of the DNA sequence of the linear double-stranded region.

The linear DNA product (e.g. the closed linear DNA product) produced by the methods of the present invention has additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes the linear DNA product produced by the methods of the present invention particularly suitable for use in a pharmaceutical composition.

Unexpectedly, the present inventors have discovered a method for production of a linear DNA product of enhanced resistance to nuclease digestion which allows for efficient production of large quantities of the linear DNA product with enhanced resistance to exonuclease digestion together with the ability to add certain features to the linear DNA product. The large-scale manufacture of the product may be in a cell-free system, which results in the production of a pure sample comprising the linear DNA product substantively free of bacterial contaminants (e.g. remaining after cell lysis).

## 1. Methods for producing closed linear DNA product

The methods described herein may be used to produce a closed linear DNA product e.g. a covalently closed linear DNA product.

The invention provides a method for producing a closed linear DNA product, the method comprises:

(a) contacting a double-stranded DNA molecule with an endonuclease and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The step of contacting the double-stranded DNA molecule with the endonuclease and first and second terminal adaptor molecules is preferably performed in the presence of a ligase. Thus, the invention provides a method for producing a closed linear DNA product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

The linear double-stranded region may be the linear portion of the double-stranded DNA molecule.

The invention provides a method for producing a closed linear DNA product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the closed linear DNA product, wherein the closed linear DNA product comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

A closed linear DNA product has particular utility as a therapeutic agent (i.e. DNA therapeutic) which can be used to express a gene product in vivo. This is because its closed structure (e.g. covalently closed structure) prevents attack by enzymes such as exonucleases, leading to enhanced stability and longevity of gene expression as compared to "open" DNA molecules with exposed DNA ends. Linear double-stranded open-ended cassettes have been demonstrated to be inefficient with respect to gene expression when introduced into host tissue. This has been attributed to cassette instability due to the action of exonucleases in the extracellular space.

Sequestering DNA ends inside closed structures also has other advantages. The DNA ends are prevented from integrating with genomic DNA and so closed linear DNA products offer improved safety. In addition, the closed linear structure reduces concatamerisation of DNA products inside host cells and thus expression levels of the gene product can be regulated in a more sensitive manner.

The method of the invention may be used for production of DNA for in vitro expression in a host cell, for example, in DNA vaccines. DNA vaccines typically encode a modified form of an infectious organism's DNA. DNA vaccines are administered to a subject where they then express the selected protein of the infectious organism, initiating an immune response against that protein which is typically protective. DNA vaccines may also encode a tumour antigen in a cancer immunotherapy approach.

The method may produce other types of therapeutic DNA molecules e.g. those used in gene therapy. For example, such DNA molecules can be used to express a functional gene where a subject has a genetic disorder caused by a dysfunctional version of that gene. Examples of such diseases include sickle cell anaemia, cystic fibrosis, Huntington disease, Duchenne's Muscular Dystrophy, Haemophilia A, $\alpha$1-antitrypsin deficiency, primary ciliary dyskinesia, or respiratory distress syndrome of prematurity. Other diseases where gene therapy may be useful include metabolic diseases, respiratory diseases, inflammatory diseases, autoimmune, chronic and infectious diseases, including such disorders as AIDS, cancer, neurological diseases, cardiovascular disease, hypercholesterolemia, various blood disorders including various anaemias, thalassemia and haemophilia, and emphysema. For the treatment of solid tumours, genes encoding toxic peptides (i.e., chemotherapeutic agents such as ricin, diptheria toxin and cobra venom factor), tumour suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The addition of intermediate adaptor molecules allows for additional flexibility in the production of the closed linear DNA products of the invention. The intermediate adaptor molecules may allow for longer closed linear DNA products to be produced. The intermediate adaptor molecules allow for bar codes, promotor sequences, labels, poly A signals and/or open reading frames to be incorporated into the closed linear DNA product of the invention. An intermediate adaptor molecule may comprise a cassette. For example, if it is advantageous to include several genes of interest, or open reading frames, in the closed linear DNA product, intermediate adaptor molecules may comprise one or more cassettes, and the linear DNA region may comprise one or more cassettes. An intermediate adaptor molecule may comprise a label, a signalling sequence, a targeting sequence, modified nucleotides, or a binding moiety. An intermediate adaptor molecule may comprise a promotor, a UTR, a transcription factor binding site, or a terminator sequence. For example, the n or m intermediate adaptor molecules that are appended or ligated upstream of the linear double-stranded region may be used to add or change a promotor sequence, add or change UTRs. The m or n intermediate adaptor molecules appended or ligated downstream of the linear double-stranded region may be used to add polyA signals or sequences, terminators, change or add UTRs. n or m intermediate adaptor molecules may be used to add barcodes, fluorophores, modified nucleotides, upstream or downstream of the linear double-stranded region.

A modified nucleotide may be 2-MethoxyEthoxy A, 2-MethoxyEthoxy MeC, 2-MethoxyEthoxy G, 2-MethoxyEthoxy T, 2'-O-Methyl RNA Bases, Fluoro Bases, 2-Aminopurine, 5-Bromo dU, deoxyUridine, 2,6-Diaminopurine (2-Amino-dA), Dideoxy-C, deoxyInosine, Hydroxymethyl dC, Iso-dG, Iso-dC, 5-Methyl dC or 5-Nitroindole.

As well as providing nuclease-resistance to the closed linear DNA product, the terminal adaptor molecules may also provide further functional features. A terminal adaptor molecule may allow for longer closed linear DNA products to be produced. Terminal adaptor molecules may allow for bar codes, promotor sequences, labels, poly A signals and/or open reading frames to be incorporated into the closed linear DNA product of the invention. A terminal adaptor molecule may comprise a label, a signalling sequence, a targeting sequence or a binding moiety. A terminal adaptor molecule may comprise a UTR or a terminator sequence.

The closing of the linear double-stranded region by the first terminal adaptor molecule and/or the second terminal adaptor molecule (directly or indirectly via one or more intermediate adaptor molecules) may be performed by hybridization or ligation of the adaptor molecules to the ends of the linear double-stranded region and/or to sequential adaptor molecules. Thus, the n intermediate adaptor molecules may be sequentially hybridized to the first end of the linear double-stranded region. The m intermediate adaptor molecules may be sequentially hybridized to the second end of the linear double-stranded region. The n intermediate adaptor molecules may be sequentially ligated to the first end of the linear double-stranded region. The m intermediate adaptor molecules may be sequentially ligated to the second end of the linear double-stranded region. The appending of the n+m intermediate adaptor molecules, first terminal adaptor molecule and the second terminal adaptor molecule may be performed by both hybridization and ligation of the adaptor molecules to the ends of the linear double-stranded region and/or to adjacent adaptor molecules. Thus, n intermediate adaptor molecules may be sequentially hybridized and ligated to the first end of the linear double-stranded region. The m intermediate adaptor molecules may be sequentially hybridized and ligated to the second end of the linear double-stranded region. The appending may be performed via a linker or spacer molecule which facilitates joining of the adaptor molecule to the first and/or second end of the linear double-stranded region or to an adjacent adaptor molecule. The first terminal adaptor molecules may be hybridized, ligated or hybridized and ligated to the nth intermediate adaptor molecule or to the first end of the double-stranded DNA molecule. The second terminal adaptor molecule may be hybridized, ligated or hybridized and ligated to the mth intermediate adaptor molecule or the second end of the double stranded DNA molecule. n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The step of incubating the single contiguous aqueous volume to generate the closed linear DNA product may comprise generating the linear portion of the double-stranded DNA molecule by digesting the double-stranded DNA molecule with the endonuclease.

The step of incubating the single contiguous aqueous volume may be performed under conditions that promote appending (or linking) of the first and second terminal adaptor molecules and/or intermediate adaptor molecules to the linear double-stranded region to produce the closed linear DNA product. The appending may be performed by creating a covalent link between the first and/or second terminal adaptor molecule and the first and/or second end of the linear double-stranded region. The appending may be performed by creating a covalent link between the first and/or second terminal adaptor molecule and an nth and/or mth intermediate adaptor molecule. The appending may be performed by creating a covalent link between an intermediate adaptor molecule and the first or second end of the linear double-stranded region. The appending may be performed by creating a covalent link between an intermediate adaptor molecule and an adjacent adaptor molecule.

The step of incubating the single contiguous aqueous volume may be performed under conditions that promote digestion of the double-stranded DNA molecule to produce the linear portion of the double-stranded DNA molecule. The digestion of the double-stranded DNA molecule to produce the linear portion of the double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the linear double-stranded region to the first and second terminal adaptor molecules and/or to an intermediate adaptor molecules. The step of incubating the single contiguous aqueous volume may be performed under conditions that promote ligation of the intermediate adaptor molecules to the first and second terminal adaptor molecules and/or adjacent intermediate adaptor molecules and/or the first and/or second end of the linear double-stranded region. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the linear double-stranded regions (or the portions of the double-stranded DNA molecules) may be incorporated into closed linear DNA products. Preferably, the ligation is at least 15% efficient.

Ligation efficiency may be established based on DNA quantification values before and after the digestion/ligation reaction. Thus, ligation efficiency may be established based on the equation:

$$\text{(starting amplified DNA amount) / (final linear DNA amount)} \times 100\%.$$

Ligation efficiency may also be established based on DNA quantification values before and after the digestion/ligation reaction and the subsequent exonuclease treatment to remove remaining open DNA constructs and adaptor molecules excess.

For example, the double-stranded DNA molecule generated by the rolling circle amplification is first quantified so that the amount of the double-stranded DNA molecule used as the starting material during the digestion/ligation reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the linear double-stranded region to the first and second terminal adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C and the second temperature is 14°C-18°C. Using these conditions the endonuclease may be a Type IIS restriction endonuclease (e.g. BsaI) and the ligase may be T4 DNA ligase, T7 DNA ligase, mammalian DNA ligase I, III and IV; Taq DNA ligase, Tth DNA ligase, or *E. coli* DNA ligase.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the n + m intermediate adaptor molecules and/or first and second terminal adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The method may further comprise, before step (a) (i.e. the step of contacting the double-stranded DNA molecule with the endonuclease, the ligase and the first and second terminal adaptor molecules), a step of amplifying a DNA template molecule to produce the double-stranded DNA molecule. Thus, the invention provides a method for producing a closed linear DNA product, the method comprises:

(a) amplifying a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the closed linear DNA product, wherein the closed linear DNA product comprises a linear double-stranded region, wherein the linear double-stranded region comprises a linear portion of the double-stranded DNA molecule , and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

The step of amplifying may be performed by in vitro or in vivo amplification. Preferably, the step of amplifying is performed by in vitro amplification. For example, the step of amplifying may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the step of amplifying is performed by rolling circle amplification. Thus, the invention provides a method for producing a closed linear DNA product, the method comprises:

(a) amplifying a DNA template molecule comprising at least one cleavable (e.g. endonuclease) target sequence to generate a double-stranded DNA molecule, wherein the DNA template molecule is amplified by rolling circle amplification;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

In the methods described herein, the DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a BbsI, BsaI, BsmBI, BspQI, BtgZI, Esp3I, SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the closed linear DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a BbsI, BsaI, BsmBI, BspQI, BtgZI, Esp3I, SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or E. coli DNA ligase.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN proteolomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette.

The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The method may further comprise (after the step of amplification and before the step of contacting the double-stranded DNA molecule with an endonuclease, a ligase and first and second terminal adaptor molecules) a step of heat-deactivation. Thus, the invention provides a method for producing a closed linear DNA product, the method comprises:

(a) amplification of a DNA template molecule comprising at least one cleavable (e.g. endonuclease) target sequence to generate a double-stranded DNA molecule;
(b) heat-deactivation of the reaction of step (a);
(c) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(d) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

Preferably, amplification is rolling-circle amplification.

The step of heat-deactivation may be performed under conditions sufficient to inactive the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

In the method described herein, after the step of amplification, the step of contacting the double-stranded DNA molecule with an endonuclease, a ligase and first and second terminal adaptor molecules and n+m intermediate adaptor molecules, to form a single contiguous aqueous volume, may be performed without purifying the product of the amplification reaction. That is to say that the step of contacting the double-stranded DNA molecule with an endonuclease, a ligase and first and second terminal adaptor molecules and n+m intermediate adaptor molecules may be performed directly after the step of amplification. The step of contacting the double-stranded DNA molecule with an endonuclease, a ligase and first and second terminal adaptor molecules and nm intermediate adaptor molecules may be performed directly after the step of heat-deactivation.

The method may further comprise, after the step of incubating the single contiguous aqueous volume, a step of purification of the closed linear DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purification. This step allows for removal of any double-stranded DNA molecules and/or adaptor molecules which have not been used in the course of performing the method. Thus, the method may comprise the steps:

(a) amplifying a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(c) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region; and
(d) incubating the single contiguous aqueous volume with a nuclease (e.g. exonuclease).

In the method described herein, after the step of amplification, the step of contacting the double-stranded DNA molecule with an endonuclease, a ligase and first and second terminal adaptor molecules and n+m intermediate adaptor molecules, to form a single contiguous aqueous volume, may be performed without purifying the product of the amplification reaction. That is to say that the step of contacting the double-stranded DNA molecule with an endonuclease, a ligase and first and second terminal adaptor molecules and n+m intermediate adaptor molecules may be performed directly after the step of amplification. The step of contacting the double-stranded DNA molecule with an endonuclease, a ligase and first and second terminal adaptor molecules and n+m intermediate adaptor molecules may be performed directly after the step of heat-deactivation.

The method may comprise the steps:

(a) amplifying a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(c) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region;
(d) purifying the closed linear DNA product; and
(e) incubating the purified product of step (d) with a nuclease (e.g. exonuclease).

The method may comprise the steps:

(a) amplifying a DNA template molecule comprising at least one cleavable (e.g. endonuclease) target sequence to generate a double-stranded DNA molecule;
(b) heat-deactivation of the reaction of step (a);
(c) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(d) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region;

(e) purifying the closed linear DNA product; and

(f) incubating the purified product of step (d) with a nuclease (e.g. exonuclease).

The method may comprise the steps:

(a) amplifying a DNA template molecule comprising at least one cleavable (e.g. endonuclease) target sequence to generate a double-stranded DNA molecule;

(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;

(c) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region;

(d) incubating the single contiguous aqueous volume with a nuclease (e.g. exonuclease); and

(e) purifying the closed linear DNA product.

The method may comprise the steps:

(a) amplifying a DNA template molecule comprising at least one cleavable (e.g. endonuclease) target sequence to generate a double-stranded DNA molecule;

(b) heat-deactivation of the reaction of step (a);

(c) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;

(d) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region;

(e) incubating the single contiguous aqueous volume with a nuclease (e.g. exonuclease); and

(f) purifying the closed linear DNA product.

The step of incubating the single contiguous aqueous volume (or the purified product of step (d)) with a nuclease may be performed at a temperature of 5-90°C, 10-80°C, 15-70°C, 20-60°C, 25-50°C, 30-45°C or 35-40°C. The step of incubating the single contiguous aqueous volume (or the purified product of step (d)) with a nuclease may be performed for at least 10, at least 20, at least 30, at least 40, at least 50, or at least 60 min. The step of incubating the single contiguous aqueous volume (or the purified product of step (d)) may be performed at two different temperatures. For example, the step of incubating the single contiguous aqueous volume (or the purified product of step (d)) may be performed at 15-40°C for 10-60 minutes followed by a temperature of 60-90°C for 10-30 min. The higher temperature typically inactivates the nuclease (e.g. exonuclease). Thus, the method further provides a step of inactivating the nuclease (e.g. exonuclease). The step of incubating the single contiguous aqueous volume (or the purified product of step (d)) may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The method may be a cell-free method.

The closed linear DNA product may be partially double-stranded and/or partially single-stranded. The closed linear DNA product may comprise a portion that is double-stranded and a portion that is single-stranded.

The closed linear DNA product may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The closed linear DNA product may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long.

The closed linear DNA product may comprise an inverted terminal repeat sequence.

The closed linear DNA product may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the closed linear DNA product is at least 50 base pairs long.

The double-stranded DNA molecule may be circular, or branched.

The double-stranded DNA molecule may not comprise an adaptor. The double-stranded DNA molecule may not comprise a hairpin, a loop or a stem-loop structure.

The double-stranded DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule.

The double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may comprise one or more cleavable (e.g. endonuclease) target sequences. The double-stranded DNA molecule may comprise two cleavable (e.g. endonuclease) target sequences. The one or more cleavable (e.g. endonuclease) target sequences may be Type IIS endonuclease target sequences. The one or more cleavable (e.g. endonuclease) target sequences may be BbsI, BsaI, BsmBI, BspQI, BtgZI, Esp3I, SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequences.

The double-stranded DNA molecule may be a product of amplification. Preferably, the amplification is rolling circle amplification.

The linear double-stranded region (e.g. the linear portion of the double-stranded molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The linear double-stranded region (e.g. the linear portion of the double-stranded molecule) may comprise a sequence that is at least 40%, 45%, 50%, 55%, 60%, 65%, 70%, 75%, 80%, 85%, 90%, 95%, or 100% identical to the sequence of the double-stranded DNA molecule.

The first end and the second end of the linear double-stranded region (e.g. the linear portion of the double-stranded molecule) may be resistant to nuclease digestion. Preferably, the first end and the second end of the linear double-stranded region are resistant to the exonuclease digestion, such as exonuclease III digestion and/or exonuclease I digestion.

The linear double-stranded region may comprise a 3'-OH group at first and/or second ends. The 3'-OH group may facilitate ligation to the first and/or second terminal adaptor molecule(s) and/or n+m intermediate adaptor molecules (which may comprise a 5' phosphate). The linear double-stranded region may comprise a 5' phosphate at first and/or second ends. The 5' phosphate may facilitate ligation to the first and/or second terminal adaptor molecule(s) (which may comprise a 3'-OH group).

The linear double-stranded region (e.g. the linear portion of the double-stranded molecule) may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the sense strand or the antisense strand of the linear double-stranded region.

The linear portion of the double-stranded DNA molecule (e.g. the linear portion of the double-stranded molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, double-stranded DNA molecule is at least 50 base pairs long.

The first terminal adaptor molecule and/or the second terminal adaptor molecule and/or one or more or n+m of the n+m intermediate adaptor molecules may be a synthetic adaptor molecule.

The first terminal adaptor molecule may be a nucleic acid adaptor molecule. The second terminal adaptor molecule may be a nucleic acid adaptor molecule. One or more or n+m of the n+m intermediate adaptor molecules may be a nucleic acid adaptor molecule. The first terminal adaptor molecule and/or the second terminal adaptor molecule may comprise a self-complementary element which creates a loop, such as a hairpin loop or a stem loop. Thus, the first terminal adaptor molecule may comprise a hairpin or a stem-loop. The second terminal adaptor molecule may comprise a hairpin or a stem-loop. Both the first and second terminal adaptor molecules may comprise a hairpin or a stem-loop. The terminal or intermediate adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (or linear portion of the double-stranded DNA molecule) may comprise a 3' or a 5' overhang.

A portion of the first terminal adaptor molecule (e.g. the overhang) may be complementary to the first end of the linear double-stranded region or complementary to a first end of an intermediate adaptor molecule, preferably the nth intermediate adaptor molecule. A portion of the second terminal adaptor molecule (e.g. the overhang) may be complementary to the second end of the linear double-stranded region or complementary to a first end of an intermediate adaptor molecule, preferably the mth adaptor molecule. A portion of the first end of the linear double-stranded region maybe complementary to a first end of an intermediate adaptor molecule, preferably n-(n-1) intermediate adaptor molecule. A portion of the second end of the linear double-stranded region maybe complementary to a first end of an intermediate adaptor molecule, preferably m-(m-1) intermediate adaptor molecule. A portion of a second end of an intermediate adaptor molecule may be complementary to a first end of an adjacent intermediate adaptor molecule, for example a portion of the second end of the nth intermediate adaptor molecule may be complementary to a portion of the first end of the n-1th intermediate adaptor molecule, and a portion of the second end of the n-1th adaptor molecule may be complementary to a portion of the first end of the n-2th intermediate adaptor molecule, and so on. A portion of a second end of an intermediate adaptor molecule may be complementary to a first end of an adjacent intermediate adaptor molecule, for example a portion of the second end of the mth intermediate adaptor molecule may be complementary to a portion of the first end of the m-1th intermediate adaptor molecule, and a portion of the second end of the m-1th adaptor molecule may be complementary to a portion of the first end of the m-2th intermediate adaptor molecule, and so on. n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The closed linear DNA product may be a covalently closed linear DNA product. Thus, in embodiments where the terminal adaptor molecules comprise a loop (e.g. a hairpin), the terminal adaptor molecules close the ends of the linear double-stranded region (or close the ends of the nth and mth intermediate adaptor molecules) forming a covalently closed linear DNA product.

The first and/or second terminal adaptor molecule may comprise or consist of the sequence of SEQ ID NO: 9 or a portion thereof. The first and/or second terminal adaptor molecule may comprise at least 13, at least 14, at least 15, at least 16, at least 17, at least 18, or at least 19 contiguous nucleotides of SEQ ID NO: 9. The double-stranded portion of the first and/or second terminal adaptor molecule may comprise the sequence of SEQ ID NO: 10 or a portion thereof. The double-stranded portion of the first and/or second terminal adaptor molecule may comprise at least 8, at least 9, at least 10, at least 11, at least 12, at least 13, at least 14 or at least 15 contiguous nucleotides of SEQ ID NO: 10. The single-stranded portion of the first and/or second terminal adaptor molecule may comprise a sequence of ACTCA. The single-stranded portion of the first terminal adaptor molecule and/or the second terminal adaptor molecule may comprise at least 1, at least 2, at least 3, at least 4 or at least 5 contiguous nucleotides of the sequence ACTCA. The first and/or second terminal adaptor molecule may comprise the sequence of SEQ ID NO: 12. The first and/or second adaptor molecule may comprise at least 13, at least 14, at least 15, at least 16, at least 17, at least 18, or at least 19 contiguous nucleotides of SEQ ID NO:12.

The invention provides a method for producing a covalently closed linear DNA product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1, wherein the first and second terminal adaptor molecules are nucleic acid adaptor molecules that each comprise a hairpin; and

(b) incubating the single contiguous aqueous volume to generate the covalently closed linear DNA product, wherein the covalently closed linear DNA product comprises a linear double-stranded region, wherein the linear double-stranded region comprises a linear portion of the double-stranded DNA molecule, wherein n intermediate adaptor molecules are sequentially appended to a first end of the linear double stranded region and m intermediate adaptor molecules are sequentially appended to a second end of the linear double-stranded region, wherein the closed linear DNA product is closed at a first end by the first terminal adaptor molecule appended to the nth intermediate adaptor molecule or, when n is 0, to the first end of the linear double stranded region, and closed at a second end by the second terminal adaptor molecule appended to the mth intermediate adaptor molecule, or when m is 0, to the second end of the linear double stranded region, wherein (i) the first terminal adaptor molecule comprises an overhang that is complementary to and anneals to an overhang at the first end of the linear double-stranded region or the nth intermediate adaptor molecule thereby closing the first end of the linear double-stranded region and (ii) the second terminal adaptor molecule comprises an overhang that is complementary to and anneals to an overhang at the second end of the linear double-stranded region or the mth intermediate adaptor molecule thereby closing the second end of the linear double-stranded region.

The invention provides a method for producing a covalently closed linear DNA product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1, wherein the first and second terminal adaptor molecules are nucleic acid adaptor molecules that each comprise a hairpin; and

(b) incubating the single contiguous aqueous volume to generate the covalently closed linear DNA product, wherein the covalently closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region, wherein (i) the first terminal adaptor molecule comprises an overhang that is complementary to and anneals to an overhang at the first end of the linear double-stranded region or to the nth intermediate adaptor molecule thereby closing the first end of the linear double-stranded region and (ii) the second terminal adaptor molecule comprises an overhang that is complementary to and anneals to an overhang at the second end of the linear double-stranded region or to the mth intermediate adaptor molecule thereby closing the second end of the linear double-stranded region, and wherein the first terminal adaptor molecule is ligated to the first end of the linear double-stranded region and the second terminal adaptor molecule is ligated to the second end of the linear double-stranded region.

The invention provides a method for producing a covalently closed linear DNA product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1, wherein the first and second terminal adaptor molecules are nucleic acid adaptor molecules that each comprise a hairpin; and

(b) incubating the single contiguous aqueous volume to generate the covalently closed linear DNA product, wherein the covalently closed linear DNA product comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region, wherein (i) the first terminal adaptor molecule comprises an overhang that is complementary to and anneals to an overhang at the first end of the linear portion of the double-stranded DNA molecule or to the nth intermediate adaptor molecule, thereby closing the first end of the linear portion of the double-stranded DNA molecule and (ii) the second terminal adaptor molecule comprises an overhang that is complementary to and anneals to an overhang at the second end of the linear portion of the double-stranded DNA molecule or to the mth intermediate adaptor molecule, thereby closing the second end of the linear portion of the double-stranded DNA molecule, and wherein the first terminal adaptor molecule is ligated to the first end of the linear portion of the double-stranded DNA molecule or to the nth intermediate adaptor molecule, and the second terminal adaptor molecule is ligated to the second end of the linear portion of the double-stranded DNA molecule or to the mth intermediate adaptor molecule. n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The first terminal adaptor molecule and/or the second terminal adaptor molecule and/or the n+m intermediate adaptor molecules may not be a plasmid or a vector DNA.

The first terminal adaptor molecule and/or the second terminal adaptor molecule may comprise a single-stranded portion. The single-stranded portion may form a hairpin or a stem-loop. Thus, the first terminal adaptor molecule and/or the second terminal adaptor molecule may comprise a loop portion.

The single-stranded portion may comprise less than 10, 9, 8, 7, 6, 5, 4, 3, 2 nucleotides. Preferably, the single-stranded portion comprises 5 nucleotides.

The first terminal adaptor molecule and/or the second terminal adaptor molecule and/or one or more of the n+m intermediate adaptor molecules may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first terminal adaptor molecule and/or the second terminal adaptor molecule and/or one or more of the n+m intermediate adaptor molecules may comprise a 5' phosphate. The 5' phosphate may facilitate ligation to the linear double-stranded region or to adjacent terminal or intermediate adaptor molecules (which may comprise a 3'-OH group at first and/or second ends). The first terminal adaptor molecule and/or the second terminal adaptor molecule and/or one or more of the n+m intermediate adaptor molecules may comprise a 3'-OH. The 3'-OH may facilitate ligation to the linear double-stranded region and/or to adjacent intermediate adaptor molecules (which may comprise a 5' phosphate at first and/or second ends).

The first terminal adaptor molecule and/or the second terminal adaptor molecule may comprise the sequence of SEQ ID NO: 1 or a portion thereof. The first terminal adaptor molecule and/or the second terminal adaptor molecule may comprise at least 13, at least 14, at least 15, at least 16, at least 17, at least 18, or at least 19 contiguous nucleotides of SEQ ID NO: 1. The double-stranded portion of the first terminal adaptor molecule and/or the second terminal adaptor molecule may comprise the sequence of SEQ ID NO: 2 or a portion thereof. The double-stranded portion of the first terminal adaptor molecule and/or the second terminal adaptor molecule may comprise at least 8, at least 9, at least 10, at least 11, at least 12, at least 13, at least 14 or at least 15 contiguous nucleotides of SEQ ID NO: 2. The single-stranded portion of the first terminal adaptor molecule and/or the second terminal adaptor molecule may comprise a sequence of ACTCA. The single-stranded portion of the first terminal adaptor molecule and/or the second terminal adaptor molecule may comprise at least 1, at least 2, at least 3, at least 4 or at least 5 contiguous nucleotides of the sequence ACTCA. The first and second adapter molecules may comprise an identical nucleic acid sequence. The first and second adapter molecules may comprise a different nucleic acid sequence.

The first terminal adaptor molecule may comprise a portion that is complementary to the first end of the linear double-stranded region (or the linear portion of the double-stranded DNA molecule). The second terminal adaptor molecule may comprise a portion that is complementary to the second end of the linear double-stranded region (or the linear portion of the double-stranded DNA molecule). The first terminal adaptor molecule may comprise a portion that anneals to the first end of the linear double-stranded region (or the linear portion of the double-stranded DNA molecule). The second terminal adaptor molecule may comprise a portion that anneals to the second end of the linear double-stranded region (or the linear portion of the double-stranded DNA molecule). The first terminal adaptor molecule may comprise a portion that is complementary and anneals to the first end of the linear double-stranded region (or the linear portion of the double-stranded DNA molecule). The second terminal adaptor molecule may comprise a portion that is complementary and anneals to the second end of the linear double-stranded region (or the linear portion of the double-stranded DNA molecule).

The portion that is complementary or anneals to the first or second end of the linear double-stranded region (or the linear portion of the double-stranded DNA molecule) may be a 5' overhang or a 3' overhang of the first and/or second terminal adaptor molecule. The overhang of the first terminal adaptor molecule may be complementary to the first end of the linear double-stranded region and/or the overhang of the second terminal adaptor molecule may be complementary to the second end of the double-stranded region. The overhang of the first terminal adaptor molecule may anneal to the first end of the linear double-stranded region and/or the overhang of the second terminal adaptor molecule may anneal to the second end of the linear double-stranded region. The overhang of the first terminal adaptor molecule may be complementary to and anneal to the first end of the linear double-stranded region and/or the overhang of the second terminal adaptor molecule may be complementary to and anneal to the second end of the linear double-stranded region.

The first terminal adaptor molecule may comprise a portion that is complementary to the first end of the nth intermediate adaptor molecule. The second terminal adaptor molecule may comprise a portion that is complementary to the first end of the mth intermediate adaptor molecule. The first terminal adaptor molecule may comprise a portion that anneals to the first end of the nth intermediate adaptor molecule. The second terminal adaptor molecule may comprise a portion that anneals to first end of the mth intermediate adaptor molecule. The first terminal adaptor molecule may comprise a portion that is complementary and anneals to first end of the nth intermediate adaptor molecule. The second terminal adaptor molecule may comprise a portion that is complementary and anneals to first end of the mth intermediate adaptor molecule.

The portion that is complementary or anneals to the first end of the nth or mth intermediate adaptor molecule may be a 5' overhang or a 3' overhang of the first and/or second terminal adaptor molecule. The overhang of the first terminal adaptor molecule may be complementary to the first end of the nth intermediate adaptor molecule and/or the overhang of the second terminal adaptor molecule may be complementary to first end of the mth intermediate adaptor molecule. The overhang of the first terminal adaptor molecule may anneal to first end of the nth intermediate adaptor molecule and/or the overhang of the second terminal adaptor molecule may anneal to first end of the mth intermediate adaptor molecule. The overhang of the first terminal adaptor molecule may be complementary to and anneal to first end of the nth intermediate adaptor molecule and/or the overhang of the second terminal adaptor molecule may be complementary to and anneal to first end of the mth intermediate adaptor molecule.

The first terminal adaptor molecule and/or the second terminal adaptor molecule and/or one or more of the n+m intermediate adaptor molecules may not comprise a Type IIS endonuclease target sequence. The first terminal adaptor molecule and/or the second terminal adaptor molecule may not comprise BbsI, BsaI, BsmBI, BspQI, BtgZI, Esp3I,SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequences.

The first terminal adaptor molecule and/or the second terminal adaptor molecule and/or one or more of the n+m intermediate adaptor molecules may comprise one or more locked nucleic acids (LNAs).

The first terminal adaptor molecule and/or the second terminal adaptor molecule and/or one or more of the n+m intermediate adaptor molecules may comprise one or more protected nucleotides (i.e. nuclease-resistant nucleotides), such as phosphorothioated nucleotides. The protected nucleotides may be located in the single-stranded portion (e.g. hairpin portion) of the terminal adaptor molecules or the double-stranded portion of the terminal or intermediate adaptor molecules. The protected nucleotides may be located in the overhang portion of the terminal or intermediate adaptor molecules.

The closed linear DNA product may comprise a plurality of phosphorothioated nucleotides at internal positions in each strand. For example, the closed linear DNA product may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions in each strand. Preferably, the closed linear DNA product comprises at least 2 protected nucleotides (e.g. phosphorothioated nucleotides) at internal positions in each strand.

The terminal and/or intermediate adaptor molecules may comprise at least two types of phosphorothioated nucleotides. For example, the at least two types of phosphorothioated nucleotides are: $\alpha$-S-dATP and $\alpha$-S-dCTP, $\alpha$-S-dATP and $\alpha$-S-dGTP, $\alpha$-S-dATP and $\alpha$-S-dTTP, $\alpha$-S-dCTP and $\alpha$-S-dGTP, $\alpha$-S-dCTP and $\alpha$-S-dTTP, or $\alpha$-S-dGTP and $\alpha$-S-dTTP.

The terminal and/or intermediate adaptor molecules may comprise at least three types of phosphorothioated nucleotides. For example, the at least three types of phosphorothioated nucleotides are:

(a) $\alpha$-S-dATP, $\alpha$-S-dCTP and $\alpha$-S-dGTP;
(b) $\alpha$-S-dATP, $\alpha$-S-dCTP and $\alpha$-S-dTTP;
(c) $\alpha$-S-dATP, $\alpha$-S-dGTP and $\alpha$-S-dTTP; or
(d) $\alpha$-S-dCTP, $\alpha$-S-dGTP and $\alpha$-S-dTTP.

The terminal and/or intermediate adaptor molecules may comprise at least four types of phosphorothioated nucleotides. For example, the at least four types of protected nucleotides are $\alpha$-S-dATP, $\alpha$-S-dCTP, $\alpha$-S-dGTP and $\alpha$-S-dTTP

The internal positions may not be located between the second and penultimate nucleotide of the closed linear DNA product.

The linear double-stranded region (or linear portion of the double-stranded molecule) may comprise a plurality of phosphorothioated nucleotides at internal positions in each strand. For example, the linear double-stranded region (or linear portion of the double-stranded molecule) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) at internal positions in each strand. Preferably, the linear double-stranded region (or linear portion of the double-stranded molecule) comprises at least 2 protected nucleotides (e.g. phosphorothioated nucleotides) at internal positions in each strand. The internal positions may not be located between the second and penultimate nucleotide of the linear double-stranded region (or linear portion of the double-stranded molecule).

One or more of the n+m intermediate adaptor molecules may comprise a plurality of phosphorothioated nucleotides at internal positions in each strand. For example, the one or more of the n+m intermediate adaptor molecules may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) at internal positions in each strand.

The nucleotides resistant to exonuclease digestion (i.e. protected nucleotides) suitable for use in the methods described herein may be phosphorothioated nucleotides. For example, phosphorothioated nucleotides may be $\alpha$-S-dATP (i.e. 2'-deoxyadenosine-5'-(a-thio)-triphosphate), $\alpha$-S-dCTP (i.e. 2'-deoxycytidine-5'-(a-thio)-triphosphate), $\alpha$-S-dGTP (i.e. 2'-deoxyguanosine-5'-(a-thio)-triphosphate), $\alpha$-S-dTTP (i.e. 2'-deoxythymidine-5'-($\alpha$-thio)-triphosphate), $\alpha$-S-dUTP (i.e. 2'-deoxyuridine-5'-($\alpha$-thio)-triphosphate), and/or uridine 2', 3'-cyclophosphorothioate.

The phosphorothioated nucleotides may be Sp-isomers, Rp-isomers or a mixture of both Sp- and Rp-isomers.

The nucleotides resistant to exonuclease digestion (i.e. protected nucleotides) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first end of the linear double-stranded region may be complementary to a portion of the first terminal adaptor molecule or to a portion of an intermediate adaptor molecule, preferably the n-(n-1)th adaptor molecule. The second end of the linear double-stranded region may be complementary to a portion of the second terminal adaptor molecule or to a portion of an intermediate adaptor molecule, preferably the m-(m-1)th adaptor molecule. The first end and/or the second end of the linear double-stranded region may be generated by endonuclease digestion.

The first terminal adaptor molecule and/or the second terminal adaptor molecule and/or one or more of the n+m intermediate adaptor molecules may comprise a functional portion. The functional portion may be a binding molecule, a targeting sequence, a signal sequence or a probe. The functional portion may be a cassette, an open reading frame or a coding sequence. The functional portion may be a promotor, and enhancer, NLS sequence, modified nucleotides, a terminator, a transcription factor binding cite, a bar code or a fluorophore.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probetarget base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the closed linear DNA product. For example, the targeting sequence may be a DNA nuclear targeting sequences (i.e. a recognition sequence for endogenous DNA-binding proteins), such as SV40 enhancer sequence (preferably downstream from the cassette). The targeting sequence may be a protelomerase targeting sequence.

To facilitate detection and/or quantification of the DNA product, the functional portion may comprise a fluorophore, a radioactive compound or a barcode.

A signal corresponding to the presence, absence and/or level of the closed linear DNA product may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the closed linear DNA product. The binding moiety is capable of binding to 3' and/or 5' end of the closed linear DNA product. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the closed linear DNA product and the second binding moiety linked to the second barcoded portion may bind to the 5' end of the closed linear DNA product. The 3' and 5' ends may comprise a 3' overhang, a 5' overhang or a blunt end.

A signal corresponding to the presence, absence and/or level of the closed linear DNA product may be measured using a fluorophore (i.e. a fluorescently-labelled molecule), which is attached or bound to the 3' overhang, the 5' overhang or the blunt end of the closed linear DNA product. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina® (e.g. the HiSeq™, MiSeq™ and/or Genome Analyzer™ sequencing systems), Oxford Nanopore™ Technologies (e.g. the MinION sequencing system), Ion Torrent™ (e.g. the Ion PGM™ and/or Ion Proton™ sequencing systems), Pacific Biosciences (e.g. the PACBIO RS II sequencing system); Life Technologies™ (e.g. a SOLiD sequencing system), Roche (e.g. the 454 GS FLX+ and/or GS Junior sequencing systems), or any other sequencing platform of interest.

The first terminal adaptor molecule and/or the second terminal adaptor molecule and/or one or more of the n+m intermediate adaptor molecules may comprise an inverted terminal repeat sequence. The inverted terminal repeat sequences of the first terminal adaptor molecule and the second terminal adaptor molecule may be symmetrical (i.e. have the same symmetrical three-dimensional organization with respect to each other) or asymmetrical (i.e. have different three-dimensional organization with respect to each other). The inverted terminal repeat sequences of the first terminal adaptor molecule and the second terminal adaptor molecule and/or one or more of the n+m intermediate adaptor molecules may be from the same or different serotypes. An inverted terminal repeat sequence may comprise a terminal resolution site and a Rep binding site.

One or more of the n+m intermediate adaptor molecules (e.g. 1, 2, 3, 4, 5, 6, 7 or 8 intermediate adaptor molecules) may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

One or more of the n+m intermediate adaptor molecules (e.g. 1, 2, 3, 4, 5, 6, 7 or 8 intermediate adaptor molecules) may comprise a spacer. The spacer may be at least 10, at least 20, at least 30, at least 40, at least 50, at least 60, at least 70, at least 80, at least 90, at least 100, at least 125, at least 150, at least 175, at least 200, or at least 300 base pairs long.

One or more of the n+m intermediate adaptor molecules (e.g. 1, 2, 3, 4, 5, 6, 7 or 8 intermediate adaptor molecules) may be at least, 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the one or more of the n+m intermediate adaptor molecules (e.g. 1, 2, 3, 4, 5, 6, 7 or 8 intermediate adaptor molecules) is at least 10 base pairs long. A terminator adaptor molecule may be may be at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, at least 50, at least 100 base pairs long.

The first terminal adaptor molecule and/or the second terminal adaptor molecule and/or one or more of the n+m intermediate adaptor molecules may comprise a polyA signal sequence.

The first terminal adaptor molecule and/or the second terminal adaptor molecule and/or one or more of the n+m intermediate adaptor molecules may comprise an aptamer.

The first terminal adaptor molecule and/or the second terminal adaptor molecule and/or one or more of the n+m intermediate adaptor molecules may confer resistance to the nuclease digestion, such as exonuclease digestion (e.g. exonuclease I and/or exonuclease III digestion).

The closing of the linear double-stranded region (or the linear portion of the double-stranded DNA molecule) at the first end may generate a first closed end of the closed linear DNA product. The closing of the linear double-stranded region (or the linear portion of the double-stranded DNA molecule) at the second end may generate a second closed end of the closed linear DNA product. The first closed end and the second closed end of the closed linear DNA product may be resistant to nuclease digestion. The nuclease digestion may be exonuclease digestion. Preferably, the nuclease digestion is exonuclease III digestion and/or exonuclease I digestion.

## 2. Methods for producing a linear DNA product comprising nuclease-resistant nucleotides

The methods described herein may be used to produce a linear DNA product comprising nuclease-resistant (i.e. protected nucleotides).

The invention provides a method for producing a linear deoxyribonucleic acid (DNA) product, wherein the method comprises:

(a) contacting a double-stranded DNA molecule with an endonuclease and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to a first end of the linear double-stranded region and the second terminal adaptor molecule is appended to a second end of the linear double-stranded region, and wherein the first and second terminal adaptor molecules are nucleic acid molecules that comprise one or more nuclease-resistant nucleotides (i.e. protected nucleotides).

The step of contacting the double-stranded DNA molecule with the endonuclease and first and second terminal adaptor molecules is preferably performed in the presence of a ligase. Thus, the method for producing a linear DNA product may comprise the steps:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to a first end of the linear double-stranded region and the second terminal adaptor molecule is appended to a second end of the linear double-stranded region, and wherein the first and second terminal adaptor molecules are nucleic acid molecules that comprise one or more nuclease-resistant nucleotides (i.e. protected nucleotides).

The linear DNA product produced by the methods described herein has enhanced resistance to nuclease (e.g. exonuclease) digestion. For example, the linear DNA product has prolonged in vivo expression when compared to a linear DNA product that does not contain protected nucleotides.

The addition of intermediate adaptor molecules allows for additional flexibility in the production of the linear DNA products of the invention. The intermediate adaptor molecules may allow for longer linear DNA products to be produced. The intermediate adaptor molecules allow for bar codes, promotor sequences, labels, poly A signals and/or open reading frames to be incorporated into the linear DNA product of the invention. An intermediate adaptor molecule may comprise a cassette. For example, if it is advantageous to include several genes of interest, or open reading frames, in the linear DNA product, intermediate adaptor molecules may comprise one or more cassettes, and the linear double-stranded region may comprise one or more cassettes. An intermediate adaptor molecule may comprise a label, a signalling sequence, a targeting sequence, a modified nucleotide, or a binding moiety. An intermediate adaptor molecule may comprise a promotor, a UTR, a transcription factor binding site, or a terminator sequence.

A modified nucleotide may be 2-MethoxyEthoxy A, 2-MethoxyEthoxy MeC, 2-MethoxyEthoxy G, 2-MethoxyEthoxy T, 2'-O-Methyl RNA Bases, Fluoro Bases, 2-Aminopurine, 5-Bromo dU, deoxyUridine, 2,6-Diaminopurine (2-Amino-dA), Dideoxy-C, deoxylnosine, Hydroxymethyl dC, Iso-dG, Iso-dC, 5-Methyl dC or 5-Nitroindole.

As well as providing nuclease-resistance to the linear DNA product, the terminal adaptor molecules may also provide further functional features. A terminator adaptor molecule may allow for longer linear DNA products to be produced. Terminator adaptor molecules may allow for bar codes, promotor sequences, labels, poly A signals and/or open reading frames to be incorporated into the linear DNA product of the invention. A terminal adaptor molecule may comprise a label, a signalling sequence (e.g. a NLS), a targeting sequence or a binding moiety. A terminal adaptor molecule may comprise a promotor, a UTR or a terminator sequence.

The appending of the first terminal adaptor molecule and/or the second terminal adaptor molecule to the first and/or second end of the linear double-stranded region (directly or indirectly via one or more intermediate adaptor molecules) may be performed by hybridization or ligation of the adaptor molecules to the ends of the linear double-stranded region and/or to sequential adaptor molecules. Thus, the n intermediate adaptor molecules may be sequentially hybridized to the first end of the linear double-stranded region. The m intermediate adaptor molecules may be sequentially hybridized to the second end of the linear double-stranded region. The n intermediate adaptor molecules may be sequentially ligated to the first end of the linear double-stranded region. The m intermediate adaptor molecules may be sequentially ligated to the second end of the linear double-stranded region. The appending of the n+m intermediate adaptor molecules, first terminal adaptor molecule and the second terminal adaptor molecule may be performed by both hybridization and ligation of the adaptor molecules to the ends of the linear double-stranded region and/or to adjacent adaptor molecules. Thus, n intermediate adaptor molecules may be sequentially hybridized and ligated to the first end of the linear double-stranded region. The m intermediate adaptor molecules may be sequentially hybridized and ligated to the second end of the linear double-stranded region. The appending may be performed via a linker or spacer molecule which facilitates joining of the adaptor molecule to the first and/or second end of the linear double-stranded region or to an adjacent adaptor molecule. The first terminal adaptor molecules may be hybridized, ligated or hybridized and ligated to the nth intermediate adaptor molecule or to the first end of the double-stranded DNA molecule. The second terminal adaptor molecule may hybridized, ligated or hybridized and ligated to the mth intermediate adaptor molecule or the second end of the double stranded DNA molecule. The hybridization is based on complementarity of a portion of the first and/or second terminal adaptor molecules and/or a portion of the first and/or second ends of the n+m intermediate adaptor molecules to the first and/or second end of the linear double-stranded region.

The method for producing a linear DNA product may comprise the steps:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides).

The method for producing a linear DNA product may comprise the steps:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the linear DNA product, wherein the linear DNA product comprises a linear portion of the double-stranded DNA molecule, and wherein n intermediate adaptor molecules are sequentially ligated to a first end of the linear double stranded region and m intermediate adaptor molecules are sequentially ligated to a second end of the linear double-stranded region, and wherein the first terminal adaptor molecule is ligated to the nth intermediate adaptor molecule or, when n is 0, to the first end of the linear double stranded region, and the second terminal adaptor molecule is ligated to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides). n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

Preferably, the step of contacting the double-stranded DNA molecule with the endonuclease, the ligase and first and second terminal adaptor molecules is performed in a single reaction (i.e. a single step).

The step of incubating the single contiguous aqueous volume to generate the linear DNA product may comprise generating the linear portion of the double-stranded DNA molecule by digesting the double-stranded DNA molecule with the endonuclease.

The step of incubating the single contiguous aqueous volume may be performed under conditions that promote appending (or linking) of the first and second terminal adaptor molecules and/or intermediate adaptor molecules to the linear double-stranded region to produce the linear DNA product. The appending may be performed by creating a covalent link between the first and/or second terminal adaptor molecule and the first and/or second end of the linear double-stranded region. The appending may be performed by creating a covalent link between the first and/or second terminal adaptor molecule and an nth and/or mth intermediate adaptor molecule. The appending may be performed by creating a covalent link between an intermediate adaptor molecule and the first or second end of the linear double-stranded region. The appending may be performed by creating a covalent link between an intermediate adaptor molecule and an adjacent adaptor molecule.

The step of incubating the single contiguous aqueous volume may be performed under conditions that promote digestion of the double-stranded DNA molecule to produce the linear portion of the double-stranded DNA molecule. The digestion of the double-stranded DNA molecule to produce the linear portion of the double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the linear double-stranded region to the first and second terminal adaptor molecules and/or to an intermediate adaptor molecules. The step of incubating the single contiguous aqueous volume may be performed under conditions that promote ligation of the intermediate adaptor molecules to the first and second terminal adaptor molecules and/or adjacent intermediate adaptor molecules and/or the first and/or second end of the linear double-stranded region. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the linear double-stranded regions (or the portions of the double-stranded DNA molecules) may be incorporated into closed linear DNA products. Preferably, the ligation is at least 15% efficient.

The step of ligation of the linear double-stranded region to the first and second terminal adaptor molecules and/or n+m intermediate adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C. Preferably, the second temperature is 14°C-18°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the n+m intermediate adaptor molecules and/or the first and second terminal adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The first and second terminal adaptor molecules and/or one or more of the n+m intermediate adaptor molecules may comprise one or more phosphorothioated nucleotides, such that, once the terminal adaptor molecules are appended (e.g. ligated) to the linear double-stranded region, the linear DNA product is resistant to nuclease digestion or has improved or enhanced resistance to nuclease digestion. The linear DNA product may be resistant to 3'-end exonuclease digestion (e.g. by exonuclease III) and/or 5'-end exonuclease digestion (e.g. by exonuclease VIII).

The first and second adaptor molecules and/or one or more of the n+m intermediate adaptor molecules may comprise a plurality of phosphorothioated nucleotides. For example, the terminal or intermediate adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

A terminal and/or intermediate adaptor molecule may be a nucleic acid adaptor molecule. The terminal or intermediate adaptor molecule may be double-stranded. The terminal or intermediate adaptor molecule may comprise a portion that is double-stranded.

The first and/or second terminal adaptor molecules may comprise at least 3, at least 4, at least 5, at least 6, at least 7, at least 8, at least 9, at least 10, at least 11, at least 12, at least 13, at least 14, at least 15, or at least 16 base pairs.

The terminal and/or intermediate adaptor molecules may comprise a plurality of phosphorothioated nucleotides in each strand. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand. One or more of the n+m intermediate adaptor molecules may comprise a plurality of phosphorothioated nucleotides at internal positions in each strand. For example, the one or more of the n+m intermediate adaptor molecules may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) at internal positions in each strand.

The terminal adaptor molecules may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The terminal or intermediate adaptor molecules may comprise at least 5%, at least 10%, at least 20%, at least 30%, at least 40%, at least 50%, at least 60%, at least 70%, at least 80%, at least 90%, at least 95%, or at least 100% of protected nucleotides.

Once the terminal adaptor molecules are appended to the linear double-stranded region (directly or indirectly via n or m intermediate adaptor molecules), the linear DNA product may comprise a protected nucleotide (e.g. phosphorothioated nucleotide) at the 5'-end (or at the 5'-end region) of one or both strands. Preferably, the linear DNA product comprises a phosphorothioated nucleotide at the 5'-end (or at the 5'-end region) of one or both strands. The linear DNA product may comprise a phosphorothioated nucleotide at the 5'-end (or at the 5'-end region) of one or both strands. As most exonucleases, for example exonuclease III, remove nucleotides from the 3'-end of the polynucleotide chain, the linear DNA product may comprise a protected nucleotide at the 3'-end (or at the 3'-end region) of one or both strands. Preferably, the linear DNA product comprises a phosphorothioated nucleotide at the 3'-end (or the 3'-end region) of one or both strands. The linear DNA product may comprise at least one phosphorothioated nucleotide at the 3'-end (or the 3'-end region) and at least one phosphorothioated nucleotide at the 5'-end (or the 5'-end region) of one or both strands. The linear DNA product may comprise a phosphorothioated nucleotide at the 3'-end (or the 3'-end region) and the 5'-end (or the 5'-end region) of one or both strands.

The linear DNA product may additionally comprise a plurality of protected nucleotides (e.g. phosphorothioated nucleotides) at internal positions in each strand. For example, the linear DNA product may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions in each strand. Preferably, the linear DNA product comprises at least 2 protected nucleotides (e.g. phosphorothioated nucleotides) at internal positions in each strand.

The internal positions may not be located between the second and penultimate nucleotide of the linear DNA product. The internal positions may be any position in the adaptor molecules other than the last nucleotide at the end of each strand.

The linear double-stranded region (or linear portion of the double-stranded molecule) may comprise a plurality of phosphorothioated nucleotides at internal positions in each strand. For example, the linear double-stranded region (or linear portion of the double-stranded molecule) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions in each strand. Preferably, the linear double-stranded region (or linear portion of the double-stranded molecule) comprises at least 2 protected nucleotides (e.g. phosphorothioated nucleotides) at internal positions in each strand. The internal positions may not be located between the second and penultimate nucleotide of the linear double-stranded region (or linear portion of the double-stranded molecule).

The nucleotides resistant to exonuclease digestion (i.e. protected nucleotides) may be phosphorothioated nucleotides of at least one type. For example, the at least one type of phosphorothioated nucleotides is α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The terminal and/or intermediate adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The terminal and/or intermediate adaptor molecules may comprise at least three types of phosphorothioated nucleotides. For example, the at least three types of phosphorothioated nucleotides are:

(e) α-S-dATP, α-S-dCTP and α-S-dGTP;
(f) α-S-dATP, α-S-dCTP and α-S-dTTP;
(g) α-S-dATP, α-S-dGTP and α-S-dTTP; or
(h) α-S-dCTP, α-S-dGTP and α-S-dTTP.

The terminal and/or intermediate adaptor molecules may comprise at least four types of phosphorothioated nucleotides. For example, the at least four types of protected nucleotides are α-S-dATP, α-S-dCTP, α-S-dGTP and α-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The method may further comprise, before step (a) (i.e. the step of contacting the double-stranded DNA molecule with the endonuclease, the ligase and the first and second terminal adaptor molecules and n+m intermediate adaptor molecules), a step of amplifying a DNA template molecule to produce the double-stranded DNA molecule. Thus, the invention provides a method for producing a linear DNA product, the method comprises:

(a) amplifying a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides).

The step of amplifying may be performed by in vitro or in vivo amplification. Preferably, the step of amplifying is performed by in vitro amplification. For example, the step of amplifying may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the step of amplifying is performed by rolling circle amplification. Thus, the invention provides a method for producing a linear DNA product, the method comprises:

(a) amplifying a DNA template molecule comprising at least one cleavable (e.g. endonuclease) target sequence to generate a double-stranded DNA molecule, wherein the DNA template molecule is amplified by rolling circle amplification;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides).

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

In the methods described herein, the DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a BbsI, BsaI, BsmBI, BspQI, BtgZI, Esp3I,SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the linear DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a BbsI, BsaI, BsmBI, BspQI, BtgZI, Esp3I,SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BsIFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, Btsl-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation in the template DNA molecule.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The method may further comprise, after the step of incubating the single contiguous aqueous volume, a step of purification of the linear DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purification. This step allows for removal of any double-stranded DNA molecules and/or adaptor molecules which have not been used in the course of performing the method. Thus, the method may comprise the steps:

(a) amplifying a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least;

(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides); and

(d) incubating the single contiguous aqueous volume with a nuclease (e.g. exonuclease).

The method may comprise the steps:

(a) amplifying a DNA template molecule comprising at least one cleavable (e.g. endonuclease) target sequence to generate a double-stranded DNA molecule;

(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;

(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides);

(d) purifying the closed linear DNA product; and

(e) incubating the purified product of step (d) with a nuclease (e.g. exonuclease).

The method may comprise the steps:

(a) amplifying a DNA template molecule comprising at least one cleavable (e.g. endonuclease) target sequence to generate a double-stranded DNA molecule;

(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;

(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides);

(d) incubating the single contiguous aqueous volume with a nuclease (e.g. exonuclease); and

(e) purifying the closed linear DNA product.

The step of incubating the single contiguous aqueous volume (or the purified product of step (d)) with a nuclease may be performed at a temperature of 5-90°C, 10-80°C, 15-70°C, 20-60°C, 25-50°C, 30-45°C or 35-40°C. The step of incubating the single contiguous aqueous volume (or the purified product of step (d)) with a nuclease may be performed for at least 10, at least 20, at least 30, at least 40, at least 50, or at least 60 min. The step of incubating the single contiguous aqueous volume (or the purified product of step (d)) may be performed at two different temperatures. For example, the step of incubating the single contiguous aqueous volume (or the purified product of step (d)) may be performed at 15-40°C for 10-60 minutes followed by a temperature of 60-90°C for 10-30 min. The higher temperature typically inactivates the nuclease (e.g. exonuclease). Thus, the method further provides a step of inactivating the nuclease (e.g. exonuclease). The step of incubating the single contiguous aqueous volume (or the purified product of step (d)) may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

A portion of the first terminal adaptor molecule (e.g. the overhang) may be complementary to the first end of the linear double-stranded region or complementary to a first end of an intermediate adaptor molecule, preferably the nth intermediate adaptor molecule. A portion of the second terminal adaptor molecule (e.g. the overhang) may be complementary to the second end of the linear double-stranded region or complementary to a first end of an intermediate adaptor molecule, preferably the mth adaptor molecule. A portion of the first end of the linear double-stranded region maybe complementary to a first end of an intermediate adaptor molecule, preferably n-(n-1) intermediate adaptor molecule. A portion of the second end of the linear double-stranded region maybe complementary to a first end of an intermediate adaptor molecule, preferably m-(m-1) intermediate adaptor molecule. A portion of a second end of an intermediate adaptor molecule may be complementary to a first end of an adjacent intermediate adaptor molecule, for example a portion of the second end of the nth intermediate adaptor molecule may be complementary to a portion of the first end of the n-1 th intermediate adaptor molecule, and a portion of the second end of the n-1th adaptor molecule may be complementary to a portion of the first end of the n-2th intermediate adaptor molecule, and so on. A portion of a second end of an intermediate adaptor molecule may be complementary to a first end of an adjacent intermediate adaptor molecule, for example a portion of the second end of the mth intermediate adaptor molecule may be complementary to a portion of the first end of the m-1th intermediate adaptor molecule, and a portion of the second end of the m-1th adaptor molecule may be complementary to a portion of the first end of the m-2th intermediate adaptor molecule, and so on. n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The first end and the second end of the linear double-stranded region may be resistant to nuclease digestion. Preferably, the first end and the second end of the linear double-stranded region are resistant to the exonuclease digestion, such as exonuclease III digestion and/or exonuclease I digestion.

The linear DNA product may be partially double-stranded and/or partially single-stranded. The linear DNA product may comprise a portion that is double-stranded and a portion that is single-stranded.

The linear DNA product may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The linear DNA product may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve ligation efficiency of the first and second terminal adaptor molecules to the linear double-stranded region. The spacer may improve a cell transfection yields.

The linear DNA product may comprise an inverted terminal repeat sequence.

The linear DNA product may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the linear DNA product is at least 50 base pairs long.

The double-stranded DNA molecule may be circular, or branched.

The double-stranded DNA molecule may not comprise an adaptor molecule. The double-stranded DNA molecule may not comprise a hairpin, a loop or a stem-loop structure.

The double-stranded DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule. The spacer may improve ligation efficiency of the first and second terminal adaptor molecules to the linear double-stranded region. The spacer may improve a cell transfection yields.

The double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may comprise one or more cleavable (e.g. endonuclease) target sequences. The double-stranded DNA molecule may comprise two cleavable (e.g. endonuclease) target sequences. The one or more endonuclease target sequences may be Type IIS endonuclease target sequences. The one or more endonuclease target sequences may be BbsI, BsaI, BsmBI, BspQI, BtgZI, Esp3!,Sap!, AarI, Acc36I, AclWI, AcuI, AjuI, AloI, Alw26I, AlwI, ArsI, AsuHPI, BaeI, BarI, BbvI, BccI, BceAI, Bcgl, BciVI, BcoDI, BfuAI, Bful, Bmrl, Bmsl, Bmul, Bpil, BpmI, BpuEI, BsaXI, Bse1I, Bse3DI, BseGI, BseMI, BseMII, BseNI, BseRI, BseXI, BsgI, BsIFI, BsmAI, BsmFI, Bsml, Bso31I, BspCNI, BspMI, BspPI, BspQI, BspTNI, BsrDI, Bsrl, Bst6!, BstF5I, BstMAI, BstV11, BstV2!, Bsul, BtgZI, BtsCI, Btsl-v2, BtsMutl, Bve!, Csel, CspCI, Eam1104I, Earl, Ecil, Eco31I, Eco57I, Esp3I, Faql, Faul, FokI, Gsul, Hgal, Hphl, HpyAV, Lgul, Lmnl, Lsp1109I, Lwel, Mboll, Mlyl, Mmel, MnlI, Mva1269!, NmeAIII, PaqCI, PciSI, Pctl, Plel, PpsI, Psrl, Schl, SfaNI, Taqll, TspDTI and/or TspGWI target sequences.

The double-stranded DNA molecule may be a product of amplification. Preferably, the amplification is rolling circle amplification.

The linear double-stranded region may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The linear double-stranded region may comprise a 3'-OH group at first and/or second ends. The 3'-OH group may facilitate ligation to the first and/or second terminal adaptor molecule(s) (which may comprise a 5' phosphate). The linear double-stranded region may comprise a 5' phosphate at first and/or second ends. The 5' phosphate may facilitate ligation to the first and/or second terminal adaptor molecule(s) (which may comprise a 3'-OH group).

The linear double-stranded region (e.g. the linear portion of the double-stranded molecule) may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the sense strand or the antisense strand of the linear double-stranded region.

The linear portion of the double-stranded DNA molecule (e.g. the linear portion of the double-stranded molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, double-stranded DNA molecule is at least 50 base pairs long.

The first terminal adaptor molecule and/or the second terminal adaptor molecule and/or one or more of the n+m intermediate adaptor molecules may be a synthetic adaptor molecule.

The first terminal adaptor molecule and/or the second terminal adaptor molecule and/or one or more of n+m intermediate adaptor molecules may not be a plasmid or a vector DNA.

The first terminal adaptor molecule and/or the second terminal adaptor molecule and/or one or more of n+m intermediate adaptor molecules may comprise a single-stranded portion. The single-stranded portion may comprise less than 10, 9, 8, 7, 6, 5, 4, 3, 2 nucleotides. Preferably, the single-stranded portion comprises 5 nucleotides.

The first terminal adaptor molecule and/or the second terminal adaptor molecule and/or one or more of the n+m intermediate adaptor molecules may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first and/or second terminal adaptor molecule may comprise or consist of the sequences of SEQ ID NO:13 and/or SEQ ID NO:14. The first and/or second terminal adaptor molecule may comprise or at least 15, 14, 13, 12, 11, 10, 19, 8, 7, 6, 5, contiguous nucleotides thereof.

The first terminal adaptor molecule and/or the second terminal adaptor molecule and/or one or more of the n+m intermediate adaptor molecules may comprise a 5' phosphate. The 5' phosphate may facilitate ligation to the linear double-stranded region or to adjacent terminal or intermediate adaptor molecules (which may comprise a 3'-OH group at first and/or second ends). The first terminal adaptor molecule and/or the second terminal adaptor molecule and/or one or more of the n+m intermediate adaptor molecules may comprise a 3'-OH. The 3'-OH may facilitate ligation to the linear double-stranded region and/or to adjacent intermediate adaptor molecules (which may comprise a 5' phosphate at first and/or second ends).

The first terminal adaptor molecule may comprise a portion that is complementary to the first end of the linear double-stranded region (or the linear portion of the double-stranded DNA molecule). The second terminal adaptor molecule may comprise a portion that is complementary to the second end of the linear double-stranded region (or the linear portion of the double-stranded DNA molecule). The first terminal adaptor molecule may comprise a portion that anneals to the first end of the linear double-stranded region (or the linear portion of the double-stranded DNA molecule). The second terminal adaptor molecule may comprise a portion that anneals to the second end of the linear double-stranded region (or the linear portion of the double-stranded DNA molecule). The first terminal adaptor molecule may comprise a portion that is complementary and anneals to the first end of the linear double-stranded region (or the linear portion of the double-stranded DNA molecule). The second terminal adaptor molecule may comprise a portion that is complementary and anneals to the second end of the linear double-stranded region (or the linear portion of the double-stranded DNA molecule).

The portion that is complementary or anneals to the first or second end of the linear double-stranded region (or the linear portion of the double-stranded DNA molecule) may be a 5' overhang or a 3' overhang of the first and/or second terminal adaptor molecule. The overhang of the first terminal adaptor molecule may be complementary to the first end of the linear double-stranded region and/or the overhang of the second terminal adaptor molecule may be complementary to the second end of the double-stranded region. The overhang of the first terminal adaptor molecule may anneal to the first end of the linear double-stranded region and/or the overhang of the second terminal adaptor molecule may anneal to the second end of the linear double-stranded region. The overhang of the first terminal adaptor molecule may be complementary to and anneal to the first end of the linear double-stranded region and/or the overhang of the second terminal adaptor molecule may be complementary to and anneal to the second end of the linear double-stranded region.

The first terminal adaptor molecule may comprise a portion that is complementary to the first end of the nth intermediate adaptor molecule. The second terminal adaptor molecule may comprise a portion that is complementary to the first end of the mth intermediate adaptor molecule. The first terminal adaptor molecule may comprise a portion that anneals to the first end of the nth intermediate adaptor molecule. The second terminal adaptor molecule may comprise a portion that anneals to first end of the mth intermediate adaptor molecule. The first terminal adaptor molecule may comprise a portion that is complementary and anneals to first end of the nth intermediate adaptor molecule. The second terminal adaptor molecule may comprise a portion that is complementary and anneals to first end of the mth intermediate adaptor molecule. n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The portion that is complementary or anneals to the first end of the nth or mth intermediate adaptor molecule may be a 5' overhang or a 3' overhang of the first and/or second terminal adaptor molecule. The overhang of the first terminal adaptor molecule may be complementary to the first end of the nth intermediate adaptor molecule and/or the overhang of the second terminal adaptor molecule may be complementary to first end of the mth intermediate adaptor molecule. The overhang of the first terminal adaptor molecule may anneal to first end of the nth intermediate adaptor molecule and/or the overhang of the second terminal adaptor molecule may anneal to first end of the mth intermediate adaptor molecule. The overhang of the first terminal adaptor molecule may be complementary to and anneal to first end of the nth intermediate adaptor molecule and/or the overhang of the second terminal adaptor molecule may be complementary to and anneal to first end of the mth intermediate adaptor molecule.

The first terminal adaptor molecule and/or the second terminal adaptor molecule may not comprise a Type IIS endonuclease target sequence. The first terminal adaptor molecule and/or the second terminal adaptor molecule may not comprise BbsI, BsaI, BsmBI, BspQI, BtgZI, Esp3!,Sap!, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, Bful, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6!, BstF5I, BstMAI, BstV1I, BstV2!, BsuI, BtgZI, BtsCI, BtsI-v2, BtsMutI, Bve!, CseI, CspCI, Eam1104I, EarI, EciI, Eco31I, Eco57!, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI SapI target sequences.

The first terminal adaptor molecule and/or the second terminal adaptor molecule and/or one or more of the n+m intermediate adaptor molecules may comprise a functional portion. The functional portion may be a binding molecule, a targeting sequence, a signal sequence or a probe. The functional portion may be a cassette, an open reading frame or a coding sequence. The functional portion may be a promotor, and enhancer, NLS sequence, a UTR, ITR or other repeats, a terminator sequence, modified nucleotides or a fluorophore.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the linear DNA product. For example, the targeting sequence may be a DNA nuclear targeting sequences (i.e. a recognition sequence for endogenous DNA-binding proteins), such as SV40 enhancer sequence (preferably downstream from the cassette). The targeting sequence may be a protelomerase targeting sequence or a truncated variant thereof.

To facilitate detection and/or quantification of the DNA product, the functional portion may comprise a fluorophore, a radioactive compound or a barcode.

A signal corresponding to the presence, absence and/or level of the linear DNA product may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the linear DNA product. The binding moiety is capable of binding to 3' and/or 5' end of the linear DNA product. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the linear DNA product and the second binding moiety linked to the second barcoded portion may bind to the 5' end of the linear DNA product. The 3' and 5' ends may comprise a 3' overhang, a 5' overhang or a blunt end.

A signal corresponding to the presence, absence and/or level of the linear DNA product may be measured using a fluorophore (i.e. a fluorescently-labelled molecule), which is attached or bound to the 3' overhang, the 5' overhang or the blunt end of the linear DNA product. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina® (e.g. the HiSeq™, MiSeq™ and/or Genome Analyzer™ sequencing systems), Oxford Nanopore™ Technologies (e.g. the MinION sequencing system), Ion Torrent™ (e.g. the Ion PGM™ and/or Ion Proton™ sequencing systems), Pacific Biosciences (e.g. the PACBIO RS II sequencing system); Life Technologies™ (e.g. a SOLiD sequencing system), Roche (e.g. the 454 GS FLX+ and/or GS Junior sequencing systems), or any other sequencing platform of interest.

One or more of the n+m intermediate adaptor molecules (e.g. 1, 2, 3, 4, 5, 6, 7 or 8 intermediate adaptor molecules) may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

One or more of the n+m intermediate adaptor molecules (e.g. 1, 2, 3, 4, 5, 6, 7 or 8 intermediate adaptor molecules) may comprise a spacer. The spacer may be at least 10, at least 20, at least 30, at least 40, at least 50, at least 60, at least 70, at least 80, at least 90, at least 100, at least 125, at least 150, at least 175, at least 200 or at least 300 base pairs long.

One or more of the n+m intermediate adaptor molecules (e.g. 1, 2, 3, 4, 5, 6, 7 or 8 intermediate adaptor molecules) may be at least, 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the one or more of the n+m intermediate adaptor molecules (e.g. 1, 2, 3, 4, 5, 6, 7 or 8 intermediate adaptor molecules) is at least 10 base pairs long.

A terminator adaptor molecule may be may be at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, at least 50, at least 100 base pairs long

The first terminal adaptor molecule and/or the second terminal adaptor molecule and/or one or more of the n+m intermediate adaptor molecules may comprise a polyA signal sequence.

The first terminal adaptor molecule and/or the second terminal adaptor molecule and/or one or more of the n+m intermediate adaptor molecules may comprise an aptamer.

The first terminal adaptor molecule and/or the second terminal adaptor molecule and/or one or more of the n+m intermediate adaptor molecules may confer resistance to the nuclease digestion, such as exonuclease digestion (e.g. exonuclease I and/or exonuclease III digestion).

**3. Methods for producing a partially closed linear DNA product comprising nuclease-resistant nucleotides**

The methods described herein may be used to produce a partially closed linear DNA product comprising nuclease-resistant (i.e. protected nucleotides).

The invention provides a method for producing a partially closed deoxyribonucleic acid (DNA) product, wherein the method comprises:

(a) contacting a double-stranded DNA molecule with an endonuclease and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides.

The step of contacting the double-stranded DNA molecule with the endonuclease and first and second terminal adaptor molecules is preferably performed in the presence of a ligase. Thus, the method for producing a partially closed linear DNA product may comprise the steps:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides.

The partially closed linear DNA product produced by the methods described herein has enhanced resistance to nuclease (e.g. exonuclease) digestion.

The appending of the first terminal adaptor molecule and/or the second terminal adaptor molecule to the first and/or second end of the linear double-stranded region (directly or indirectly via one or more intermediate adaptor molecules) may be performed by hybridization or ligation of the adaptor molecules to the ends of the linear double-stranded region and/or to sequential adaptor molecules. Thus, the n intermediate adaptor molecules may be sequentially hybridized to the first end of the linear double-stranded region. The m intermediate adaptor molecules may be sequentially hybridized to the second end of the linear double-stranded region. The n intermediate adaptor molecules may be sequentially ligated to the first end of the linear double-stranded region. The m intermediate adaptor molecules may be sequentially ligated to the second end of the linear double-stranded region. The appending of the n+m intermediate adaptor molecules, first terminal adaptor molecule and the second terminal adaptor molecule may be performed by both hybridization and ligation of the adaptor molecules to the ends of the linear double-stranded region and/or to adjacent adaptor molecules. Thus, n intermediate adaptor molecules may be sequentially hybridized and ligated to the first end of the linear double-stranded region. The m intermediate adaptor molecules may be sequentially hybridized and ligated to the second end of the linear double-stranded region. The appending may be performed via a linker or spacer molecule which facilitates joining of the adaptor molecule to the first and/or second end of the linear double-stranded region or to an adjacent adaptor molecule. The first terminal adaptor molecules may be hybridized, ligated or hybridized and ligated to the nth intermediate adaptor molecule or to the first end of the double-stranded DNA molecule. The second terminal adaptor molecule may hybridized, ligated or hybridized and ligated to the mth intermediate adaptor molecule or the second end of the double stranded DNA molecule.

The method for producing a partially closed linear DNA product may comprise the steps:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides (i.e. protected nucleotides). n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0,1, 2, 3 or 4..

The method for producing a partially closed linear DNA product may comprise the steps:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate the partially closed linear DNA product, wherein the partially closed linear DNA product comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides (i.e. protected nucleotides).

As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

Preferably, the step of contacting the double-stranded DNA molecule with the endonuclease, the ligase and first and second terminal adaptor molecules is performed in a single reaction (i.e. a single step).

The step of incubating the single contiguous aqueous volume to generate the partially closed linear DNA product may comprise generating the linear portion of the double-stranded DNA molecule by digesting the double-stranded DNA molecule with the endonuclease.

The addition of intermediate adaptor molecules allows for additional flexibility in the production of the partially closed linear DNA products of the invention. The intermediate adaptor molecules may allow for longer partially closed linear DNA products to be produced. The intermediate adaptor molecules allow for bar codes, promotor sequences, labels, poly A signals and/or open reading frames to be incorporated into the partially closed linear DNA product of the invention. An intermediate adaptor molecule may comprise a cassette. For example, if it is advantageous to include several genes of interest, or open reading frames, in the partially closed linear DNA product, intermediate adaptor molecules may comprise one or more cassettes, and the linear DNA region may comprise one or more cassettes. An intermediate adaptor molecule may comprise a label, a signalling sequence, a targeting sequence or a binding moiety. An intermediate adaptor molecule may comprise a promotor, a UTR or a terminator sequence.

As well as providing nuclease-resistance to the partially closed linear DNA product, the terminal adaptor molecules may also provide further functional features. A terminator adaptor molecule may allow for longer partially closed linear DNA products to be produced. Terminator adaptor molecules may allow for bar codes, promotor sequences, labels, poly A signals and/or open reading frames to be incorporated into the partially closed linear DNA product of the invention. A terminal adaptor molecule may comprise a label, a signalling sequence, a targeting sequence or a binding moiety. A terminal adaptor molecule may comprise a UTR or a terminator sequence.

The step of incubating the single contiguous aqueous volume may be performed under conditions that promote appending (or linking) of the first and second terminal adaptor molecules and/or intermediate adaptor molecules to the linear double-stranded region to produce the partially closed linear DNA product. The appending may be performed by creating a covalent link between the first and/or second terminal adaptor molecule and/or n+m intermediate adaptor molecules and the first and/or second end of the linear double-stranded region. The appending may be performed by creating a covalent link between the first and/or second terminal adaptor molecule and an nth and/or mth intermediate adaptor molecule. The appending may be performed by creating a covalent link between an intermediate adaptor molecule and the first or second end of the linear double-stranded region. The appending may be performed by creating a covalent link between an intermediate adaptor molecule and an adjacent adaptor molecule.

The step of incubating the single contiguous aqueous volume may be performed under conditions that promote digestion of the double-stranded DNA molecule to produce the linear portion of the double-stranded DNA molecule. The digestion of the double-stranded DNA molecule to produce the linear portion of the double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the linear double-stranded region to the first and second terminal adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the linear double-stranded regions (or the portions of the double-stranded DNA molecules) may be incorporated into closed linear DNA products. Preferably, the ligation is at least 15% efficient.

The step of ligation of the linear double-stranded region to the first and second terminal adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C. Preferably, the second temperature is 14°C-18°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the first and second terminal adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The first and second terminal adaptor molecules may comprise one or more phosphorothioated nucleotides, such that, once the adaptor molecules are appended (e.g. ligated) to the linear double-stranded region, the partially closed linear DNA product is resistant to nuclease digestion or has improved or enhanced resistance to nuclease digestion. The partially closed linear DNA product may be resistant to 3'-end exonuclease digestion (e.g. by exonuclease III) and/or 5'-end exonuclease digestion (e.g. by exonuclease VIII).

A terminal or intermediate adaptor molecule may comprise a plurality of phosphorothioated nucleotides. For example, the terminal or intermediate adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

A terminal or intermediate adaptor molecule may be a nucleic acid adaptor molecule. The terminal or intermediate adaptor molecule may be double-stranded. The terminal or intermediate adaptor molecule may comprise a portion that is double-stranded.

The first and/or second terminal adaptor molecules and/or one or more of n+m intermediate adaptor molecules may comprise at least 3, at least 4, at least 5, at least 6, at least 7, at least 8, at least 9, at least 10, at least 11, at least 12, at least 13, at least 14, at least 15, or at least 16 base pairs. The first and/or second terminal adaptor molecules and/or one or more of n+m intermediate adaptor molecules may comprise at least 1000 base pairs, at least 750 base pairs, at least 500 base pairs, at least 250 base pairs, at least 200 base pairs, at least 150 base pairs, at least 100 base pairs or at least 75 base pairs.

The terminal or intermediate adaptor molecule may comprise a plurality of phosphorothioated nucleotides in each strand. For example, the terminal or intermediate adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The terminal or intermediate adaptor molecule may comprise a plurality of phosphorothioated nucleotides at internal positions in each strand. For example, the terminal or intermediate adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the terminal or intermediate adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The terminal or intermediate adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

Once the adaptor molecules are appended to the linear double-stranded region, the partially closed linear DNA product may comprise a protected nucleotide (e.g. phosphorothioated nucleotide) at the 5'-end (or at the 5'-end region) of one or both strands. Preferably, the partially closed linear DNA product comprises a phosphorothioated nucleotide at the 5'-end (or at the 5'-end region) of one strand. The partially closed linear DNA product may comprise a phosphorothioated nucleotide at the 5'-end (or at the 5'-end region) of one strand. As most exonucleases, for example exonuclease III, remove nucleotides from the 3'-end of the polynucleotide chain, the linear DNA product may comprise a protected nucleotide at the 3'-end (or at the 3'-end region) of one strand. Preferably, the partially closed linear DNA product comprises a phosphorothioated nucleotide at the 3'-end (or the 3'-end region) of one strand. The partially closed linear DNA product may comprise at least one phosphorothioated nucleotide at the 3'-end (or the 3'-end region) and at least one phosphorothioated nucleotide at the 5'-end (or the 5'-end region) of one strand. The partially closed linear DNA product may comprise at least one phosphorothioated nucleotide at the 3'-end (or the 3'-end region) of the sense strand and the 5'-end (or the 5'-end region) if the antisense strand. The partially closed linear DNA product may comprise at least one phosphorothioated nucleotide at the 5'-end (or the 5'-end region) of the sense strand and the 3'-end (or the 3'-end region) if the antisense strand. Thus, both the sense and antisense strands in the double stranded partially closed linear DNA product may be protected from nuclease digestion by using nuclease-resistant nucleotides at one end of the partially closed linear DNA product.

One of the terminal adaptor molecules used in the methods described herein may comprise a self-complementary element which creates a loop, such as a hairpin loop or a stem loop. Thus, the one of the terminal adaptor molecules may comprise a hairpin or a stem-loop. The terminal adaptor molecule may comprise a double-stranded portion comprising a sense strand and an antisense strand, wherein the sense strand and the antisense strand are linked together in a hairpin such that the sense strand is hybridized to the antisense strand. The double-stranded portion of a terminal adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (or linear portion of the double-stranded DNA molecule) may comprise a 3' or a 5' overhang. A portion of the terminal adaptor molecule (e.g. the overhang) may be complementary to the first end or the second end of the linear double-stranded region.

The method described herein may use a first terminal adaptor molecule which comprises hairpin loop or stem loop, or any other structure which is able to close one end of the linear DNA molecule, and a second terminal adaptor molecule which is a linear nucleic acid molecule comprising nuclease-resistant nucleotides to produce a partially closed linear DNA product.

The partially closed linear DNA product may be a partially covalently closed DNA product. Thus, in embodiments where the first terminal adaptor molecule comprise a loop (e.g. a hairpin), the terminal adaptor molecule closes one end of the linear double-stranded region forming a partially covalently closed DNA product.

The first terminal adaptor molecule may comprise a single-stranded portion. The single-stranded portion may form a hairpin or a stem-loop. The single-stranded portion may comprise less than 10, 9, 8, 7, 6, 5, 4, 3, 2 nucleotides. Preferably, the single-stranded portion comprises 5 nucleotides.

The first terminal adaptor molecule may comprise or consist of the sequence of SEQ ID NO: 9 or a portion thereof. The first terminal adaptor molecule may comprise at least 13, at least 14, at least 15, at least 16, at least 17, at least 18, or at least 19 contiguous nucleotides of SEQ ID NO: 9. The double-stranded portion of the first terminal adaptor molecule may comprise the sequence of SEQ ID NO: 10 or a portion thereof. The double-stranded portion of the first and/or second terminal adaptor molecule may comprise at least 8, at least 9, at least 10, at least 11, at least 12, at least 13, at least 14 or at least 15 contiguous nucleotides of SEQ ID NO: 10. The single-stranded portion of the first terminal adaptor molecule may comprise a sequence of ACTCA. The single-stranded portion of the first terminal adaptor molecule may comprise at least 1, at least 2, at least 3, at least 4 or at least 5 contiguous nucleotides of the sequence ACTCA. The first terminal adaptor molecule may comprise the sequence of SEQ ID NO: 12. The first terminal adaptor molecule may comprise at least 13, at least 14, at least 15, at least 16, at least 17, at least 18, or at least 19 contiguous nucleotides of SEQ ID NO:12.

The second terminal adaptor molecule may comprise or consist of the sequences of SEQ ID NO:13 and/or SEQ ID NO:14. The second terminal adaptor molecule may comprise or at least 15, 14, 13, 12, 11, 10, 19, 8, 7, 6, 5, contiguous nucleotides thereof.

The partially closed linear DNA product may additionally comprise a plurality of protected nucleotides (e.g. phosphorothioated nucleotides) at internal positions in each strand. For example, the linear DNA product may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions in each strand. Preferably, the partially closed linear DNA product comprises at least 2 protected nucleotides (e.g. phosphorothioated nucleotides) at internal positions in each strand.

The internal positions may not be located between the second and penultimate nucleotide of the partially closed linear DNA product. The internal positions may be any position in the adaptor molecules other than the last nucleotide at the end of each strand.

The linear double-stranded region (or linear portion of the double-stranded molecule) may comprise a plurality of phosphorothioated nucleotides at internal positions in each strand. For example, the linear double-stranded region (or linear portion of the double-stranded molecule) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions in each strand. Preferably, the linear double-stranded region (or linear portion of the double-stranded molecule) comprises at least 2 protected nucleotides (e.g. phosphorothioated nucleotides) at internal positions in each strand. The internal positions may not be located between the second and penultimate nucleotide of the linear double-stranded region (or linear portion of the double-stranded molecule).

The nucleotides resistant to exonuclease digestion (i.e. protected nucleotides) may be phosphorothioated nucleotides of at least one type. For example, the at least one type of phosphorothioated nucleotides is $\alpha$-S-dATP (i.e. 2'-deoxyadenosine-5'-(a-thio)-triphosphate), $\alpha$-S-dCTP (i.e. 2'-deoxycytidine-5'-(a-thio)-triphosphate), $\alpha$-S-dGTP (i.e. 2'-deoxyguanosine-5'-($\alpha$-thio)-triphosphate), $\alpha$-S-dTTP (i.e. 2'-deoxythymidine-5'-($\alpha$-thio)-triphosphate), $\alpha$-S-dUTP (i.e. 2'-deoxyuridine-5'-($\alpha$-thio)-triphosphate), and/or uridine 2', 3'-cyclophosphorothioate.

The terminal or intermediate adaptor molecules may comprise at least two types of phosphorothioated nucleotides. For example, the at least two types of phosphorothioated nucleotides are: $\alpha$-S-dATP and $\alpha$-S-dCTP, $\alpha$-S-dATP and $\alpha$-S-dGTP, $\alpha$-S-dATP and $\alpha$-S-dTTP, $\alpha$-S-dCTP and $\alpha$-S-dGTP, $\alpha$-S-dCTP and $\alpha$-S-dTTP, or $\alpha$-S-dGTP and $\alpha$-S-dTTP.

The terminal or intermediate adaptor molecules may comprise at least three types of phosphorothioated nucleotides. For example, the at least three types of phosphorothioated nucleotides are:

(i) α-S-dATP, α-S-dCTP and α-S-dGTP;
(j) α-S-dATP, α-S-dCTP and α-S-dTTP;
(k) α-S-dATP, α-S-dGTP and α-S-dTTP; or
(l) α-S-dCTP, α-S-dGTP and α-S-dTTP.

The adaptor molecules may comprise at least four types of phosphorothioated nucleotides. For example, the at least four types of protected nucleotides are α-S-dATP, α-S-dCTP, α-S-dGTP and α-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The method may further comprise, before step (a) (i.e. the step of contacting the double-stranded DNA molecule with the endonuclease, the ligase and the first and second terminal adaptor molecules), a step of amplifying a DNA template molecule to produce the double-stranded DNA molecule. Thus, the invention provides a method for producing a partially closed linear DNA product, the method comprises:

(a) amplifying a DNA template molecule comprising at least one cleavable (e.g. endonuclease) target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides (i.e. protected nucleotides).

The step of amplifying may be performed by in vitro or in vivo amplification. Preferably, the step of amplifying is performed by in vitro amplification. For example, the step of amplifying may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the step of amplifying is performed by rolling circle amplification. Thus, the invention provides a method for producing a partially closed linear DNA product, the method comprises:

(a) amplifying a DNA template molecule comprising at least one cleavable (e.g. endonuclease) target sequence to generate a double-stranded DNA molecule, wherein the DNA template molecule is amplified by rolling circle amplification;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides (i.e. protected nucleotides).

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification *in vitro* under isothermal conditions using a suitable nucleic acid polymerase, such as Phi29 DNA polymerase.

In the methods described herein, the DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a BbsI, BsaI, BsmBI, BspQI, BtgZI, Esp3!,Sap!, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAI, Bful, Bmrl, Bmsl, Bmul, Bpil, Bpml, BpuEl, BsaXI, Bse1I, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6!, BstF51, BstMAI, BstV1I, BstV2!, Bsul, BtgZI, BtsCI, Btsl-v2, BtsMutl, Bve!, Csel, CspCI, Eam11 04!, Earl, Ecil, Eco31I, Eco57!, Esp3I, Faql, Faul, Fokl, Gsul, Hgal, Hphl, HpyAV, Lgul, Lmnl, Lsp11 09I, Lwel, Mboll, Mlyl, Mmel, Mnll, Mva1269!, NmeAIII, PaqCI, PciSI, Pctl, Plel, Ppsl, Psrl, Schl, SfaNI, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the partially closed linear DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3!,Sap!, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAI, Bful, Bmrl, Bmsl, Bmul, Bpil, Bpml, BpuEl, BsaXI, Bse1I, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6!, BstF51, BstMAI, BstV11, BstV2!, Bsul, BtgZI, BtsCI, Btsl-v2, BtsMutl, Bve!, Csel, CspCI, Eam11 04!, Earl, Ecil, Eco31I, Eco57!, Esp3I, Faql, Faul, Fokl, Gsul, Hgal, Hphl, HpyAV, Lgul, Lmnl, Lsp11 09I, Lwel, Mboll, Mlyl, Mmel, Mnll, Mva1269!, NmeAIII, PaqCI, PciSI, Pctl, Plel, Ppsl, Psrl, Schl, SfaNI, Taqll, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation in the template DNA molecule.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the partially closed linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The method may further comprise, after the step of incubating the single contiguous aqueous volume, a step of purification of the partially closed linear DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purification. This step allows for removal of any double-stranded DNA molecules and/or adaptor molecules which have not been used in the course of performing the method. Thus, the method may comprise the steps:

> (a) amplifying a DNA template molecule comprising at least one cleavable (e.g. endonuclease) target sequence to generate a double-stranded DNA molecule;
> (b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
> (c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides (i.e. protected nucleotides); and
> (d) incubating the single contiguous aqueous volume with a nuclease (e.g. exonuclease).

The method may comprise the steps:

> (a) amplifying a DNA template molecule comprising at least one cleavable (e.g. endonuclease) target sequence to generate a double-stranded DNA molecule;
> (b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
> (c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides (i.e. protected nucleotides);
> (d) purifying the closed linear DNA product; and
> (e) incubating the purified product of step (d) with a nuclease (e.g. exonuclease).

The method may comprise the steps:

> (a) amplifying a DNA template molecule comprising at least one cleavable (e.g. endonuclease) target sequence to generate a double-stranded DNA molecule;

(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;

(c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides (i.e. protected nucleotides);

(d) incubating the single contiguous aqueous volume with a nuclease (e.g. exonuclease); and

(e) purifying the closed linear DNA product.

The step of incubating the single contiguous aqueous volume (or the purified product of step (d)) with a nuclease may be performed at a temperature of 5-90°C, 10-80°C, 15-70°C, 20-60°C, 25-50°C, 30-45°C or 35-40°C. The step of incubating the single contiguous aqueous volume (or the purified product of step (d)) with a nuclease may be performed for at least 10, at least 20, at least 30, at least 40, at least 50, or at least 60 min. The step of incubating the single contiguous aqueous volume (or the purified product of step (d)) may be performed at two different temperatures. For example, the step of incubating the single contiguous aqueous volume (or the purified product of step (d)) may be performed at 15-40°C for 10-60 minutes followed by a temperature of 60-90°C for 10-30 min. The higher temperature typically inactivates the nuclease (e.g. exonuclease). Thus, the method further provides a step of inactivating the nuclease (e.g. exonuclease). The step of incubating the single contiguous aqueous volume (or the purified product of step (d)) may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The first terminal adaptor molecule and/or the second terminal adaptor molecule may comprise an overhang. The end of the linear double-stranded region may comprise a 3' or a 5' overhang. A portion of the first terminal adaptor molecule (e.g. the overhang) may be complementary to the first end of the linear double-stranded region. A portion of the second terminal adaptor molecule may be complementary to the second end of the linear double-stranded region.

The first end and the second end of the linear double-stranded region may be resistant to nuclease digestion. Preferably, the first end and the second end of the linear double-stranded region are resistant to the exonuclease digestion, such as exonuclease III digestion and/or exonuclease I digestion.

The linear DNA product may be partially double-stranded and/or partially single-stranded. The linear DNA product may comprise a portion that is double-stranded and a portion that is single-stranded.

The partially closed linear DNA product may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The partially closed linear DNA product may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve ligation efficiency of the first and second terminal adaptor molecules to the linear double-stranded region. The spacer may improve a cell transfection yields.

The partially closed linear DNA product may comprise an inverted terminal repeat sequence.

The partially closed linear DNA product may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the partially closed linear DNA product is at least 50 base pairs long.

The double-stranded DNA molecule may be circular or branched.

The double-stranded DNA molecule may not comprise an adaptor molecule. The double-stranded DNA molecule may not comprise a hairpin, a loop or a stem-loop structure.

The double-stranded DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule. The spacer may improve ligation efficiency of the first and second terminal adaptor molecules to the linear double-stranded region. The spacer may improve a cell transfection yields.

The double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may comprise one or more cleavable (e.g. endonuclease) target sequences. The double-stranded DNA molecule may comprise two cleavable (e.g. endonuclease) target sequences. The one or more endonuclease target sequences may be Type IIS endonuclease target sequences. The one or more endonuclease target sequences may be BbsI, BsaI, BsmBI, BspQI, BtgZI, Esp3!,Sap!, AarI, Acc36I, AclWI, AcuI, AjuI, AloI, Alw26I, AlwI, ArsI, AsuHPI, BaeI, BarI, BbvI, BccI, BceAI, Bcgl, BciVI, BcoDI, BfuAI, Bful, BmrI, BmsI, BmuI, BpiI, BpmI, BpuEI, BsaXI, Bse1I, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6!, BstF5I, BstMAI, BstV11, BstV2!, Bsul, BtgZI, BtsCI, Btsl-v2, BtsMutl, Bve!, Csel, CspCI, Eam1104I, Earl, EciI, Eco31I, Eco57!, Esp3I, FaqI, Faul, Fokl, Gsul, Hgal, Hphl, HpyAV, Lgul, Lmnl, Lsp1109I, LweI, MboII, MlyI, MmeI, MnlI, Mva1269!, NmeAIII, PaqCI, PciSI, PctI, PleI, PpsI, PsrI, SchI, SfaNI, TaqII, TspDTI and/or TspGWI target sequences.

The double-stranded DNA molecule may be a product of amplification. Preferably, the amplification is rolling circle amplification.

The linear double-stranded region may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The linear double-stranded region may comprise a 3'-OH group at first and/or second ends. The 3'-OH group may facilitate ligation to the first and/or second terminal adaptor molecule(s) and/or n+M intermediate adaptor molecules (which may comprise a 5' phosphate). The linear double-stranded region may comprise a 5' phosphate at first and/or second ends. The 5' phosphate may facilitate ligation to the first and/or second terminal adaptor molecule(s) and/or intermediate adaptor molecules (which may comprise a 3'-OH group).

The linear double-stranded region (e.g. the linear portion of the double-stranded molecule) may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the sense strand or the antisense strand of the linear double-stranded region.

The linear portion of the double-stranded DNA molecule (e.g. the linear portion of the double-stranded molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, double-stranded DNA molecule is at least 50 base pairs long.

The first terminal adaptor molecule and/or the second terminal adaptor molecule or one or more of n+m intermediate adaptor molecules may be a synthetic adaptor molecule.

The first terminal adaptor molecule and/or the second terminal adaptor molecule and/or one or more of n+m intermediate adaptor molecules may not be a plasmid or a vector DNA.

The adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The adaptor molecule may comprise a 5' phosphate. The 5' phosphate may facilitate ligation to the linear double-stranded region.

The first terminal adaptor molecule may comprise a portion that is complementary to the first end of the linear double-stranded region (or the linear portion of the double-stranded DNA molecule). The second terminal adaptor molecule may comprise a portion that is complementary to the second end of the linear double-stranded region (or the linear portion of the double-stranded DNA molecule). The first terminal adaptor molecule may comprise a portion that anneals to the first end of the linear double-stranded region (or the linear portion of the double-stranded DNA molecule). The second terminal adaptor molecule may comprise a portion that anneals to the second end of the linear double-stranded region (or the linear portion of the double-stranded DNA molecule). The first terminal adaptor molecule may comprise a portion that is complementary and anneals to the first end of the linear double-stranded region (or the linear portion of the double-stranded DNA molecule). The second terminal adaptor molecule may comprise a portion that is complementary and anneals to the second end of the linear double-stranded region (or the linear portion of the double-stranded DNA molecule).

The portion that is complementary or anneals to the first or second end of the linear double-stranded region (or the linear portion of the double-stranded DNA molecule) may be a 5' overhang or a 3' overhang of the first and/or second terminal adaptor molecule. The overhang of the first terminal adaptor molecule may be complementary to the first end of the linear double-stranded region and/or the overhang of the second terminal adaptor molecule may be complementary to the second end of the double-stranded region. The overhang of the first terminal adaptor molecule may anneal to the first end of the linear double-stranded region and/or the overhang of the second terminal adaptor molecule may anneal to the second end of the linear double-stranded region. The overhang of the first terminal adaptor molecule may be complementary to and anneal to the first end of the linear double-stranded region and/or the overhang of the second terminal adaptor molecule may be complementary to and anneal to the second end of the linear double-stranded region.

The first terminal adaptor molecule may comprise a portion that is complementary to the first end of the nth intermediate adaptor molecule. The second terminal adaptor molecule may comprise a portion that is complementary to the first end of the mth intermediate adaptor molecule. The first terminal adaptor molecule may comprise a portion that anneals to the first end of the nth intermediate adaptor molecule. The second terminal adaptor molecule may comprise a portion that anneals to first end of the mth intermediate adaptor molecule. The first terminal adaptor molecule may comprise a portion that is complementary and anneals to first end of the nth intermediate adaptor molecule. The second terminal adaptor molecule may comprise a portion that is complementary and anneals to first end of the mth intermediate adaptor molecule.

The portion that is complementary or anneals to the first end of the nth or mth intermediate adaptor molecule may be a 5' overhang or a 3' overhang of the first and/or second terminal adaptor molecule. The overhang of the first terminal adaptor molecule may be complementary to the first end of the nth intermediate adaptor molecule and/or the overhang of the second terminal adaptor molecule may be complementary to first end of the mth intermediate adaptor molecule. The overhang of the first terminal adaptor molecule may anneal to first end of the nth intermediate adaptor molecule and/or the overhang of the second terminal adaptor molecule may anneal to first end of the mth intermediate adaptor molecule. The overhang of the first terminal adaptor molecule may be complementary to and anneal to first end of the nth intermediate adaptor molecule and/or the overhang of the second terminal adaptor molecule may be complementary to and anneal to first end of the mth intermediate adaptor molecule.

The first terminal adaptor molecule and/or the second terminal adaptor molecule and/or one or more of the n+m intermediate adaptor molecules may comprise a 5' phosphate. The 5' phosphate may facilitate ligation to the linear double-stranded region or to adjacent terminal or intermediate adaptor molecules (which may comprise a 3'-OH group at first and/or second ends). The first terminal adaptor molecule and/or the second terminal adaptor molecule and/or one or more of the n+m intermediate adaptor molecules may comprise a 3'-OH. The 3'-OH may facilitate ligation to the linear double-stranded region and/or to adjacent intermediate adaptor molecules (which may comprise a 5' phosphate at first and/or second ends).

A portion of the first terminal adaptor molecule (e.g. the overhang) may be complementary to the first end of the linear double-stranded region or complementary to a first end of an intermediate adaptor molecule, preferably the nth intermediate adaptor molecule. A portion of the second terminal adaptor molecule (e.g. the overhang) may be complementary to the second end of the linear double-stranded region or complementary to a first end of an intermediate adaptor molecule, preferably the mth adaptor molecule. A portion of the first end of the linear double-stranded region maybe complementary to a first end of an intermediate adaptor molecule, preferably n-(n-1) intermediate adaptor molecule. A portion of the second end of the linear double-stranded region maybe complementary to a first end of an intermediate adaptor molecule, preferably m-(m-1) intermediate adaptor molecule. A portion of a second end of an intermediate adaptor molecule may be complementary to a first end of an adjacent intermediate adaptor molecule, for example a portion of the second end of the nth intermediate adaptor molecule may be complementary to a portion of the first end of the n-1th intermediate adaptor molecule, and a portion of the second end of the n-1th adaptor molecule may be complementary to a portion of the first end of the n-2th intermediate adaptor molecule, and so on. A portion of a second end of an intermediate adaptor molecule may be complementary to a first end of an adjacent intermediate adaptor molecule, for example a portion of the second end of the mth intermediate adaptor molecule may be complementary to a portion of the first end of the m-1th intermediate adaptor molecule, and a portion of the second end of the m-1th adaptor molecule may be complementary to a portion of the first end of the m-2th intermediate adaptor molecule, and so on. n may be 0, 1, 2, 3, 4, 5, 6, 7 or 8. m may be 0, 1, 2, 3, 4, 5, 6, 7 or 8. Preferably, n is 0, 1, 2, 3 or 4, and m is 0, 1, 2, 3 or 4..

The first terminal adaptor molecule and/or the second terminal adaptor molecule may not comprise a Type IIS endonuclease target sequence. The first terminal adaptor molecule and/or the second terminal adaptor molecule may not comprise BbsI, BsaI, BsmBI, BspQI, BtgZI, Esp3!,Sap!, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, Bful, BmrI, BmsI, Bmul, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6!, BstF5I, BstMAI, BstV1I, BstV2!, BsuI, BtgZI, BtsCI, BtsI-v2, BtsMutI, Bve!, CseI, CspCI, Eam1104I, EarI, EciI, Eco31I, Eco57!, Esp3I, FaqI, FauI, FokI, GsuI, HgaI, HphI, HpyAV, LguI, LmnI, Lsp11091, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI SapI target sequences.

The first terminal adaptor molecule and/or the second terminal adaptor molecule and/or one or more of the n+m intermediate adaptor molecules may comprise a functional portion. The functional portion may be a binding molecule, a targeting sequence, or a probe. The functional portion may be a cassette, an open reading frame or a coding sequence. The functional portion may be a promotor, and enhancer, NLS sequence, a UTR, ITR or other repeats, a terminator sequence, modified nucleotides or a fluorophore.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the partially closed linear DNA product. For example, the targeting sequence may be a DNA nuclear targeting sequences (i.e. a recognition sequence for endogenous DNA-binding proteins), such as SV40 enhancer sequence (preferably downstream from the cassette). The targeting sequence may be a protelomerase targeting sequence, or a truncated variant thereof.

To facilitate detection and/or quantification of the DNA product, the functional portion may comprise a fluorophore, a radioactive compound or a barcode.

A signal corresponding to the presence, absence and/or level of the partially closed linear DNA product may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the partially closed linear DNA product. The binding moiety is capable of binding to 3' and/or 5' end of the partially closed linear DNA product. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the partially closed linear DNA product and/or the second binding moiety linked to the second barcoded portion may bind to the 5' end of the partially closed linear DNA product.

A signal corresponding to the presence, absence and/or level of the partially closed linear DNA product may be measured using a fluorophore (i.e. a fluorescently-labelled molecule), which is attached or bound to the 3' overhang, the 5' overhang or the blunt end of the partially closed linear DNA product. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina® (e.g. the HiSeq™, MiSeq™ and/or Genome Analyzer™ sequencing systems), Oxford Nanopore™ Technologies (e.g. the MinION sequencing system), Ion Torrent™ (e.g. the Ion PGM™ and/or Ion Proton™ sequencing systems), Pacific Biosciences (e.g. the PACBIO RS II sequencing system); Life Technologies™ (e.g. a SOLiD sequencing system), Roche (e.g. the 454 GS FLX+ and/or GS Junior sequencing systems), or any other sequencing platform of interest.

One or more of the n+m intermediate adaptor molecules (e.g. 1, 2, 3, 4, 5, 6, 7 or 8 intermediate adaptor molecules) may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

One or more of the n+m intermediate adaptor molecules (e.g. 1, 2, 3, 4, 5, 6, 7 or 8 intermediate adaptor molecules) may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long.

One or more of the n+m intermediate adaptor molecules (e.g. 1, 2, 3, 4, 5, 6, 7 or 8 intermediate adaptor molecules) may be at least, 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the one or more of the n+m intermediate adaptor molecules (e.g. 1, 2, 3, 4, 5, 6, 7 or 8 intermediate adaptor molecules) is at least 10 base pairs long.

The first terminal adaptor molecule and/or the second terminal adaptor molecule and/or one or more of the n+m intermediate adaptor molecules may comprise a polyA signal sequence. One or more of the n+m intermediate adaptor molecules may comprise a polyA signal downstream of a barcode.

The first terminal adaptor molecule and/or the second terminal adaptor molecule and/or one or more of the n+m intermediate adaptor molecules may comprise an aptamer.

## 4. Methods for transcription and protein expression

The invention provides a method for in vitro transcription of a linear DNA product (e.g. a closed linear DNA product or a partially closed linear DNA product), wherein the method comprises contacting the linear DNA product (e.g. a closed linear DNA product or a partially closed linear DNA product), produced by the methods described herein, with a polymerase and producing a transcription product encoded by the linear DNA product (e.g. the closed linear DNA product or a partially closed linear DNA product).

The invention provides a method for in vitro transcription of a linear DNA product (e.g. a closed linear DNA product or a partially closed linear DNA product), wherein the method comprises:

(a) producing a linear DNA product (e.g. a closed linear DNA product or a partially closed linear DNA product) by any of the methods described herein;
(b) contacting the linear DNA product (e.g. the closed linear DNA product or a partially closed linear DNA product), with a polymerase; and
(c) producing a transcription product encoded by the linear DNA product (e.g. the closed linear DNA product or a partially closed linear DNA product).

The invention provides a method for in vitro transcription of a linear DNA product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0 , to the first end of the linear double stranded region, and the second terminal adaptor molecule is appended to the mth intermediate adaptor molecule, or when m is 0, to the second end of the linear double stranded region; and
(c) contacting the linear DNA product with a polymerase; and
(d) producing a transcription product encoded by the linear DNA product.

The method may use adaptor molecules which generate a closed linear DNA product, such as adaptor molecules described herein. A method for in vitro transcription of a closed linear DNA product may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the closed linear DNA product, wherein the closed linear DNA product comprises a linear double-stranded region, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region;

(c) contacting the closed linear DNA product with a polymerase; and

(d) producing a transcription product encoded by the closed linear DNA product.

The method may use adaptor molecules which comprise protected nucleotides, such as the adaptor molecules described herein. A method for in vitro transcription of a linear DNA product may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides);

(c) contacting the linear DNA product with a polymerase; and

(d) producing a transcription product encoded by the linear DNA product.

The method may use adaptor molecules which comprise protected nucleotides and adaptor molecules which comprise a hairpin or a stem loop, such as the adaptor molecules described herein. A method for in vitro transcription of a partially closed linear DNA product may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;

(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides;

(c) contacting the partially closed linear DNA product with a polymerase; and

(d) producing a transcription product encoded by the partially closed linear DNA product.

The invention provides a method for producing a protein, wherein the method comprises introducing the linear DNA product (e.g. the closed linear DNA product or the partially closed linear DNA product), produced by the methods described herein, into a cell (e.g. a prokaryotic cell or a eukaryotic cell) or a cell-free expression system to generate a protein encoded by the linear DNA product (e.g. the closed linear DNA product or the partially closed linear DNA product).

The invention provides a method for producing a protein, wherein the method comprises:

(a) producing a linear DNA product (e.g. a closed linear DNA product or partially closed linear DNA product) by any of the methods described herein; and

(b) introducing the linear DNA product (e.g. the closed linear DNA product or partially closed linear DNA product)

into a cell (e.g. a prokaryotic cell or a eukaryotic cell) or a cell-free expression system to generate a protein encoded by the linear DNA product (e.g. the closed linear DNA product or partially closed linear DNA product).

The invention provides a method for producing a protein comprising:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region; and
(c) introducing the linear DNA product into a cell (e.g. a prokaryotic cell or a eukaryotic cell) or a cell-free expression system to generate a protein encoded by the linear DNA product.

The method may use adaptor molecules which generate a closed linear DNA product, such as adaptor molecules described herein. A method for producing a protein may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region; and
(c) introducing the closed linear DNA product into a cell (e.g. a prokaryotic cell or a eukaryotic cell) or a cell-free expression system to generate a protein encoded by the closed linear DNA product.

The method may use adaptor molecules which comprise protected nucleotides, such as the adaptor molecules described herein. A method for producing a protein may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides); and
(c) introducing the linear DNA product into a cell (e.g. a prokaryotic cell or a eukaryotic cell) or a cell-free expression system to generate a protein encoded by the linear DNA product.

The method may use adaptor molecules which generate a partially closed linear DNA product, such as adaptor molecules described herein. A method for producing a protein may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1 ;

(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides; and

(c) introducing the partially closed linear DNA product into a cell (e.g. a prokaryotic cell or a eukaryotic cell) or a cell-free expression system to generate a protein encoded by the partially closed linear DNA product.

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The linear DNA product or the closed linear DNA product may comprise a cassette. The desired protein might be encoded by the cassette.

The step of introducing the linear DNA product into a cell may be performed in vivo or in vitro.

The nuclease-resistant nucleotides (i.e. protected nucleotides) may be any protected nucleotides described herein.

## 5. Methods for cell transfection and cell transfection compositions

The invention provides a method for cell transfection of a linear DNA product (e.g. a closed linear DNA product or a partially closed linear DNA product), produced by any of the methods described herein, into a cell.

The invention provides a method for cell transfection of a linear DNA product (e.g. a closed linear DNA product or a partially closed linear DNA product) into a cell, wherein the method comprises:

(a) producing a linear DNA product (e.g. a closed linear DNA product or a partially closed linear DNA product) by any of the methods described herein;

(b) contacting a cell with the linear DNA product (e.g. the closed linear DNA product or a partially closed linear DNA product); and

(c) transfecting the linear DNA product (e.g. the closed linear DNA product or a partially closed linear DNA product) into the cytosol of the cell.

The invention provides a method for cell transfection of a linear DNA into a cell, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1, and wherein n+m is at least 1; and

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region;

(c) contacting the linear DNA product with the cell; and

(d) transfecting the linear DNA product into the cytosol of the cell.

The method may use adaptor molecules which generate a closed linear DNA product, such as adaptor molecules described herein. A method for cell transfection of a closed linear DNA product into a cell may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region;
(c) contacting the closed linear DNA product with the cell; and
(d) transfecting the closed linear DNA product into the cytosol of the cell.

The method may use adaptor molecules which comprise protected nucleotides, such as the adaptor molecules described herein. A method for cell transfection of a linear DNA product into a cell may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides);
(c) contacting the linear DNA product with the cell; and
(d) transfecting the linear DNA product into the cytosol of the cell.

The method may use adaptor molecules which comprise protected nucleotides, such as the adaptor molecules described herein. A method for cell transfection of a partially closed linear DNA product into a cell may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides;
(c) contacting the partially closed linear DNA product with the cell; and
(d) transfecting the partially closed linear DNA product into the cytosol of the cell.

The invention provides a cell transfection composition comprising a linear DNA product (e.g. a closed linear DNA product) produced by (or obtainable by) the methods described herein.

The cell transfection composition may or may not comprise a carrier e.g. an agent or a formulation. Preferably, the carrier promotes accumulation of the linear DNA product at a target site and/or protects the linear DNA product from undesirable interactions with biological milieu components and/or protects the linear DNA product from metabolism and/or degradation.

The invention further provides a cell transfection method comprising contacting (in vitro) a cell to be transfected with a linear DNA product (e.g. a closed linear DNA product) produced by the methods of the invention, and wherein the linear DNA product (e.g. the closed linear DNA product) is transfected into the cytosol of the cell.

The cell or cells to be transfected may be provided in a cell culture medium (e.g. in a Petri dish, culture vessel or well, etc). The linear DNA product (e.g. the closed linear DNA product) may be added directly to the cell culture medium or the cells may be added to a solution, such as saline, a buffered solution or a cell culture medium, comprising the linear DNA product (e.g. the closed linear DNA product).

The carrier may be a viral carrier or a non-viral carrier. Viral carriers include a lentiviral vector, an adenoviral vector, a retroviral vector, or an adeno associate viral vector for delivery of the linear DNA product. Non-viral carriers (or vectors) include complexing the linear DNA product with a cationic agent such as a cationic cell penetrating peptide (CPP); a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the linear DNA product. The carrier may be a nanoparticulate formulation used to encapsulate the linear DNA product (e.g. the closed linear DNA product).

The carrier may be a modification of the linear DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the linear DNA product at a target site and/or protects the linear DNA product from metabolism and/or degradation.

The invention further provides a cell obtainable by the methods of the invention. Thus, the cell may comprise a linear DNA product (e.g. the closed linear DNA product) produced by the methods of the invention.

The invention further provides a cell transfected with a linear DNA product (e.g. the closed linear DNA product) produced by the methods of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a linear DNA product (e.g. the closed linear DNA product) may be performed in vivo. For example the linear DNA product (e.g. the closed linear DNA product) may be administered to an organism (e.g. a subject) in need thereof. The organism (e.g. the subject) may be an animal, such as mammal (e.g. a human), a fungus, a micro-organism, or a plant.

Any or all of the linear DNA products (e.g. the closed linear DNA products) described herein may be delivered to a cell via delivery particles such as liposomes, nanoparticles, exosomes, macrovesicles, viral or non-viral vectors. Any or all of the linear DNA products (e.g. the closed linear DNA products) described herein may be delivered to a cell using a gene-gun. Any or all of the linear DNA products (e.g. the closed linear DNA products) described herein may be delivered to a cell by electroporation. Any or all of the linear DNA products (e.g. the closed linear DNA products) described herein may be delivered to a cell by hydrodynamic needle. The linear DNA products (e.g. the closed linear DNA products) described herein may be delivered to a cell without a carrier.

The nanoparticle is preferably a self-assembled nanoparticle. The nanoparticle may be a nanoparticle which is produced by a process in which pre-existing components (e.g. a lipid component, a DNA product described herein) form an organized structure as a consequence of specific, local interactions among the components themselves, without external direction.

The DNA product and the lipid component may reversibly interact to form a self-assembled nanoparticle. The DNA product and the lipid component may reversibly interact in the self-assembled nanoparticle through intermolecular forces. The DNA product and the lipid component may reversibly interact in the self-assembled nanoparticle through non-covalent interactions. The DNA product and the lipid component may reversibly interact in the self-assembled nanoparticle through hydrogen bonds, van der Waals, hydrophobic, and/or electrostatic interactions. The DNA product and the lipid component may not be conjugated or linked by forces other than inter-molecular forces in the self-assembled nanoparticle.

**6. Pharmaceutical compositions and methods for producing pharmaceutical compositions**

The invention provides a pharmaceutical composition comprising a linear DNA product (e.g. a closed linear DNA product) described herein, and a pharmaceutically acceptable carrier or excipient.

The invention provides a pharmaceutical composition comprising a linear DNA product (e.g. a closed linear DNA product) produced by (or obtainable by) the methods described herein, and a pharmaceutically acceptable carrier or excipient.

The invention provides a method for producing a pharmaceutical composition comprising the linear DNA product, wherein the method comprises performing the method described herein and formulating the resulting linear DNA product with a pharmaceutically acceptable carrier or excipient.

The invention provides a method for producing a pharmaceutical composition, wherein the method comprises:

(a) producing a linear DNA product (e.g. a closed linear DNA product) by any of the methods described herein;
(b) formulating the linear DNA product (e.g. the closed linear DNA product) with a pharmaceutically acceptable carrier or excipient.

The invention provides a method for producing a pharmaceutical composition, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate a linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region; and
(c) formulating the linear DNA product with a pharmaceutically acceptable carrier or excipient

The method may use adaptor molecules which generate a closed linear DNA product, such as adaptor molecules described herein. A method for producing a pharmaceutical composition may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate a closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region; and
(c) formulating the closed linear DNA product with a pharmaceutically acceptable carrier or excipient.

The method may use adaptor molecules which comprise protected nucleotides, such as the adaptor molecules described herein. A method for producing a pharmaceutical composition may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides (i.e. protected nucleotides);

(c) formulating the linear DNA product with a pharmaceutically acceptable carrier or excipient.

The method may use adaptor molecules which generate a partially closed linear DNA product, such as adaptor molecules described herein. A method for producing a pharmaceutical composition may comprise:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;

(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides; and

(c) formulating the partially closed linear DNA product with a pharmaceutically acceptable carrier or excipient.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the linear DNA product (e.g. closed linear DNA product) can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochiear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

**7. Linear DNA products**

The invention provides a linear DNA product (e.g. a closed linear DNA product) as described herein.

The invention provides a closed linear DNA product comprising a linear double-stranded region, wherein the linear double-stranded region comprises a linear portion of a double-stranded DNA molecule, and wherein the linear double-stranded region is closed at a first end by a first terminal adaptor molecule and closed at a second end by the second terminal adaptor molecule, wherein the closed linear DNA product comprises n intermediate adaptor molecules between the first end of the linear double stranded region and the first terminal adaptor molecule, and m intermediate adaptor molecules between the second end of the linear double-stranded region and the second terminal adaptor molecule, wherein m and m are 0 or an integer of at least 1, and n+m is at least 1.

The invention provides a closed linear DNA product comprising a linear portion of a double-stranded DNA molecule, wherein the linear portion of the double-stranded DNA molecule is closed at a first end by a first terminal adaptor molecule and closed at a second end by a second terminal adaptor molecule wherein the closed linear DNA product comprises n intermediate adaptor molecules between the first end of the linear double stranded region and the first terminal adaptor molecule, and m intermediate adaptor molecules between the second end of the linear double-stranded region and the second terminal adaptor molecule wherein m and m are 0 or an integer of at least 1, and n+m is at least 1. The closed linear DNA product may comprise a barcode, a binding moiety, a polyA signal, a polyA sequence, a label and/or a targeting sequence.

The invention provides a linear DNA product comprising a linear double-stranded region, wherein the linear double-stranded region comprises a linear portion of a double-stranded DNA molecule, and wherein the first terminal adaptor molecule is ligated to a first end of the linear double-stranded region and the second terminal adaptor molecule is ligated to a second end of the linear double-stranded region, wherein the linear DNA product comprises n intermediate adaptor molecules between the first end of the linear double stranded region and the first terminal adaptor molecule, and m intermediate adaptor molecules between the second end of the linear double-stranded region and the second terminal adaptor molecule, and wherein the first and second terminal adaptor molecules are nucleic acid molecules that comprise one or more nuclease-resistant nucleotides wherein m and m are 0 or an integer of at least 1, and n+m is at least 1. The linear DNA product may comprise a barcode, a binding moiety, a polyA signal, a polyA sequence, a label and/or a targeting sequence.

The invention provides a linear DNA product comprising a linear portion of a double-stranded DNA molecule, wherein a first terminal adaptor molecule is ligated to a first end of the linear portion of the double-stranded DNA molecule and the second terminal adaptor molecule is ligated to a second end of the linear portion of the double-stranded DNA molecule, and wherein the first and second terminal adaptor molecules are nucleic acid molecules that comprise one or more nuclease-resistant nucleotides wherein the linear DNA product comprises n intermediate adaptor molecules

between the first end of the linear double stranded region and the first terminal adaptor molecule, and m intermediate adaptor molecules between the second end of the linear double-stranded region and the second terminal adaptor molecule, and wherein the first and second terminal adaptor molecules are nucleic acid molecules that comprise one or more nuclease-resistant nucleotides wherein m and m are 0 or an integer of at least 1, and n+m is at least 1.

The invention provides a partially closed linear DNA product comprising a linear portion of a double-stranded DNA molecule, wherein a first terminal adaptor molecule is ligated to a first end of the linear portion of the double-stranded DNA molecule and the second terminal adaptor molecule is ligated to a second end of the linear portion of the double-stranded DNA molecule, and wherein the first terminal adaptor molecule is a nucleic acid molecule that comprise one or more nuclease-resistant nucleotides, and wherein the linear portion of the double-stranded DNA molecule is closed at the second end by the second terminal adaptor molecule, wherein the linear DNA product comprises n intermediate adaptor molecules between the first end of the linear double stranded region and the first terminal adaptor molecule, and m intermediate adaptor molecules between the second end of the linear double-stranded region and the second terminal adaptor molecule, and wherein the first and second terminal adaptor molecules are nucleic acid molecules that comprise one or more nuclease-resistant nucleotides wherein m and m are 0 or an integer of at least 1, and n+m is at least 1. Thus, the invention provides a partially closed linear DNA product which is closed (or covalently closed) at a second end and open at a first end. The partially closed linear DNA product comprises one or more nuclease-resistant nucleotides in the open-end region and/or at the first end. The open-end region may be at the 3' end or 5' end of the molecule. The partially closed linear DNA product may comprise a barcode, a binding moiety, a polyA signal, a polyA sequence, a label and/or a targeting sequence.

The open-end region refers to at least 5, at least 10, at least 15, or at least 20 base pairs located closest to the open end of the DNA product. The partially closed linear DNA product may comprise one or more nuclease-resistant nucleotides in a sense and/or an antisense strand. Thus, for example, one or more nucleotides of the 20 base pairs located closest to the open end of the partially closed linear DNA product may be a nuclease-resistant nucleotide. Preferably, the partially closed linear DNA product comprises at least 5 nuclease-resistant nucleotides in the open-end region.

The partially closed linear DNA product may comprise a double-stranded DNA portion that is closed at a first end and open at a second end. The partially closed linear DNA product may comprise a double-stranded DNA portion that is closed at a first end by a single-stranded portion (i.e. it may comprise a first hairpin at the first end) and open at a second end. The partially closed linear DNA product may comprise one or more nuclease-resistant nucleotides in an open-end region adjacent to the second end. The open-end region adjacent to the second end may be at the 3' end or 5' end of the molecule. The open-end region adjacent to the second end may comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 nucleotides located at the second end of the partially closed linear DNA product. This is to say that the open-end region adjacent to the second end may comprise any nucleotide between and including the end nucleotide of the second end and a nucleotide at location 2, 3, 4, 5, 6, 7, 8, 9, 10, 11, 12, 13, 14, 15, 16, 17, 18, 19, 20, 25, 30, 35, 40, 45, or 50 counting from the end nucleotide of the second end.

The open-end region adjacent to the second end may comprise a sense strand and an antisense strand. The open-end region adjacent to the second end may comprise one or more nuclease-resistant nucleotides in the sense strand or the antisense strand. The open-end region adjacent to the second end may comprise one or more nuclease-resistant nucleotides in both the sense and antisense strand. The open-end region adjacent to the second end may comprise two or more, three or more, four or more, or five or more nuclease-resistant nucleotides in both the sense and antisense strand. Preferably, the open-end region adjacent to the second end comprises five nuclease-resistant nucleotides in both the sense and antisense strand.

The partially closed linear DNA product may comprise a hairpin-loop at the 5' end or the 3' end. The partially closed linear DNA product may comprise a first terminal adaptor molecule at a first end and a second terminal adaptor molecule at a second end. The first terminal adaptor molecule may comprise a hairpin and a second adaptor may comprise one or more protected nucleotides (i.e. nucleotides resistant to nuclease (e.g. exonuclease) digestion). The hairpin may confer resistance to nuclease (e.g. exonuclease) digestion. The presence of protected nucleotides may confer resistance to nuclease (e.g. exonuclease) digestion. The partially closed linear DNA product may be a DNA molecule having a double-stranded portion closed at a first end by ligation of a first adaptor (e.g. hairpin adaptor) to the first end and comprising at a second end a double stranded linear adaptor comprising protected nucleotides (i.e. nucleotides resistant to nuclease (e.g. exonuclease) digestion).

The partially closed linear DNA product may comprise (i) a cassette, wherein the cassette comprises a sense strand and an antisense strand; and (ii) one or more nuclease-resistant nucleotides in an open-end region of the partially closed linear DNA product, wherein the open-end region is 5' of the sense strand of the cassette. The partially closed linear DNA product may comprise (i) a cassette, wherein the cassette comprises a sense strand and an antisense strand; and (ii) one or more nuclease-resistant nucleotides in an open-end region of the partially closed linear DNA product, wherein the open-end region is 5' of the antisense strand of the cassette. The partially closed linear DNA product may comprise (i) a cassette, wherein the cassette comprises a sense strand and an antisense strand; and (ii) one or more nuclease-resistant nucleotides in an open-end region of the partially closed linear DNA product, wherein the open-end region is 3' of the sense strand of the cassette. The partially closed linear DNA product may comprise (i) a cassette, wherein the cassette comprises a sense strand and an antisense strand; and (ii) one or more nuclease-resistant nucleotides in an open-end region of the partially closed linear DNA product, wherein the open-end region is 3' of the antisense strand of the cassette.

The partially closed linear DNA product may comprise (i) a cassette, wherein the cassette comprises a sense strand and an antisense strand; (ii) one or more nuclease-resistant nucleotides in an open-end region of the partially closed linear DNA product, wherein the open-end region is 5' of the sense strand of the cassette; and (iii) one or more nuclease-resistant nucleotides in an open-end region of the partially closed linear DNA product, wherein the open-end region is 3' of the antisense strand of the cassette.

The partially closed linear DNA product may comprise (i) a cassette, wherein the cassette comprises a sense strand and an antisense strand; (ii) one or more nuclease-resistant nucleotides in an open-end region of the partially closed linear DNA, wherein the open-end region is 3' of the sense strand of the cassette; and (iii) one or more nuclease-resistant nucleotides in an open-end region of the partially closed linear DNA product, wherein the open-end region is 5' of the antisense strand of the cassette.

The closed linear DNA product, linear DNA product or partially closed linear DNA product may comprise a cassette, optionally a single cassette. The linear portion of a double-stranded DNA molecule (of a closed linear DNA product, linear DNA product or partially closed linear DNA product) may comprise a cassette, optionally a single cassette. Accordingly, the cassette (or single cassette) is located between the first and second terminal adaptor molecules.

The term "single cassette" as used herein is intended to encompass a molecules that do not comprise or consist of a plurality of cassettes. That is to say that the closed linear DNA product, linear DNA product, or partially closed linear DNA product, comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

Embodiments of linear DNA products of the invention are described below:

1. A linear DNA product, wherein the first terminal adaptor molecule is a hairpin and the second terminal adaptor molecule is a hairpin, n=1 and m=1, and the intermediate adaptor molecules comprise a spacer sequence.

2. A linear DNA product, wherein the first terminal adaptor molecule is a hairpin and the second terminal adaptor molecule comprises one or more nuclease-resistant nucleotides, n=0 and m=3, and the m intermediate adaptor molecules comprise a spacer sequence, a promoter sequence and an open reading frame.

3. A linear DNA product, wherein the first terminal adaptor molecule is a hairpin and the second terminal adaptor molecule is a hairpin, n=2 and m=0, and the n intermediate adaptor molecules comprise a spacer sequence and a bar code, with each closed DNA product comprising a different barcode, i.e. each individual nth intermediate adaptor molecule comprises a different bar code sequence.

4. A linear DNA product, wherein the first terminal adaptor molecule comprises one or more nuclease-resistant nucleotides, and the second terminal adaptor molecule comprises one or more nuclease-resistant nucleotides, n=1 and m=1, and the nth intermediate adaptor sequence comprises a linker sequence and the mth intermediate adaptor molecule comprises a polyA signal sequence.

The invention provides a linear DNA product (e.g. a closed linear DNA product or a partially closed linear DNA product) obtainable by any of the methods described herein.

## 8. Uses and applications

The invention provides a use of a linear DNA product (e.g. a closed linear DNA product) as described herein in the production of viral or non-viral delivery system.

The invention provides a use of a linear DNA product (e.g. a closed linear DNA product) in the production of viral or non-viral delivery system, wherein the linear DNA product (e.g. the closed linear DNA product) is produced by performing the method described herein.

The invention provides a viral or non-viral delivery system comprising a linear DNA product (e.g. a closed linear DNA product) as described herein. The invention provides a viral or non-viral delivery system comprising a linear DNA product (e.g. a closed linear DNA product), wherein the linear DNA product (e.g. the closed linear DNA product) is produced by performing the method described herein.

Viral vectors

Methods of producing viral vectors, such as AAV vectors, are known in the art. The most widely used method involves the co-transfection of HEK293 by three bacterial plasmids. The first plasmid encodes the *Rep* and *Cap* element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli;* 3) the incorporation of the plasmid backbone into the viral capsid (e.g. AAV capsid) might be challenging (e.g. issues with antibiotic resistance markers). Methods of producing viral vectors may be using other cell lines. For example, a cell line suitable for production of viral vectors may be a Vero cell, or any other stable cell line. Together these represent the main bottleneck in viral vector production (e.g. AAV production). Thus, the methods described herein provide a linear DNA product (e.g. a closed linear DNA product) suitable for use in production of viral vectors. The linear DNA product (e.g. the closed linear DNA product) overcomes the above issues with plasmid vectors.

The invention provides a method of producing a viral vector, the method comprising introducing the linear DNA product (e.g. a closed linear DNA product) produced by the methods described herein into a cell under conditions such that the viral vector is produced. The linear DNA product (e.g. the closed linear DNA product) may encode at least one element required for the production of the viral vector. For example, the linear DNA product (e.g. the closed linear DNA product) may encode Rep and/or Cap elements. The linear DNA product (e.g. the closed linear DNA product) may encode the helper plasmid elements. The linear DNA product (e.g. the closed linear DNA product) may encode Rep, Cap and helper plasmid elements. The linear DNA product (e.g. the closed linear DNA product) may encode a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. the closed linear DNA product) to a cell, comprising contacting the viral vector produced by the methods described herein with the cell. The cell may be an animal cell, preferably mammal cell, such as human cell.

The invention also provides a cell obtainable by the methods described herein.

Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the linear DNA product (e.g. a closed linear DNA product) produced by the methods described herein is suitable for use in production of non-viral vectors. The linear DNA product (e.g. the closed linear DNA product) produced by the methods of the invention overcomes the issues of using plasmid vectors in non-viral vector preparation. For example, the linear DNA product (e.g. the closed linear DNA product), unlike plasmid DNA, does not comprise a bacterial backbone, allowing more transgene copies per mg of DNA. In addition, the linear DNA product (e.g. the closed linear DNA product) does not comprise antibiotic resistant genes and bacterial contaminants. Moreover, the linear DNA product (e.g. the closed linear DNA product) provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the linear DNA product (e.g. a closed linear DNA product) with the cell. The cell may be an animal cell, preferably mammal cell, such as human cell.

The invention also provides a cell obtainable by the methods described herein.

General therapeutic and diagnostic uses

The linear DNA product (e.g. a closed linear DNA product) produced by the methods described herein is particularly suitable for use in therapy. The invention provides a linear DNA product (e.g. a closed linear DNA product) as described herein for use in therapy. The invention provides a linear DNA product (e.g. a closed linear DNA product) obtainable by the method described herein for use in therapy. The linear DNA product (e.g. the closed linear DNA product) may encode a sequence of a therapeutic protein, a part of a vaccine, or an element of a genetic engineering mechanism, which is used to treat a disease or infection in a subject.

The invention further provides the linear DNA product (e.g. a closed linear DNA product) as described herein for use as a medicament. The invention further provides the linear DNA product (e.g. a closed linear DNA product) obtainable by the methods described herein for use as a medicament. The invention also provides the use of a linear DNA product (e.g. a closed linear DNA product) as described herein for the manufacture of a medicament for treating a disease. The invention also provides the use of a linear DNA product (e.g. a closed linear DNA product) obtainable by the methods described herein for the manufacture of a medicament for treating a disease.

The linear DNA product (e.g. the closed linear DNA product) may encode a sequence of a therapeutic protein, a part of a vaccine, or an element of a genetic engineering mechanism, which is used to treat a disease or infection in a subject.

The invention further provides the linear DNA product produced by the methods described herein for use in treating a disease.

The invention also provides a method of treating a disease in a subject comprising administering to the subject a linear DNA product (e.g. a closed linear DNA product) described herein. The invention also provides a method of treating a disease in a subject comprising administering to the subject the linear DNA product (e.g. a closed linear DNA product) obtainable by the methods described herein. Preferably, the amount of the linear DNA product administered to the subject is a therapeutic active amount.

The linear DNA product (e.g. the closed linear DNA product) described herein may be used to treat any disease or disorder. For example, the linear DNA product (e.g. the closed linear DNA product) may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product (e.g. the closed linear DNA product) is used to treat a genetic disorder. More preferably still, the linear DNA product (e.g. the closed linear DNA product) is used to treat a monogenic disorder. For example, the linear DNA product (e.g. the closed linear DNA product), may be used to treat sickle cell anaemia, cystic fibrosis, Huntington disease, and Duchenne's Muscular Dystrophy, Haemophilia A, $\alpha$1-antitrypsin deficiency, primary ciliary dyskinesia, or respiratory distress syndrome of prematurity.

A subject treated with the linear DNA product (e.g. a closed linear DNA product) may receive the linear DNA product (e.g. a closed linear DNA product) in the form of any of the pharmaceutical compositions described herein.

A subject treated with the linear DNA product (e.g. a closed linear DNA product) may receive the linear DNA product (e.g. the closed linear DNA product) in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the linear DNA product into the subject's body as described in more detail above (see "Methods for producing a pharmaceutical composition"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the linear DNA product that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides the use of the linear DNA product (e.g. a closed linear DNA product) as described herein in a method of diagnosing a disease and/or a disorder. The invention also provides the use of the linear DNA product (e.g. the closed linear DNA product) obtainable by the methods described herein in a method of diagnosing a disease and/or a disorder.

The invention provides the use of the linear DNA product (e.g. the closed linear DNA product) in the "in vitro" diagnosis of a disease. The invention provides the use of the linear DNA product (e.g. the closed linear DNA product) obtainable by the methods described herein in the "in vitro" diagnosis of a disease.

The invention also provides the linear DNA product (e.g. the closed linear DNA product) for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to diagnose a genetic disorder. More preferably still, the linear DNA product is used to diagnose a monogenic disorder. For example, the linear DNA product may be used to diagnose sickle cell anaemia, cystic fibrosis, Huntington disease, and Duchenne's Muscular Dystrophy, Haemophilia A, $\alpha$1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the linear DNA product (e.g. closed linear DNA product).

To facilitate detection and/or quantification of the linear DNA product, the linear DNA product (e.g. the closed linear DNA product) may be attached or bound to a functional portion. The functional portion may be any functional portion described herein. For example the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the linear DNA product. For example, the signal may be measured by flow cytometry and/or fluorescence-activated cell sorting of a linear DNA product attached to a fluorescent probe.

The linear DNA product (e.g. the closed linear DNA product) may be detected by being bound to a capture moiety, for example in a lateral flow assay. In this example, the functional portion is a protein, for example, an antibody specific for the capture moiety. The capture of the antibody attached to the linear DNA product may produce a visual signal (e.g. a band of a different colour).

The invention also provides a method that combines diagnosis of a disease with a treatment of the disease.

Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the linear DNA product (e.g. the closed linear DNA product) may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the linear DNA product (e.g. the closed linear DNA product) described herein for use in cell therapy. The invention provides the linear DNA product (e.g. the closed linear DNA product) obtainable by the methods described herein for use in cell therapy.

The invention provides the linear DNA product (e.g. the closed linear DNA product) described herein for use in cell therapy. The invention provides the linear DNA product (e.g. the closed linear DNA product) obtainable by the methods described herein for use in cell therapy.

Preferably, cell therapy is ex-vivo cell therapy. The cell may be an animal cell, preferably mammal cell, such as human cell.

The invention also provides a cell obtainable by any methods described herein. For example, the cell may be suitable for use in cell therapy.

Vaccines

The linear DNA products (e.g. the closed linear DNA products) produced by the methods described herein are particularly suitable for use in vaccine production. A vaccine may comprise a linear DNA product (e.g. a closed linear DNA product) described herein. A vaccine may comprise a linear DNA product (e.g. a closed linear DNA product) obtainable by the methods described herein. Alternatively, the linear DNA product (e.g. the closed linear DNA product) may be used to produce a vaccine, preferably an mRNA-based vaccine. For example, vaccines such as the BioNTech and Moderna mRNA vaccines against COVID-19.

Thus, the invention provides the use of the linear DNA product (e.g. the closed linear DNA product) described herein in the production of a vaccine. The invention also provides the use of the linear DNA product (e.g. the closed linear DNA product) obtainable by the methods described herein in the production of a vaccine.

The linear DNA product (e.g. the closed linear DNA product) may encode an antigen, which may cause an immune response in a subject. The subject may be human. Preferably, the antigen is encoded on the cassette.

CAR-T cells

The invention provides the use of a linear DNA product (e.g. the closed linear DNA product) described herein in the production of a CAR-T cell. The invention provides the use of the linear DNA product (e.g. the closed linear DNA product) obtainable by the methods described herein in the production of a CAR-T cell.

The invention provides a method for producing a genetically engineered CAR-T cell comprising: (a) introducing the linear DNA product (e.g. a closed linear DNA product) described herein into a T cell; and (b) expressing a gene of interest encoding by the linear DNA product (e.g. the closed linear DNA product). Preferably, the gene of interest is a tumour-specific CAR.

The invention provides a method for producing a genetically engineered CAR-T cell comprising: (a) introducing the linear DNA product (e.g. a closed linear DNA product) obtainable by the methods described herein into a T cell; and (b) expressing a gene of interest encoding by the linear DNA product (e.g. the closed linear DNA product). Preferably, the gene of interest is a tumour-specific CAR.

The method may further comprise, before step (a), a step of removing mononuclear cells from a patient. Preferably, the step of removing is performed using leukapheresis. Preferably the mononuclear cells are T cells. The method may further comprise, after step (b), a step of returning the genetically engineered cells to the patient.

The invention also provides an engineered T cell obtainable by any methods described herein. The engineered T cell may be suitable for use in CAR T-cell therapy.

CRISPR delivery

The products produced by the methods described herein are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The linear DNA product (e.g. the closed linear DNA product) may comprise a gene sequence encoding any component of the CRIPSR machinery. The linear DNA product (e.g. the closed linear DNA product) may encode all components of the CRIPSR machinery.

The linear DNA product (e.g. the closed linear DNA product) may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1, or MAD7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The linear DNA product (e.g. the closed linear DNA product) encoding the repair template may be delivered to a cell by a nanoparticle, a non-viral vector or a viral vector, or without the aid of any carrier. The linear DNA product (e.g. the closed linear DNA product) encoding the repair template may be delivered to a cell by electroporation. The linear DNA product (e.g. the closed linear DNA product) encoding the repair template may be delivered to a cell by hydrodynamic needle.

The linear DNA product (e.g. the closed linear DNA product) may comprise (or further comprise) a gene sequence encoding a nuclease protein of a CRISPR system (e.g. Cas9, Cpf1, or MAD7), and/or a guide RNA. The linear DNA product (e.g. the closed linear DNA product) may comprise (or further comprise) a gene sequence encoding a nuclease protein of a CRISPR system (e.g. Cas9, Cpf1, or MAD7). The linear DNA product (e.g. the closed linear DNA product) may comprise (or further comprise) a gene sequence encoding a guide RNA. The linear DNA product (e.g. the closed linear DNA product) may comprise (or further comprise) a gene sequence encoding a nuclease protein of a CRISPR system (e.g. Cas9, Cpf1, or MAD7) and a guide RNA. The linear DNA product (e.g. the closed linear DNA product) may comprise (or further comprise) a gene sequence encoding a genomic target to be modified (e.g. a spacer). The linear DNA product (e.g. the closed linear DNA product) may be ligated to a vector. The vector may comprise a sequence of some of the components of the CRIPSR system. The vector may comprise a sequence of guide RNA or a sequence of a part of the guide RNA. If the vector comprises a sequence of a part of the guide RNA, the linear DNA product (e.g. the closed linear DNA product) may comprise the missing sequence part of the guide RNA, so that upon ligation, the ligated vector comprises a full sequence of a guide RNA. The nuclease of the CRISPR system and guide RNA may be encoded on a single vector or on two different vectors. The linear DNA product (e.g. the closed linear DNA product) may encode the nuclease of the CRISPR system and guide RNA. One linear DNA product (e.g. one closed linear DNA product) may encode the nuclease of the CRISPR system, and the other linear DNA product (e.g. the other closed linear DNA product) may encode guide RNA.

The linear DNA product (e.g. the closed linear DNA product) may be for use in CRISPR-Cas mediated repair by recombination, homology-directed repair, or non-homologous end joining.

If the nuclease of the CRISPR system and the guide RNA are encoded by a different linear DNA product (e.g. a different closed linear DNA product), they may be part of a different or the same delivery mechanism. For example, the linear DNA product (e.g. the closed linear DNA product) encoding the nuclease of the CRISPR system may be delivered to a cell by a first nanoparticle, non-viral vector or viral vector, whereas the linear DNA product (e.g. the closed linear DNA product) encoding the guide RNA may be delivered to a cell by a second nanoparticle, a non-viral vector, or viral vector. For example, the linear DNA product (e.g. the closed linear DNA product) encoding the nuclease of the CRISPR system and the linear DNA product (e.g. the closed linear DNA product) encoding the guide RNA may be delivered to a cell by the same nanoparticle, non-viral vector or viral vector.

If the nuclease of the CRISPR system and the guide RNA are encoded by the same linear DNA product (e.g. the closed linear DNA product) (or by a vector that comprises the linear DNA product (e.g. the closed linear DNA product)) they may be part of the same delivery mechanism. For example, the linear DNA product (e.g. the closed linear DNA product), or the vector, encoding the nuclease of the CRISPR system and the guide RNA may be delivered to a cell by a nanoparticle, a non-viral vector or a viral vector.

The linear DNA product (e.g. the closed linear DNA product) encoding the nuclease of the CRISPR system and the linear DNA product (e.g. the closed linear DNA product) encoding the guide RNA may be delivered to a cell by electroporation. The linear DNA product (e.g. the closed linear DNA product) encoding the nuclease of the CRISPR system and the linear DNA product (e.g. the closed linear DNA product) encoding the guide RNA may be delivered to a cell by hydrodynamic needle.

Thus, the invention provides the linear DNA product (e.g. the closed linear DNA product) described herein for use in CRISPR system delivery to a cell. The invention provides a method of delivering the CRISPR system to a cell, comprising contacting the linear DNA product (e.g. the closed linear DNA product) described herein with a cell. The invention also provides the linear DNA product (e.g. the closed linear DNA product) obtainable by the methods described herein for use in CRISPR system delivery to a cell. The invention provides a method of delivering the CRISPR system to a cell, comprising contacting the linear DNA product (e.g. the closed linear DNA product) obtainable by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

The linear DNA product produced by the methods described herein may be used in transcription, to generate RNA, preferably mRNA, in vitro or in vivo.

## 9. Kits

The invention provides a kit comprising components required to carry out the method described herein. The kit comprises at least:

(a) first and second terminal adaptor molecules;
(b) n+m intermediate adaptor molecules (where n and m are each 0 or an integer of at least 1, and n+m is at least 1)
(c) an endonuclease; and
(d) a ligase.

The kit may additionally comprise a DNA polymerase, at least one buffer and/or a nuclease.

The first and/or second terminal adaptor molecule may comprise or consist of the sequence of SEQ ID NO: 9 or a portion thereof. The first and/or second terminal adaptor molecule may comprise at least 13, at least 14, at least 15, at least 16, at least 17, at least 18, or at least 19 contiguous nucleotides of SEQ ID NO: 9. The double-stranded portion of the first and/or second terminal adaptor molecule may comprise the sequence of SEQ ID NO: 10 or a portion thereof. The double-stranded portion of the first and/or second terminal adaptor molecule may comprise at least 8, at least 9, at least 10, at least 11, at least 12, at least 13, at least 14 or at least 15 contiguous nucleotides of SEQ ID NO: 10. The single-stranded portion of the first and/or second terminal adaptor molecule may comprise a sequence of ACTCA. The single-stranded portion of the first terminal adaptor molecule and/or the second terminal adaptor molecule may comprise at least 1, at least 2, at least 3, at least 4 or at least 5 contiguous nucleotides of the sequence ACTCA. The first and/or second terminal adaptor molecule may comprise the sequence of SEQ ID NO: 12. The first and/or second adaptor molecule may comprise at least 13, at least 14, at least 15, at least 16, at least 17, at least 18, or at least 19 contiguous nucleotides of SEQ ID NO:12. The first and/or second terminal adaptor molecule may comprise or consist of the sequences of SEQ ID NO:13 and/or SEQ ID NO:14. The first and/or second terminal adaptor molecule may comprise or at least 15, 14, 13, 12, 11, 10, 19, 8, 7, 6, 5, contiguous nucleotides thereof.

The first and second adapter molecules may comprise an identical nucleic acid sequence. The first and second adapter molecules may comprise a different nucleic acid sequence. The first terminal adaptor molecule may comprise one or more protected nucleotides and the second terminal adaptor molecule may comprise a hairpin or a stem loop region.

The first and second terminal adaptor molecule and n+m intermediate adaptor molecules may be provided in a kit together or separately.

The first terminal adaptor molecule and/or the second terminal adaptor molecule may comprise one or more locked nucleic acids (LNAs).

The first terminal adaptor molecule and/or the second terminal adaptor molecule and/or n+m intermediate adaptor molecules may comprise one or more protected nucleotides (i.e. nuclease-resistant nucleotides), such as phosphorothioated nucleotides.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a BbsI, BsaI, BsmBI, BspQI, BtgZI, Esp3I, SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, Bful, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6!, BstF51, BstMAI, BstV11, BstV2!, Bsul, BtgZI, BtsCI, BtsI-v2, BtsMutI, Bve!, Csel, CspCI, Eam1104I, EarI, Ecil, Eco31I, Eco57!, Esp3I, FaqI, FauI, FokI, GsuI, HgaI, HphI, HpyAV, LguI, LmnI, Lsp1109I, LweI, MboII, MlyI, MmeI, MnlI, Mva1269!, NmeAIII, PaqCI, PciSI, PctI, PleI, PpsI, PsrI, SchI, SfaNI, TaqII, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The nuclease may be an exonuclease e.g. exonuclease I, exonuclease III and/or exonuclease VIII.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.


## BRIEF DESCRIPTION OF THE DRAWINGS

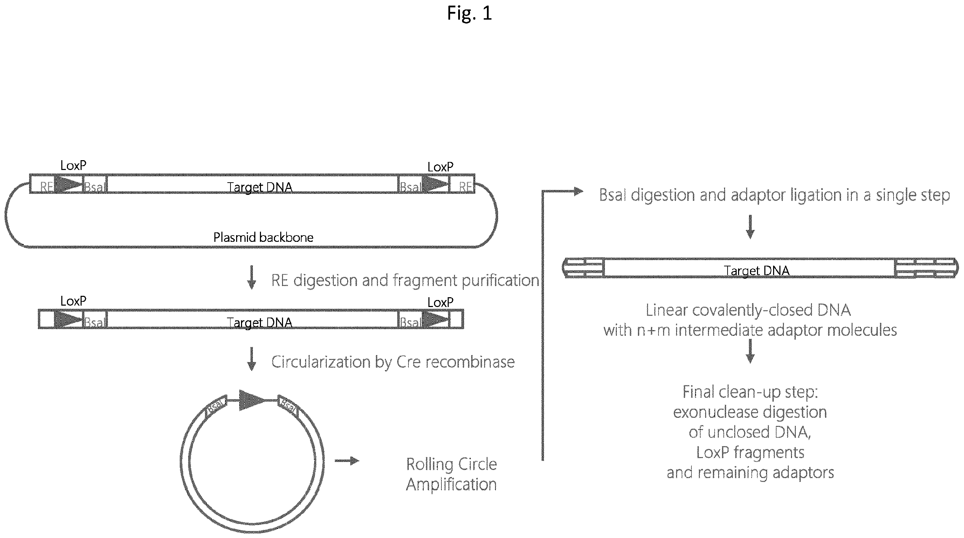

FIG. 1 illustrates the workflow to obtain closed linear DNA product by digestion and ligation of intermediate and terminal adaptor molecules in a single step, starting from amplified DNA obtained through rolling circle amplification (RCA) of a circular DNA template generated through the action of Cre recombinase on substrates containing two LoxP sequences (SEQ ID NO: 11) flanking the DNA of interest in the same direction.

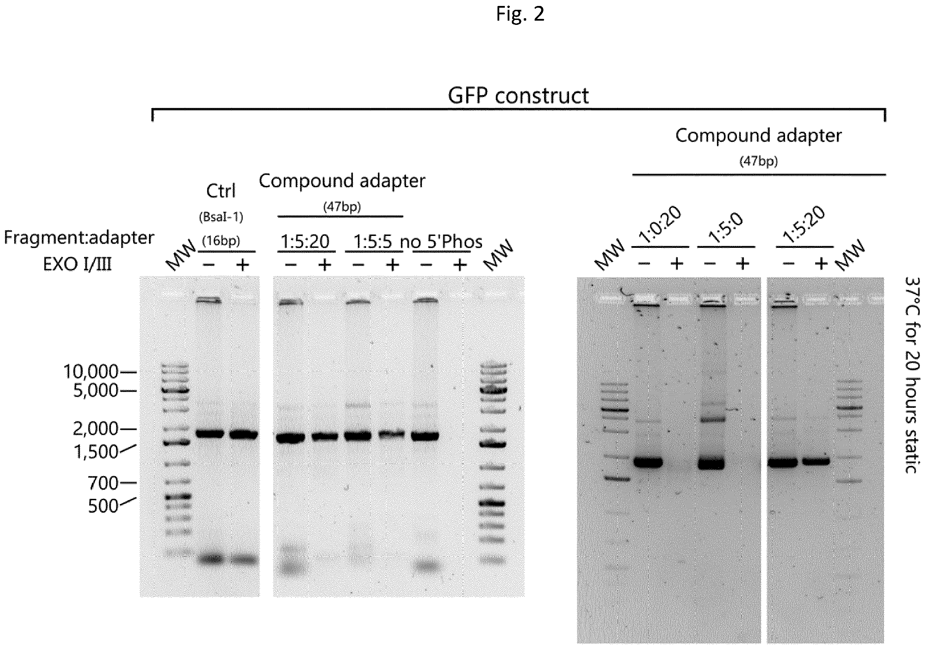

FIG. 2 illustrates the production of nuclease-resistant DNA product encoding a GFP by ligation of intermediate and terminal adaptor molecules to the first and second ends of a linear double-stranded DNA molecule.

The sequences discussed in the application are provided in the table below:

Table 1. Sequences discussed in the application.

| SEQ ID NO: | | |
|---|---|---|
| 1 | Ctrl Adaptor-BsaI | /5Phos/AGGGCTAACATTTGTTGGCCACTCAGGCCAACAAATGT TAG |
| 2 | Long-Adaptor-1 | /5Phos/AGGGCTGGCCACTCAGGCCAACAAATGTTAGACTCAC TAACATTTGTTGGCCTGAGTGGCCAG |

(continued)

| SEQ ID NO: | | |
|---|---|---|
| 3 | Long-Adaptor-2 | /5Phos/AGGGCTAACATTTGTTGGCCACTCAGGCCAACAAATGT TAGACTCACTAACATTTGTTGGCCTGAGTGGCCAACAAATGTT AG |
| 4 | Long-Adaptor-3 | /5Phos/AGGGCGAGCCTAACATTTGTTGGCCACTCAGGCCAAC AAATGTTAGACTCACTAACATTTGTTGGCCTGAGTGGCCAACA AATGTTAGGCTCG |
| 5 | Long-Adaptor-4 | /5Phos/AGGGCGAGCTGAGCCTAACATTTGTTGGCCACTCAGG CCAACAAATGTTAGACTCACTAACATTTGTTGGCCTGAGTGGC CAACAAATGTTAGGCTCAGCTCG |
| 6 | Compound-Adaptor-1A | /5Phos/AGGGCGAGCTGAGCCTAACATTTGTTGGCCAC |
| 7 | Compound-Adaptor-1B | /5Phos/CTGAGTGGCCAACAAATGTTAGGCTCAGCTCG |
| 8 | Compound-Adaptor-1C | /5Phos/TCAGGCCAACAAATGTTAGACTCACTAACATTTGTTGG C |
| 9 | Adaptor sequence | AGGGCTAACATTTGTTGGCC |
| 10 | Adaptor sequence | GGCCAACAAATGTTAG |
| 11 | loxP | ATAACTTCGTATAATGTATG CTATACGAAG TTAT |
| 12 | Adaptor sequence | AGGGCTAACATTTGTTGGCCACTCAGGCCA CAAATGTTAG |
| 13 | Adaptor sequence | GGCCAACAAATGTTAG |
| 14 | Adaptor sequence | AGGGCTAACATTTGTTGGCC |

**CLAUSES**

1. A method for producing a linear deoxyribonucleic acid (DNA) product, wherein the method comprises:

(a) contacting a double-stranded DNA molecule with an endonuclease and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0 , to the first end of the linear double stranded region, and the second terminal adaptor molecule is appended to the mth intermediate adaptor molecule, or when m is 0, to the second end of the linear double stranded region.

2. The method of clause 1, wherein the appending is performed by ligation and/or hybridization.

3. A method for producing a closed linear deoxyribonucleic acid (DNA) product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

4. A method for producing a closed linear deoxyribonucleic acid (DNA) product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and

(b) incubating the single contiguous aqueous volume to generate the closed linear DNA product, wherein the closed linear DNA product comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

5. The method of clause 3 or clause 4, wherein the linear portion of the double-stranded DNA molecule is closed at the first end and the second end by ligation of the first and second terminal adaptor molecules.

6. The method of any one of clauses 3-5, wherein the first closed end and the second closed end are resistant to nuclease digestion.

7. The method of clause 6, wherein the nuclease digestion is exonuclease digestion, optionally exonuclease III digestion and/or exonuclease I digestion.

8. The method of any one of clauses 3-7, wherein the closed linear DNA product is a covalently closed linear DNA product.

9. The method of any one of clauses 3-8, wherein the closed linear DNA product is partially double-stranded and/or partially single-stranded.

10. The method of any one of clauses 3-9, wherein the closed linear DNA product comprises at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides in length.

11. The method of any one of clauses 1-10, wherein the double-stranded DNA molecule is circular or branched.

12. The method of any one of clauses 1-11, wherein the double-stranded DNA molecule comprises a cassette, optionally wherein the cassette comprises a coding sequence.

13. The method of any one of clauses 1-12, wherein the double-stranded DNA molecule comprises a spacer, optionally wherein the spacer is at least 10, at least 20, at least 30, at least 40, at least 50, at least 60, at least 70, at least 80, at least 90, at least 100, at least 125, at least 150, at least 175, or at least 200 base pairs long.

14. The method of any one of clauses 3-13, wherein the closed DNA product comprises a homopolymeric sequence, such as a polyA, poly C, polyT or polyG sequence.

15. The method of any one of clauses 3-14, wherein the closed DNA product comprises an inverted terminal repeat sequence.

16. The method of any one of clauses 1-15, wherein the double-stranded DNA molecule comprises one or more endonuclease target sequences, optionally wherein the one or more endonuclease target sequences are Type IIS endonuclease target sequences, such as BbsI, BsaI, BsmBI, BspQI, BtgZI, Esp3I, and/or SapI, AarI, Acc36I, AclWI, AcuI, AjuI, AloI, Alw26I, AlwI, ArsI, AsuHPI, BaeI, BarI, BbvI, BccI, BceAI, Bcgl, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6I, BstF5I, BstMAI, BstV11, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp11091, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequences.

17. The method of any one of clauses 1-16, wherein the double-stranded DNA molecule is a product of amplification, optionally rolling circle amplification.

18. The method of any one of clauses 1-17, wherein the endonuclease is a restriction enzyme endonuclease, optionally wherein the endonuclease is Type IIS restriction enzyme endonuclease, such as BbsI, BsaI, BsmBI, BspQI, BtgZI, Esp3I,SapI, AarI, Acc36I, AclWI, AcuI, AjuI, AloI, Alw26I, AlwI, ArsI, AsuHPI, BaeI, BarI, BbvI, BccI, BceAI, Bcgl, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6I, BstF51, BstMAI, BstV11, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp11091, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI restriction enzyme endonuclease.

19. The method of any one of clauses 1-18, wherein the ligase is a DNA ligase, optionally wherein the DNA ligase is a T4 DNA ligase, T7 DNA ligase, mammalian DNA ligase I, III and IV; Taq DNA ligase, Tth DNA ligase, or *E. coli* DNA ligase.

20. The method of any one of clauses 1-19, wherein the first terminal adaptor molecule and/or the second terminal adaptor molecule and/or one or more of the n+m intermediate adaptor molecules is a synthetic adaptor molecule.

21. The method of any one of clauses 1-20, wherein the first terminal adaptor molecule comprises a hairpin and/or the second terminal adaptor molecule comprises a hairpin.

22. The method of any one of clauses 1-21, wherein the first terminal adaptor molecule is a nucleic acid adaptor molecule.

23. The method of any one of clauses 1-22, wherein the second terminal adaptor molecule is a nucleic acid adaptor molecule.

24. The method of any one of clauses 1-23, wherein the first terminal adaptor molecule and/or the second terminal adaptor molecule comprise a single-stranded portion, optionally wherein:

(a) the single-stranded portion forms a hairpin;
(b) the single-stranded portion comprises less than 10, 9, 8, 7, 6, 5, 4, 3, 2 nucleotides; and/or
(c) the single-stranded portion comprises 5 nucleotides.

25. The method of any one of clauses 1-24, wherein the first terminal adaptor molecule and/or the second terminal adaptor molecule and/or one or more of the n+m intermediate adaptor molecules comprise a double-stranded portion, optionally wherein:

(a) the double-stranded portion comprises less than 50, 45, 40, 35, 30 base pairs; and/or
(b) the double-stranded portion comprises at least 10, 11, 12, 13, 14, 15 base pairs.

26. The method of any one of clauses 1-25, wherein the first terminal adaptor molecule and/or the second terminal adaptor molecule and/or one or more of the n+m intermediate adaptor molecules comprise a 5' phosphate.

27. The method of any one of clauses 1-26, wherein the first terminal adaptor molecule and/or the second terminal adaptor molecule comprise a sequence of SEQ ID NO: 1.

28. The method of any one of clauses 1-26, wherein the first terminal adaptor molecule and/or the second terminal adaptor molecule comprise at least 13, at least 14, at least 15, at least 16, at least 17, at least 18, or at least 19 contiguous nucleotides of SEQ ID NO: 1.

29. The method of clause 25, wherein the double-stranded portion of the first terminal adaptor molecule and/or the second terminal adaptor molecule comprises a sequence of SEQ ID NO: 2.

30. The method of clause 25, wherein the double-stranded portion of the first terminal adaptor molecule and/or the second terminal adaptor molecule comprise at least 8, at least 9, at least 10, at least 11, at least 12, at least 13, at least 14 or at least 15 contiguous nucleotides of SEQ ID NO: 2.

31. The method of clause 24, wherein the single-stranded portion of the first terminal adaptor molecule and/or the second terminal adaptor molecule comprise a sequence of ACTCA.

32. The method of clause 24, wherein the single-stranded portion of the first terminal adaptor molecule and/or the second terminal adaptor molecule may comprise at least 1, at least 2, at least 3, at least 4 or at least 5 contiguous nucleotides of ACTCA.

33. The method of any one of clauses 1-32, wherein the first and second terminal adaptor molecules are different.

34. The method of any one of clauses 1-33, wherein the first terminal adaptor molecule comprises a portion that is complementary to the first end of the linear double-stranded region or to a first end of the nth intermediate adaptor molecule.

35. The method of any one of clauses 1-34, wherein the second terminal adaptor molecule comprises a portion that is complementary to the second end of the linear double-stranded region or to a first end the mth intermediate adaptor molecule.

36. The method of any one of clauses 1-35, wherein the first terminal adaptor molecule comprises a portion that anneals to the first end of the linear double-stranded region or to a first end of the nth intermediate adaptor molecule.

37. The method of any one of clauses 1-37, wherein the second terminal adaptor molecule comprises a portion that anneals to the second end of the linear double-stranded region or to a first end of the mth intermediate adaptor molecule.

38. The method of any one of clauses 1-37, wherein the first terminal adaptor molecule and/or the second terminal adaptor molecule and/or one or more of the n+m intermediate adaptor molecules comprise an overhang.

39. The method of any one of clauses 1-38, wherein the first terminal adaptor molecule and/or the second terminal adaptor molecule and/or one or more of the n+m intermediate adaptor molecules comprise a functional portion, optionally wherein the functional portion is a binding molecule, a targeting sequence, or a probe.

40. The method of any one of clauses 1-39, wherein the first terminal adaptor molecule and/or the second terminal adaptor molecule and/or one or more of the n+m intermediate adaptor molecules comprise a nuclear localization sequence.

41. The method of any one of clauses 1-40, wherein the first terminal adaptor molecule and/or the second terminal adaptor molecule and/or one or more of the n+m intermediate adaptor molecules comprise a barcode.

42. The method of any one of clauses 1-41, wherein the first terminal adaptor molecule and/or the second terminal adaptor molecule and/or one or more of the n+m intermediate adaptor molecules comprise a fluorophore.

43. The method of any one of clauses 1-42, wherein the first terminal adaptor molecule and/or the second terminal adaptor molecule and/or one or more of the n+m intermediate adaptor molecules comprise a radioactive compound.

44. The method of any one of clauses 1-43, wherein the first terminal adaptor molecule and/or the second terminal adaptor molecule and/or one or more of the n+m intermediate adaptor molecules comprise a portion that facilitates sequencing, detection or quantification.

45. The method of any one of clauses 1-44, wherein the first terminal adaptor molecule and/or the second terminal adaptor molecule and/or one or more of the n+m intermediate adaptor molecules comprise an inverted terminal repeat sequence.

46. The method of any one of clauses 1-45, wherein the first terminal adaptor molecule and/or the second terminal adaptor molecule and/or one or more of the n+m intermediate adaptor molecules comprise an aptamer.

47. The method of any one of clauses 1-46, wherein the first terminal adaptor molecule and/or the second terminal adaptor molecule confer resistance to the nuclease digestion, optionally exonuclease digestion (e.g. exonuclease I and/or exonuclease III digestion).

48. The method of any one of clauses 1-47, wherein the first and second terminal adaptor molecules are ligated to the linear double-stranded region or the nth and/or mth intermediate adaptor molecule.

49. The method of clause 48, wherein ligation is at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient.

50. The method of any one of clauses 1-49, wherein the step of incubating the single contiguous aqueous volume comprises incubating at a first temperature and then incubating at a second temperature; optionally wherein:

(a) the first temperature is 1°C-100°C, 4°C-70°C, 10°C-60°C, 16°C-55°C, 20°C-50°C, 25°C-45°C, 30°C-40°C, or 35°C-39°C; and/or
(b) the second temperature is 1°C-100°C, 4°C-70°C, 8°C-60°C, 10°C-55°C, 23°C-50°C, 14°C-40°C, 14°C-30°C, or 15°C-18°C.

51. The method of clause 50, wherein the first temperature is 35°C-39°C and the second temperature is 15°C-18°C.

52. The method of clause 50 or 51, wherein the first temperature is 37°C and the second temperature is 16°C.

53. The method of any one of clauses 1-52, wherein the step of incubating the single contiguous aqueous volume comprises cycling between the first temperature and the second temperature, optionally wherein the step of incubating the single contiguous aqueous volume comprises cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times.

54. The method of any one of clauses 1-52, wherein the step of incubating the single contiguous aqueous volume comprises incubating at a constant temperature.

55. The method of claims 54, wherein the constant temperature is about 30°C or about 37°C.

56. The method of any one of clauses 1-55, wherein the method further comprises, before step (a) (i.e. the step of contacting the double-stranded DNA molecule with the endonuclease, the ligase and the first and second terminal adaptor molecules and/or n+m intermediate adaptor molecules), a step of amplification of a DNA template molecule to produce the double-stranded DNA molecule, optionally wherein amplification is rolling circle amplification.

57. The method of any one of clauses 1-56, wherein the method further comprises, after step (b) (i.e. the step of incubating the single contiguous aqueous volume), a step of purification of the linear DNA product or closed linear DNA product.

58. The method of any one of clauses 1-57, wherein the method further comprises, after step (b) (i.e. the step of incubating the single contiguous aqueous volume), a step of nuclease digestion, optionally wherein the nuclease digestion is exonuclease digestion, such as exonuclease I and/or exonuclease III digestion.

59. The method of clause 58, wherein the step of nuclease digestion takes places before or after the step of purification

60. A method for producing a linear deoxyribonucleic acid (DNA) product, wherein the method comprises:

(a) contacting a double-stranded DNA molecule with an endonuclease and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides.

61. The method of clause 60, wherein step (a) comprises contacting the double-stranded DNA molecule with an endonuclease, a ligase and first and second terminal adaptor molecules and n+m intermediate adaptor molecules, to form a single contiguous aqueous volume.

62. The method of clauses 60 or 61, wherein the first terminal adaptor molecule is ligated to a first end of the linear double-stranded region or to a first end of the nth intermediate adaptor molecule and the second terminal adaptor molecule is ligated to a second end of the linear double-stranded region or to a first end of the mth intermediate adaptor molecule.

63. The method of any one of clauses 60-62, wherein the linear DNA product is resistant to nuclease digestion, optionally wherein the nuclease digestion is exonuclease digestion, such as exonuclease III digestion and/or exonuclease I digestion.

64. The method of any one of clauses 60-63, wherein the nuclease-resistant nucleotides are phosphorothioated nucleotides.

65. A method for producing a closed linear deoxyribonucleic acid (DNA) product, the method comprises:

(a) rolling circle amplification of a DNA template molecule comprising at least one endonuclease target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

66. A method for producing a partially closed deoxyribonucleic acid (DNA) product, wherein the method comprises:

(a) contacting a double-stranded DNA molecule with an endonuclease and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides.

67. The method of clause 66, wherein step (a) comprises contacting the double-stranded DNA molecule with an endonuclease, a ligase and first and second terminal adaptor molecules and n+m intermediate adaptor molecules, to form a single contiguous aqueous volume.

68. The method of clauses 66 or 67, wherein the first terminal adaptor molecule is ligated to a first end of the linear double-stranded region or a first end of the nth intermediate adaptor molecule and the second terminal adaptor molecule is ligated to a second end of the linear double-stranded region or a first end of the mth intermediate adaptor molecule.

69. The method of any one of clauses 66-68, wherein the linear DNA product is resistant to nuclease digestion, optionally wherein the nuclease digestion is exonuclease digestion, such as exonuclease III digestion and/or exonuclease I digestion.

70. The method of any one of clauses 66-69, wherein the nuclease-resistant nucleotides are phosphorothioated nucleotides.

71. A method for producing a partially closed deoxyribonucleic acid (DNA) product, the method comprises:

(a) rolling circle amplification of a DNA template molecule comprising at least one endonuclease target sequence to generate a double-stranded DNA molecule;
(b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adaptor molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides.

71. A method for in vitro transcription of a closed linear deoxyribonucleic acid (DNA) product, the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, , and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region;
(c) contacting the closed linear DNA product with a polymerase; and

(d) producing a transcription product.

72. A method for protein expression, the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate a closed linear DNA product, wherein the closed linear DNA product comprises a linear double-stranded region, wherein the linear double-stranded region comprises a linear portion of the double-stranded DNA molecule, , and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region; and
(c) introducing the closed linear DNA product into a prokaryotic cell or a eukaryotic cell or a cell-free protein expression system to generate a desired RNA or protein.

73. A method for cell transfection of a closed linear deoxyribonucleic acid (DNA) product into a cell, the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1;
(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, , and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region;
(c) contacting the closed linear DNA product with the cell; and
(d) transfecting the closed linear DNA product into the cytosol of the cell.

73. A method for producing a pharmaceutical composition comprising the closed linear DNA product, the method comprising performing the method of any of one of clauses 3-59 and formulating the resulting closed linear DNA product with a pharmaceutically acceptable carrier or excipient.

74. A method for producing a pharmaceutical composition comprising the linear DNA product, the method comprising performing the method of any of one of clauses 60-64 and formulating the resulting linear DNA product with a pharmaceutically acceptable carrier or excipient.

75. A method for producing a pharmaceutical composition comprising the partially closed linear DNA product, the method comprising performing the method of any of one of clauses 66-71 and formulating the resulting linear DNA product with a pharmaceutically acceptable carrier or excipient.

76. Use of a closed linear DNA product in the manufacture of a medicament for treatment of a human or animal body by therapy, wherein the manufacture comprises performing the method of any one of clauses 3-59.

77. Use of a linear DNA product in the manufacture of a medicament for treatment of a human or animal body by therapy, wherein the manufacture comprises performing the method of any one of clauses 60-64.

78. Use of a partially closed linear DNA product in the manufacture of a medicament for treatment of a human or animal body by therapy, wherein the manufacture comprises performing the method of any one of clauses 66-71.

79. Use of a closed linear DNA product in the production of viral or non-viral delivery system, wherein the closed linear DNA product is produced by performing the method of any one of clauses 3-59.

80. Use of a linear DNA product in the production of viral or non-viral delivery system, wherein the linear DNA product is produced by performing the method of any one of clauses 60-64.

81. Use of a partially closed linear DNA product in the production of viral or non-viral delivery system, wherein the partially closed linear DNA product is produced by performing the method of any one of clauses 66-71 .

82. A closed linear DNA product obtainable by the method of any one of clauses 3-59.

83. A linear DNA product obtainable by the method of any one of clauses 60-64.

84. A partially closed linear DNA product obtainable by the method of any one of clauses 66-71.

85. A closed linear DNA product obtainable by the method of any one of clauses 3-59 for use in therapy.

86. A linear DNA product obtainable by the method of any one of clauses 60-64 for use in therapy.

87. A partially closed linear DNA product obtainable by the method of any one of clauses 66-71 for use in therapy.

88. A kit comprising:

(a) first and second terminal adaptor molecules;
(b) n+m intermediate adaptor molecules, wherein n and m are each 0 or an integer of at least 1, and n+m is at least 1
(c) an endonuclease; and
(d) a ligase.

89. A kit comprising:

(a) a first terminal adaptor molecule;
(b) a second terminal adaptor molecule;
(c) n intermediate adaptor molecules, where n is 0 or an integer of at least 1;
(d) m intermediate adaptor molecules, where m is 0 or an integer of at least 1 and where n+m is at least 1;
(e) an endonuclease; and
(d) a ligase.

90. A method for producing a closed linear deoxyribonucleic acid (DNA) product, wherein the method comprises:

(a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, , and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

91. The method of claim 90, wherein the closed linear DNA product comprises a spacer, optionally wherein the spacer is at least 20 base pairs long.

92. The method of claim 90 or 91, wherein the endonuclease is a Type IIS restriction endonuclease, optionally wherein the endonuclease is Bbsl, Bsal, BsmBI, BspQI, BtgZl, Esp3!,Sap!, Aarl, Acc361, AclWI, Acul, Ajul, Alol, Alw261, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXl, Bse1I, Bse3DI, BseGI, BseMI, BseMII, BseNI, BseRI, BseXl, Bsgl, BslFI, BsmAl, BsmFI, Bsml, Bso31I, BspCNI, BspMI, BspPI, BspQI, BspTNI, BsrDI, Bsrl, Bst6!, BstF5I, BstMAI, BstV1I, BstV2!, Bsul, BtgZI, BtsCI, Btsl-v2, BtsMutl, Bve!, Csel, CspCI, Eam1104!, Earl, Ecil, Eco31I, Eco57!, Esp3I, Faql, Faul, Fokl, Gsul, Hgal, Hphl, HpyAV, Lgul, Lmnl, Lsp1109I, Lwel, Mboll, Mlyl, Mmel, Mnll, Mva12691, NmeAlII, PaqCI, PciSI, Pctl, Plel, Ppsl, Psrl, Schl, SfaNI, TaqlI, TspDTI and/or TspGWI restriction endonuclease.

93. The method of any one of claims 90-92, wherein the first terminal adaptor molecule and/or the second terminal adaptor molecule and/or one or more of n+m intermediate adaptor molecules are nucleic acid adaptor molecules.

94. The method of any one of claims 90-93, wherein the first terminal adaptor molecule comprises a hairpin and/or the second terminal adaptor molecule comprise a hairpin.

95. The method of any one of claims 90-94, wherein the first terminal adaptor molecule and/or the second terminal adaptor molecule and/or one or more of n+m intermediate adaptor molecules comprises a double-stranded region with an overhang.

96. The method of any one of claims 90-95, wherein the method comprises the steps:

   (a) amplifying a DNA template molecule comprising at least one endonuclease target sequence to generate the double-stranded DNA molecule, wherein the DNA template molecule is amplified by rolling circle amplification;
   (b) contacting the double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
   (c) incubating the single contiguous aqueous volume to generate the closed linear DNA product, wherein the closed linear DNA product comprises a linear double-stranded region, wherein the linear double-stranded region comprises a linear portion of the double-stranded DNA molecule, , and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

97. A method for producing a linear deoxyribonucleic acid (DNA) product, wherein the method comprises:

   (a) contacting a double-stranded DNA molecule with an endonuclease, a ligase and first and second terminal adaptor molecules and n+m intermediate adaptor molecules, to form a single contiguous aqueous volume, wherein n and m are each 0 or an integer of at least 1, and wherein n+m is at least 1; and
   (b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides.

98. The method of claim 97, wherein the one or more nuclease-resistant nucleotides are one or more phosphorothioated nucleotides.

99. A method for in vitro transcription of a closed linear deoxyribonucleic acid (DNA) product, or a linear DNA product, wherein the method comprises:

   (a) producing a closed linear DNA product according to the method of any one of claims 90-96, or producing a linear DNA product according to the method of claim 97 or claim 98;

(b) contacting the closed linear DNA product, or the linear DNA product, with a polymerase; and

(c) producing a transcription product encoded by the closed linear DNA product, or the linear DNA product.

100. A method for producing a protein, wherein the method comprises:

(a) producing a closed linear DNA product according to the method of any one of claims 90-96, or producing a linear DNA product according to the method of claim 97 or claim 98;

(b) introducing the closed linear DNA product, or the linear DNA product, into a cell or a cell-free expression system to generate a protein encoded by the closed linear DNA product or the linear DNA product.

101. A method for cell transfection of a closed linear deoxyribonucleic acid (DNA) product, or a linear DNA product, into a cell, wherein the method comprises:

(a) producing a closed linear DNA product according to the method of any one of claims 90-96, or producing a linear DNA product according to the method of claim 97 or claim 98;

(b) contacting a cell with the closed linear DNA product or the linear DNA product; and

(c) transfecting the closed linear DNA product, or the linear DNA product, into the cytosol of the cell.

102. The method of claim 101, wherein the transfection of the closed linear DNA product, or the linear DNA product, into the cytosol of the cell is performed by electroporation.

103. Use of a closed linear DNA product, or a linear DNA product, in the production of viral or non-viral delivery system, wherein the closed linear DNA product is produced by performing the method of any one of claims 90-96 and the linear DNA product is produced by the method of claim 97 or claim 98.

104. The method of any one of clauses 1-102 wherein n=0 and m=1

105. The method of any one of clauses 1-102 wherein n=0 and m=2

106. The method of any one of clauses 1-102 wherein n=0 and m=3

107. The method of any one of clauses 1-102 wherein n=0 and m=4

108. The method of any one of clauses 1-102 wherein n=1 and m=1

109. The method of any one of clauses 1-102 wherein n=1 and m=2

110. The method of any one of clauses 1-102 wherein n=1 and m=3

111. The method of any one of clauses 1-102 wherein n=1 and m=4

112. The method of any one of clauses 1-102 wherein n=2 and m=2

113. The method of any one of clauses 1-102 wherein n=2 and m=3

114. The method of any one of clauses 1-102 wherein n=2 and m=4

115. The method of any one of clauses 1-102 wherein n=3 and m=3

116. The method of any one of clauses 1-102 wherein n=3 and m=4

117. The method of any one of clauses 1-102 wherein n=4 and m=4

118. the method of anyone of clauses 1-102, wherein each adaptor molecule has a sequence at its first end that is complementary to a sequence at a second end of the adjacent adaptor molecule. 119. The method of an one of clauses 1 -102 and 118, wherein each adaptor molecule has a different sequence at its first end, and each adaptor molecule has a different sequence at is second end.

120. The method of any one of clauses 1-102 and 118-119, wherein the sequences at the first and second ends of each adaptor sequence are different.

## EXAMPLES

### Example 1: Rolling circle amplification of Cre-derived circular DNA

Cre recombinase from the P1 bacteriophage is a Type I topoisomerase. The enzyme catalyzes the site-specific recombination of DNA between loxP sites (SEQ ID NO: 11). LoxP recognition site (34 bp) consists of two 13 bp inverted repeats which flank an 8 bp spacer region, which confers directionality. The products of Cre-mediated recombination are dependent upon the location and relative orientation of the loxP sites. Two DNA species containing single loxP sites were fused. DNA found between two loxP sites oriented in the same direction was excised as a circular loop of DNA, while DNA between opposing loxP sites was inverted with respect to external sequences. Cre recombinase requires no additional cofactors or accessory proteins for its function.

Cre reaction conditions: reaction volume 50 $\mu$l, DNA of interest purified from agarose gel electrophoresis after restriction enzyme digestion (100 ng), Cre recombinase (NEB, 4 units), incubation time and temperature: 30 min at 37°C and 20 min at 80°C. Next, to remove remaining non-circular DNA molecules before the amplification step, E. coli exonuclease I (NEB, 20 units) and III (NEB, 100 units) were added and the reaction was incubated 30 min at 37°C and 20 min at 80°C.

Rolling circle amplification (RCA) is a faithful and isothermal DNA amplification method based on Phi29 DNA polymerase (Phi29DNApol). Phi29DNApol is the monomeric enzyme responsible for the replication of the linear double stranded DNA of bacteriophage phi29 from *Bacillus subtilis* (Blanco and Salas, 1984). It is an extremely processive polymerase (up to more than 70 kb per binding event) with a strong strand displacement capacity (Blanco *et al,* 1989). The enzyme displays 3'->5' proofreading exonuclease activity (Garmendia *et al,* 1992), resulting in an extremely high fidelity of synthesis (Esteban *et al,* 1993). These special features make this enzyme the perfect choice for isothermal DNA amplification.

RCA can be initiated by random synthetic primers (Dean *et al,* 2001) or a DNA primase like *Tth*PrimPol (Picher *et al,* 2016) that synthesizes the primers for Phi29DNApol during the amplification reaction.

Before the amplification, circularized DNA samples were first denatured by adding 1 volume of buffer D (400 mM KOH, 10 mM EDTA) and incubating 3 min at room temperature. Samples were then neutralized by adding 1 volume of buffer N (400 mM HCl, 600 mM Tris-HCl pH 7.5). Rolling circle amplification conditions: 10 ml reaction volume, 1 ml TruePrime WGA reaction buffer 10x (4basebio), 500 $\mu$l denatured DNA sample, 1 ml *Tth*PrimPol (1 $\mu$M), 160 $\mu$l QualiPhi Phi29DNApol (12,5 $\mu$M), 2.5 units PPase (Thermo) and 1 ml dNTPs (10 mM). Incubation time and temperature: 20 hours at 30°C and 10 min at 65°C.

### Example 2: ligation of adaptors to produce a nuclease-resistant DNA product

Amplified DNAs were incubated with Type II restriction enzyme Bsal, T4 DNA ligase and complementary intermediate double-stranded adaptors to the 5' protruding ends generated by Bsal on the amplified DNA, and terminal adaptors complementary to the opposite ends of the intermediate adaptor molecules. A control reaction was carried out with terminal adaptor only (SEQ ID NO:1). The intermediate double-stranded adaptors with a 5' single-stranded overhangs on each end of the molecule and phosphorylation modification on each 5' end (SEQ ID NO:s 6+7); and a self-complementary terminal adaptor with a single-stranded 5' overhang complementary to the intermediate adaptor on one end and a hairpin structure at the other end (SEQ ID NO:8). A control without the intermediate adaptors and a control without the terminal adaptors were also performed under the same conditions. The adaptors are supplied at molar excess, ranging from 1:2 to 1:20 molar ratio, in comparison to the molarity of RCA tandem repeats.

Concentration of DNA generated by RCA is measured (Qubit) in its concatemer form. The expected molarity of unique, tandem repeats is determined based on the length of a single copy of DNA sequence and the DNA concentration.

The simultaneous digestion and ligation reaction was carried out in the following conditions in a single reaction volume:

a) digestion/ligation reaction buffer (50 mM Tris-HCl pH 7.5, 10 mM $MgCl_2$, 10 mM DTT, 10mM ATP),
b) RCA material (240 ng/uL),
c) upstream intermediate adaptors (5x molar excess), upstream terminal adaptors (5 or 20x molar excess), downstream intermediate adaptors (5x molar excess), downstream terminal adaptors (5 or 20x molar excess),
d) T4 Ligase 4Basebio (33.2 ng/uL),
e) and Bsal or other endonuclease with a restriction site represented in the RCA concatemer (3.2 ng/uL 4Basebio

BsaI).

The reaction is incubated at 37°C for 20 hours and treated with exonucleases I and III in order to remove any non-ligated DNA material and further purified to remove enzymes and exchange salts.

Figure 2 shows the products of the reactions. In the left hand gel, lanes 1 and 2 show the control with terminal hairpin adaptors (SEQ ID NO: 1) complementary to the 5' overhang of the digested amplified DNA. The reaction produces a closed end (nuclease-resistant) linear DNA product. Lanes 3 and 4 and lanes 5 and 6 show the reaction with the intermediate adaptor molecules (SEQ ID 6+7) and the terminal adaptor molecule (SEQ ID NO:8) pre and post exonuclease treatment. Lanes 3 and 4 show the results of the reaction with the intermediate adaptor at 5x molar excess and the terminal adaptor at 20x molar excess. This result is also shown in the right hand gel in lanes 5 and 6. Lanes 5 and 6 of the left hand gel show the results of the reaction with the intermediate adaptor at 5x molar excess and the terminal adaptor at 5x molar excess. The presence of the target band after exonuclease treatment (lanes 4 and 6 of the left hand gel, and lane 6 of the right hand gel) indicates the correct ligation of the intermediate and terminal adaptor molecules. Lanes 7 and 8 show that without having the 5' phosphate, ligation of the intermediate and/or terminal adaptors does not occur, indicated by the digestion of the product with exonuclease.

In the right hand gel, lanes 1 and 2 show the product of the reaction carried out with the terminal adaptor molecules only (SEQ ID NO:8) and no intermediate adaptor. A single band is seen pre exonuclease treatment (the digested DNA with no adaptors ligated), which is digested and therefore no longer visible post exonuclease treatment (due to lack of an intermediate adaptor). Lanes 3 and 4 show the product of the reaction carried out with the intermediate adaptor molecules only (SEQ ID NO:6+7) and no terminal adaptor. The digested DNA band is no longer visible after exonuclease treatment, (due to lack of a terminal adaptor).

## Claims

1. A method for producing a closed linear deoxyribonucleic acid (DNA) product, wherein the method comprises:

    (a) contacting a double-stranded DNA molecule with an endonuclease, a first terminal adaptor molecule, a second terminal adaptor molecule and n + m intermediate adaptor molecules, to form a single contiguous aqueous volume, wherein n and m are each 0 or an integer of at least 1, and wherein n + m is at least 1; and
    (b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the closed linear DNA product is closed at a first end by the first terminal adaptor molecule appended to the nth intermediate adaptor molecule or, when n is 0 , to the first end of the linear double stranded region, and closed at a second end by the second terminal adaptor molecule appended to the mth intermediate adaptor molecule, or when m is 0, to the second end of the linear double stranded region.

2. The method of claim 1, wherein the first and/or second terminal adaptor molecules comprise a hairpin.

3. A method for producing a linear deoxyribonucleic acid (DNA) product, wherein the method comprises:

    (a) contacting a double-stranded DNA molecule with an endonuclease, a first terminal adaptor molecule, a second terminal adaptor molecule and n + m intermediate adaptor molecules, to form a single contiguous aqueous volume, wherein n and m are each 0 or an integer of at least 1, and wherein n + m is at least 1; and
    (b) incubating the single contiguous aqueous volume to generate the linear DNA product, wherein the linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the first terminal adaptor molecule is appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the first terminal adaptor molecule and second terminal adaptor molecules are nucleic acid molecules that comprise one or more nuclease-resistant nucleotides.

4. A method for producing a partially closed deoxyribonucleic acid (DNA) product, wherein the method comprises:

(a) contacting a double-stranded DNA molecule with an endonuclease, a first terminal adapter molecule, a second terminal adaptor molecule and n + m intermediate adaptor molecules, to form a single contiguous aqueous volume, wherein n and m are each 0 or an integer of at least 1, and wherein n + m is at least 1; and
(b) incubating the single contiguous aqueous volume to generate the partially closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the partially closed DNA product is closed at a first end by the first terminal adapter molecule appended to the nth intermediate adaptor molecule or, when n is 0, to the first end of the linear double stranded region, and the second terminal adaptor molecule is appended to the mth intermediate adaptor molecule, or when m is 0, to the second end of the linear double stranded region, and wherein the second terminal adaptor molecule is a nucleic acid molecules that comprise one or more nuclease-resistant nucleotides.

5. The method of claim 4, wherein the first terminal adaptor molecule comprises a hairpin

6. The method of any one of claims 1-5, wherein the endonuclease is a Type IIS restriction endonuclease, optionally wherein the endonuclease is BbsI, BsaI, BsmBI, BspQI, BtgZI, Esp3I, SapI, AarI, Acc36I, AclWI, AcuI, AjuI, AloI, Alw26I, AlwI, ArsI, AsuHPI, BaeI, BarI, BbvI, BccI, BceAI, Bcgl, BciVI, BcoDI, BfuAI, BfuI, BmrI, BmsI, BmuI, BpiI, BpmI, BpuEI, BsaXI, Bse11, Bse3DI, BseGI, BseMI, BseMII, BseNI, BseRI, BseXI, BsgI, BsIFI, BsmAI, BsmFI, BsmI, Bso31I, BspCNI, BspMI, BspPI, BspQI, BspTNI, BsrDI, BsrI, Bst6I, BstF5I, BstMAI, BstV11, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp11091, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI restriction endonuclease.

7. The method of any one of claims 1-6, wherein the first terminal adaptor molecule and/or the second terminal adaptor molecule and/or one or more intermediate adaptor molecules are nucleic acid adaptor molecules

8. The method of any one of claims 1-7 wherein step a) further comprises contacting the double stranded DNA molecule with a ligase.

9. The method of any one of claims 1-8, wherein the first end-adaptor molecule and/or the second end-adaptor molecule and/or one or more intermediate adaptor molecules comprises a double-stranded region with an overhang.

10. The method of anyone of claims 3-9, wherein the one or more nuclease-resistant nucleotides are one or more phosphorothioated nucleotides.

11. The method of any one of claims 1-10, wherein one or more of the n+m intermediate adaptor molecules are 10-500, 50-400, 100-300 or 200-250 base pairs in length.

12. The method of any one of claims 1-11, wherein one or more of the n+m intermediate adaptor molecules comprises a fluorophore, a barcode, a polyA signal, a biotinylated nucleotide, protected nucleotides, spacers, polyA sequence, a promotor, an open reading frame, a targeting sequence or a localisation signal.

13. The method of any one of claims 1 to 12, wherein n is 0, 1, 2 or 3.

14. The method of any one of claims 1 to 13, wherein m is 0, 1, 2 or 3.

Fig. 1

EP 4 410 995 A1

Fig. 2

Europäisches
Patentamt

European
Patent Office

Office européen
des brevets

## EUROPEAN SEARCH REPORT

**Application Number**

EP 23 38 2089

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2021/152147 A1 (TYRIS THERAPEUTICS S L [ES]) 5 August 2021 (2021-08-05) * figure 1 * | 1-14 | INV. C12Q1/6855 |
| A | WO 2005/047547 A1 (APPLERA CORP [US]; CHEN CAIFU [US] ET AL.) 26 May 2005 (2005-05-26) * figures 1, 7 * | 1-14 | |
| A | EP 2 048 248 A1 (SAMSUNG ELECTRONICS CO LTD [KR]) 15 April 2009 (2009-04-15) * figures 1,2 * | 1-14 | |
| A | WO 2018/108328 A1 (HOFFMANN LA ROCHE [CH]; ROCHE DIAGNOSTICS GMBH [DE] ET AL.) 21 June 2018 (2018-06-21) * figure 1 * | 1-14 | |
| A | WO 2013/036685 A1 (GEN PROBE INC [US]; NELSON NORMAN C [US] ET AL.) 14 March 2013 (2013-03-14) * figure 1 * | 1-14 | TECHNICAL FIELDS SEARCHED (IPC) C12Q |
| A | WO 2005/118877 A2 (VICUS BIOSCIENCE LLC [US]; KAUFMAN JOSEPH C [US]) 15 December 2005 (2005-12-15) * figure 1 * | 1-14 | |

The present search report has been drawn up for all claims

| Place of search | Date of completion of the search | Examiner |
|---|---|---|
| The Hague | 23 June 2023 | Aslund, Fredrik |

EPO FORM 1503 03.82 (P04C01)

## ANNEX TO THE EUROPEAN SEARCH REPORT
## ON EUROPEAN PATENT APPLICATION NO.

EP 23 38 2089

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2023

| Patent document cited in search report | | Publication date | Patent family member(s) | | Publication date |
|---|---|---|---|---|---|
| WO 2021152147 | A1 | 05-08-2021 | AU | 2021213927 A1 | 28-07-2022 |
| | | | CA | 3164390 A1 | 05-08-2021 |
| | | | CN | 115003829 A | 02-09-2022 |
| | | | EP | 4097253 A1 | 07-12-2022 |
| | | | JP | 2023511992 A | 23-03-2023 |
| | | | KR | 20220133999 A | 05-10-2022 |
| | | | WO | 2021152147 A1 | 05-08-2021 |
| WO 2005047547 | A1 | 26-05-2005 | AT | 462801 T | 15-04-2010 |
| | | | EP | 1680517 A1 | 19-07-2006 |
| | | | EP | 2204458 A1 | 07-07-2010 |
| | | | US | 2006063163 A1 | 23-03-2006 |
| | | | WO | 2005047547 A1 | 26-05-2005 |
| EP 2048248 | A1 | 15-04-2009 | EP | 2048248 A1 | 15-04-2009 |
| | | | KR | 20090037118 A | 15-04-2009 |
| | | | US | 2009098612 A1 | 16-04-2009 |
| | | | US | 2011269193 A1 | 03-11-2011 |
| WO 2018108328 | A1 | 21-06-2018 | CN | 110036117 A | 19-07-2019 |
| | | | EP | 3555305 A1 | 23-10-2019 |
| | | | ES | 2866896 T3 | 20-10-2021 |
| | | | JP | 6925424 B2 | 25-08-2021 |
| | | | JP | 2020501554 A | 23-01-2020 |
| | | | US | 2020010875 A1 | 09-01-2020 |
| | | | US | 2021363570 A1 | 25-11-2021 |
| | | | WO | 2018108328 A1 | 21-06-2018 |
| WO 2013036685 | A1 | 14-03-2013 | AU | 2012304537 B2 | 20-08-2015 |
| | | | AU | 2015246165 A1 | 12-11-2015 |
| | | | AU | 2017248555 A1 | 09-11-2017 |
| | | | EP | 2753712 A1 | 16-07-2014 |
| | | | EP | 3225698 A1 | 04-10-2017 |
| | | | EP | 3620533 A1 | 11-03-2020 |
| | | | US | 2014329282 A1 | 06-11-2014 |
| | | | WO | 2013036685 A1 | 14-03-2013 |
| WO 2005118877 | A2 | 15-12-2005 | US | 2006057608 A1 | 16-03-2006 |
| | | | US | 2010055745 A1 | 04-03-2010 |
| | | | WO | 2005118877 A2 | 15-12-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010086626 A1

- WO 2008095927 A

**Non-patent literature cited in the description**

- **STEIN ; CASTANOTTO.** FDA-approved oligonucleotide therapies in 2017. *Molecular Therapy,* 2017, vol. 25 (5), 1069-1075

- **PUTNEY et al.** A DNA fragment with an alpha-phosphorothioate nucleotide at one end is asymmetrically blocked from digestion by exonuclease III and can be replicated in vivo. *Proceedings of the National Academy of Sciences,* 1981, vol. 78 (12), 7350-7354